# EUROPEAN PATENT SPECIFICATION

(11) **EP 3 464 272 B1**
(45) Date of publication and mention of the grant of the patent: **08.12.2021**
(21) Application number: 17809742.4
(22) Date of filing: 07.06.2017
(51) Int. Cl.: C07D 401/14, C07D 405/14, C07D 413/14, C07D 401/04, C07D 401/12, C07D 471/04, C07D 471/20, C07D 491/107, C07D 519/00, A61K 31/506, A61K 31/537, A61P 35/00, A61P 9/00, A61P 19/00, A61P 37/00

(54) **NOVEL HETEROCYCLIC DERIVATIVES USEFUL AS SHP2 INHIBITORS**
NEUARTIGE, ALS SHP2-INHIBITOREN NÜTZLICHE HETEROCYCLISCHE DERIVATE
NOUVEAUX DÉRIVÉS HÉTÉROCYCLIQUES UTILES EN TANT QU'INHIBITEURS DE SHP2

(30) Priority: 07.06.2016 WO PCT/CN2016/085122
(43) Date of publication of application: 10.04.2019
(73) Proprietor: Jacobio Pharmaceuticals Co., Ltd., Beijing 101111 (CN)
(72) Inventor: MA, Cunbo, Beijing 101111 (CN); GAO, Panliang, Beijing 101111 (CN); CHU, Jie, Beijing 101111 (CN); WU, Xinping, Beijing 101111 (CN); WEN, Chunwei, Beijing 101111 (CN); KANG, Di, Beijing 101111 (CN); BAI, Jinlong, Beijing 101111 (CN); PEI, Xiaoyan, Beijing 101111 (CN)
(74) Representative: Arch, Peter Jonathan Sanders
(86) International application number: PCT/CN2017/087471
(87) International publication number: WO 2017/211303

(56) References cited:
- WO-A1-2015/107493
- WO-A1-2015/107494
- WO-A1-2015/107495
- WO-A1-2016/203405
- WO-A1-2016/203406

## Description

### TECHNICAL FIELD

This invention relates to certain novel pyrazine derivatives (Formula I) as SHP2 inhibitors which is shown as formula I, their synthesis and their use for treating a SHP2 mediated disorder. More particularly, this invention is directed to fused heterocyclic derivatives useful as inhibitors of SHP2.

### BACKGROUND ART

SHP2 (The Src Homolgy-2 phosphatase) is a non-receptor protein tyrosine phosphatase encoded by the PTPN11 gene that harbors a classical tyrosine phosphatase domain and two N-terminal Src homology 2 (SH2) domains and a C-terminal tail. The two SH2 domains control the subcellular localization and functional regulation of SHP2. In its inactive state, the N-terminal SH2 domain blocks the PTP domain and this autoinhibition is relieved by binding of the SH2 domains to specific phosphotyrosine sites on receptors or receptor-associated adaptor proteins. The stimulation, for example, by cytokines or growth factors leads to exposure of the catalytic site resulting in enzymatic activation of SHP2.

SHP2 is widely expressed and participated in multiple cell signaling processes, such as the Ras-Erk, PI3K-Akt, Jak-Stat, Met, FGFR, EGFR, and insulin receptors and NF-kB pathways, in which plays an important role in proliferation, differentiation, cell cycle maintenance and migration.

The hyperactivation of SHP2 catalytic activity caused by either germline or somatic mutations in PTPN11 has been identified in patients with Noonan syndrome, Leopard syndrome, juvenile myelomonocytic leukemias, myelodysplastic syndrome, B cell acute lymphoblastic leukemia/lymphoma, and acute myeloid leukemia. In addition, activating mutations of PTPN11 have been found in solid tumors as well, such as lung cancer, colon cancer, melanoma, neuroblastoma, and hepatocellular carcinoma. Therefore, the presence of the activated or up-regulated SHP2 protein in human cancers and other disease make SHP2 an excellent target for development of novel therapies.

WO 2015/107495 discloses n-azaspirocycloalkane substituted n-heteroaryl compounds and compositions for inhibiting the activity of SHP2.

WO 2015/107493 discloses 1-pyridazin-/triazin-3-yl-piper(-azine)/idine/pyrolidine derivatives and compositions thereof for inhibiting the activity of SHP2.

WO 2015/107494 discloses 1 -(triazin-3-yi_/pyridazin-3-yl)-piper(-azine)idine derivatives and compositions thereof for inhibiting the activity of SHP2.

The compounds of the present invention fulfill the need of small molecules in order to inhibit the activity of SHP2.

### SUMMARY OF INVENTION

The present invention relates to heterocyclic pyrazine compounds useful as SHP2 inhibitors and for the treatment of conditions mediated by SHP2. The compounds of the invention have the general structure as Formula I or a pharmaceutically acceptable salt: 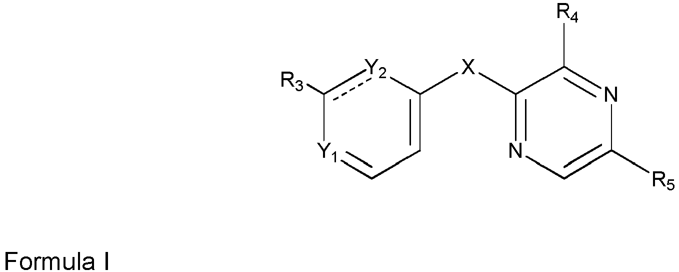 and
X is S;
Y₁ is N or CR₁;
Y₂ is N or CR₂;
R₁ combines with R₃, or R₂ combines with R₃, to form a 5-10 member heteroaryl, 5-10 member carbocyclic or 5-10 member heterocyclic ring, wherein each of the ring systems is independently optionally substituted with halogen, -CN, -OH, -NR₈R₉, -N₃, -NO₂, carbonyl, =O, oxo, substituted or unsubstituted C₁₋₆alkyl, substituted or unsubstituted C₁₋₆alkoxy, or C(O)R₈; when R₁ combines with R₃ to form a 5-10 member heteroaryl, 5-10 member carbocyclic ring or 5-10 member heterocyclic ring, R₂ is-H, halogen, -CN, -OH, -NH₂, -N₃, -NO₂, substituted or unsubstituted C₁₋₆alkoxy, or substituted or unsubstituted C₁₋₆alkyl;
when R₂ combines with R₃ to form a 5-10 member heteroaryl, 5-10 member carbocyclic ring or 5-10 member heterocyclic ring, R₁ is -H, halogen, -CN, -OH, -NH₂, -N₃, -NO₂, substituted or unsubstituted C₁₋₆alkoxy, or substituted or unsubstituted C₁₋₆alkyl;
R₄ is halogen, -CN, -OH, -NR₈R₉, -N₃, -NO₂, substituted or unsubstituted C₁₋₆alkoxy, substituted or unsubstituted C₁₋₆alkyl, C₅₋₁₈heterocyclic or C₅₋₁₈carbocyclic; wherein each of the ring systems is independently optionally substituted with halogen, -CN, - OH, -NO₂, carbonyl, =O, oxo, substituted or unsubstituted C₁₋₆alkyl, substituted or unsubstituted C₁₋₆alkoxy, -NR₈R₉, or -CH₂NR₈R₉;
R₅ is -H, halogen, -CN, -OH, -NR₈R₉, -N₃, -NO₂, C₁₋₆alkyl, C₁₋₆alkoxy, C₆₋₁₀aryl, C₆₋₁₀arylalkyl, C₆₋₁₀heteroaryl, C₅₋₁₈heterocyclic or C₅₋₁₈carbocyclic; and each of which is independently optionally substituted with halogen, -CN, -OH, -N₃, -NO₂, -NH₂, carbonyl, =O, oxo, substituted or unsubstituted C₁₋₆alkyl, substituted or unsubstituted C₁₋₆alkoxy, (CH₂)ₖNR₈R₉, (CH₂)ₖNHC(O)OR₈ or C(O)R₈; and k is 0, 1 or 2;
each R₈ and R₉ is independently -H, halogen, -CN, -OH, -N₃, -NO₂, C₁₋₆alkyl, C₁₋₆alkoxy, C₂₋₆alkenyl, NH(C₁₋₆alkyl), N(C₁₋₆alkyl)₂, C₅₋₁₀heterocyclic or C₅₋₁₀carbocyclic; and each of which may be independently optionally substituted with halogen, -CN, - OH, -NH₂, -N₃, -NO₂, -C₁₋₆alkylene-NH₂, -C₁₋₆alkylene-NH-C₁₋₆alkyl, -C₁₋₆alkylene-N(C₁₋₆alkyl)₂, -NHBoc, -CH₂NHBoc, -NH-C₁₋₆alkyl, -N(C₁₋₆alkyl)₂, -NH-C₁₋₆alkoxy,-N(C₁₋₆alkoxy)₂, substituted or unsubstituted C₁₋₆alkyl, or substituted or unsubstituted C₁₋₆alkoxy; and each heterocyclic ring contains 1, 2, 3 or 4 heteroatoms selected from N, O or S;
wherein alkyl is saturated monovalent hydrocarbon radicals having straight, branched or cyclic moieties, alkoxy is straight, branched chain or cyclic alkyl group, and heterocyclic is unsubstituted and substituted mono- or polycyclic non-aromatic ring system containing one or more heteroatoms.

The present invention further provides some preferred technical solutions with regard to compound of Formula (I).

In some embodiments of Formula (I), when R₂ combines with R₃ to form a 5-10 member heteroaryl, 5-10 member carbocyclic ring or 5-10 member heterocyclic ring, R₁ is -H, -F, -CI, -Br, -I, -CN, -OH, -NH₂, substituted or unsubstituted C₁₋₃alkoxy, or substituted or unsubstituted C₁₋₃alkyl.

In some embodiments of Formula (I), when R₂ combines with R₃ to form a 5-10 member heteroaryl, 5-10 member carbocyclic ring or 5-10 member heterocyclic ring, R₁ is -H, -F, -CI, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with halogen, OH or NH₂.

In some embodiments of Formula (I), when R₁ combines with R₃ to form a 5-10 member heteroaryl, 5-10 member carbocyclic ring or 5-10 member heterocyclic ring, R₂ is -H, -F, -CI, -Br, -I, -CN, -OH, -NH₂, substituted or unsubstituted C₁₋₃alkoxy, or substituted or unsubstituted C₁₋₃alkyl.

In some embodiments of Formula (I), when R₁ combines with R₃ to form a 5-10 member heteroaryl, 5-10 member carbocyclic ring or 5-10 member heterocyclic ring, R₂ is -H, -F, -CI, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with halogen, OH or NH₂.

In some embodiments of Formula (I), R₃ is -,

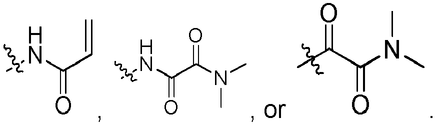

In some embodiments of Formula (I), R₁ combines with R₃, or R₂ combines with R₃, to form a 5-10 member heteroaryl or 5-10 member heterocyclic ring, wherein each of the ring systems is optionally substituted with halogen, -CN, -OH, -NH₂, carbonyl, =O, oxo, substituted or unsubstituted C₁₋₆alkyl, substituted or unsubstituted C₁₋₆alkoxy, or C(O)R₈.

In some embodiments of Formula (I), R₁ combines with R₃, or R₂ combines with R₃, to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring; wherein each of the ring systems contains 1, 2, 3 or 4 heteroatoms select from N, O or S, and is optionally substituted with -F, -CI, -Br, -I, -CN, -OH, -NH₂, carbonyl, =O, oxo, substituted or unsubstituted C₁₋₃alkyl, substituted or unsubstituted C₁₋₃alkoxy, or C(O)R₈.

In some embodiments of Formula (I), R₁ combines with R₃ to form a 5-10 member heteroaryl or a 5-10 member heterocyclic ring; wherein each of the ring systems is optionally substituted with halogen, -CN, -OH, -NH₂, carbonyl, =O, oxo, substituted or unsubstituted C₁₋₆alkyl, substituted or unsubstituted C₁₋₆alkoxy, or C(O)R₈.

In some embodiments of Formula (I), R₁ combines with R₃ to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring, wherein each of the ring systems contains 1, 2, 3 or 4 heteroatoms select from N, O or S, and is optionally substituted with -F, -CI, -Br, -I, -CN, -OH, -NH₂, carbonyl, =O, oxo, substituted or unsubstituted C₁₋₃alkyl, substituted or unsubstituted C₁₋₃alkoxy, or C(O)R₈.

In some embodiments of Formula (I), R₁ combines with R₃ to form a 5-membered heteroaryl, 6-membered heteroaryl, 5-membered heterocyclic ring or 6-membered heterocyclic, wherein each of the ring systems contains 1, 2 or 3 heteroatoms select from N, O or S, and is optionally substituted with -F, -CI, -Br, -I, -CN, -OH, -NH₂, - carbonyl, =O, oxo, methyl, ethyl, propyl, isopropyl, methoxy, CHF₂, CH₂F, CF₃ or C(O)R₈; and each methyl, ethyl, propyl, isopropyl, methoxy, CHF₂, CH₂F or C(O)R₈ is independently optionally substituted with -F, -CI, -Br or -I.

In some embodiments of Formula (I), R₂ combines with R₃ to form a 5-10 member heteroaryl or 5-10 member heterocyclic ring, wherein each of the ring systems is optionally substituted with halogen, -CN, -OH, -NH₂, carbonyl, =O, oxo, substituted or unsubstituted C₁₋₆alkyl, substituted or unsubstituted C₁₋₆alkoxy or C(O)R₈.

In some embodiments of Formula (I), R₂ combines with R₃ to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring, wherein each of the ring systems contains 1, 2, 3 or 4 heteroatoms select from N, O or S, and is optionally substituted with -F, -CI, -Br, -I, -CN, -OH, -NH₂, carbonyl, =O, oxo, substituted or unsubstituted C₁₋₃alkyl, substituted or unsubstituted C₁₋₃alkoxy, or C(O)R₈.

In some embodiments of Formula (I), R₄ is -F, -CI, -Br, -CN, -OH, -NR₈R₉, substituted or unsubstituted C₁₋₆alkyl, substituted or unsubstituted C₁₋₆alkoxy, C₅₋₁₈heterocyclic or C₅₋₁₀carbocyclic, wherein each of the ring systems is optionally substituted with -F, -CI, -Br, -CN, -OH, -NO₂, carbonyl, =O, oxo, substituted or unsubstituted C₁₋₆alkyl,-NH-C₁₋₆alkyl, -NH-C₁₋₆alkoxy, -C₁₋₆alkylene-NH₂, or -C₁₋₆alkylene-NH-C₁₋₆alkyl.

In some embodiments of Formula (I), R₄ is -F, -CI, -CN, -OH, -NH₂, substituted or unsubstituted C₁₋₃alkyl, substituted or unsubstituted C₁₋₃alkoxy, 5-membered heterocyclic containing 1, 2 or 3 heteroatoms select from N or O, 6-membered heterocyclic containing 1, 2 or 3 heteroatoms select from N or O, 5-membered carbocyclic or 6-membered carbocyclic; wherein each of the ring systems is optionally substituted with -F, -CI, -Br, -CN, -OH, carbonyl, =O, oxo, substituted or unsubstituted C₁₋₃alkyl, substituted or unsubstituted C₁₋₃alkoxy, -NH₂, -NH-C₁₋₃alkyl,-NH-C₁₋₃alkoxy, -C₁₋₃alkylene-NH₂ or -C₁₋₃alkylene-NH-C₁₋₃alkyl.

In some embodiments of Formula (I), R₄ is -CI, -NH₂, methyl or piperidinyl, wherein the piperidinyl is optionally substituted with methyl, -NH₂ or -CH₂NH₂.

In some embodiments of Formula (I), R₅ is -H, -F, -CI, -Br, -I, -NR₈R₉, C₁₋₃alkyl, C₁₋₃alkoxy, C₆₋₉aryl, C₆₋₉arylalkyl, C₆₋₉heteroaryl, C₆₋₁₇heterocyclic or C₆₋₁₇carbocyclic, wherein each of which is independently optionally substituted with halogen, -CN, - OH, -N₃, -NO₂, -NH₂, carbonyl, =O, oxosubstituted or unsubstituted C₁₋₆alkyl, substituted or unsubstituted C₁₋₆alkoxy, (CH₂)ₖNR₈R₉, (CH₂)ₖNHC(O)OR₈ or C(O)R₈; and k is 0, 1 or 2.

In some embodiments of Formula (I), the C₆₋₉heteroaryl contains 1, 2, 3 or 4 heteroatoms select from N, O or S, and the C₆₋₉heteroaryl is 6-membered heteroaryl, 7-membered heteroaryl, 8-membered heteroaryl or 9-membered heteroaryl; the C₆₋₁₇heterocyclic contains 1, 2, 3, 4, 5, or 6 heteroatoms select from N, O, or S, and the C₆₋₁₇heterocyclic is 6-membered heterocyclic, 7-membered heterocyclic, 8-membered heterocyclic, 9-membered heterocyclic, 10-membered heterocyclic, 11-membered heterocyclic, 12-membered heterocyclic, 13-membered heterocyclic, 14-membered heterocyclic, 15-membered heterocyclic, 16-membered heterocyclic or 17-membered heterocyclic.

In some embodiments of Formula (I), R₅ is -F, -CI, -Br, -NR₈R₉, methyl, ethyl, propyl, isopropyl, methoxy, ethoxy, propoxy, isopropoxy, 6-membered aryl, 7-membered aryl, 8-membered aryl, 9-membered aryl, 6-membered arylalkyl, 7-membered arylalkyl, 8-membered arylalkyl, 9-membered arylalkyl, 6-membered heteroaryl, 7-membered heteroaryl, 8-membered heteroaryl, 9-membered heteroaryl, 6-membered heterocyclic, 7-membered heterocyclic, 8-membered heterocyclic, 9-membered heterocyclic 10-membered heterocyclic, 11-membered heterocyclic, 12-membered heterocyclic, 13-membered heterocyclic, 14-membered heterocyclic, 15-membered heterocyclic, 16-membered heterocyclic, 6-membered carbocyclic, 7-membered carbocyclic, 8-membered carbocyclic, 9-membered carbocyclic, 10-membered carbocyclic, 11-membered carbocyclic, 12-membered carbocyclic, 13-membered carbocyclic, 14-membered carbocyclic, 15-membered carbocyclic or 16-membered carbocyclic; and each heteroaryl contains 1, 2 or 3 heteroatoms select from N, O or S, and each heterocyclic contains 1, 2, 3, or 4 heteroatoms select from N, O or S; wherein each of which is independently optionally substituted with -F, -CI, -Br, -I, -CN, -OH, -NO₂, -NH₂, carbonyl, =O, oxo, substituted or unsubstituted C₁₋₃alkyl, substituted or unsubstituted C₁₋₃alkoxy, (CH₂)ₖNR₈R₉, (CH₂)ₖNHC(O)OR₈, or substituted or unsubstituted C(O)R₈; and k is 0, 1 or 2.

In some embodiments of Formula (I),
R₅ is 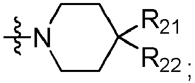
each R₂₁ and R₂₂ is independently halogen, C₁₋₃alkyl, -NH₂, -C₁₋₃alkylene-NH₂, -C₁₋₂alkylene-NH-C₁₋₃alkyl, -C₁₋₂alkylene-N(C₁₋₃alkyl)₂, -NHBoc or -CH₂NHBoc;
or R₂₁ and R₂₂ together with the carbon atom to which they are both attached form a 5-10 member heteroaryl, 5-10 member carbocyclic or a 5-10 member heterocyclic ring, wherein each of the ring system is optionally substituted with halogen, -CN, -OH, carbonyl, =O, oxo, -C₁₋₃alkylene-NH₂, -C₁₋₂alkylene-NH-C₁₋₃alkyl, -C₁₋₂alkylene-N(C₁₋₃alkyl)₂, -NHBoc, -CH₂NHBoc; -NH₂, C₁₋₃alkoxy or C₁₋₃alkyl.

In some embodiments of Formula (I), R₂₁ and R₂₂ together with the carbon atom to which they are both attached form a 5-membered heteroaryl, 6-membered heteroaryl, 7-membered heteroaryl, 8-membered heteroaryl, 9-membered heteroaryl, 10-membered heteroaryl, 5-membered heterocyclic ring, 6-membered heterocyclic ring, 7-membered heterocyclic ring, 8-membered heterocyclic ring, 9-membered heterocyclic ring or 10-membered heterocyclic ring; wherein each of the ring system contains 1, 2 or 3 heteroatoms select from N, O or S, and is independently optionally substituted with halogen, -CN, -OH, carbonyl, =O, oxo, -NH₂, C₁₋₃alkoxy or C₁₋₃alkyl.

In all embodiments of Formula (I), X is S.

In some embodiments of Formula (I), Y₁ is N and Y₂ is CR₂.

In some embodiments of Formula (I), Y₂ is N and Y₁ is CR₁.

In some embodiments of Formula (I), R₄ is -NH₂.

In some embodiments of Formula (I), R₅ is -NH₂,

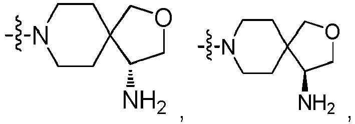

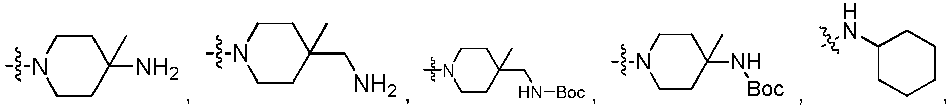

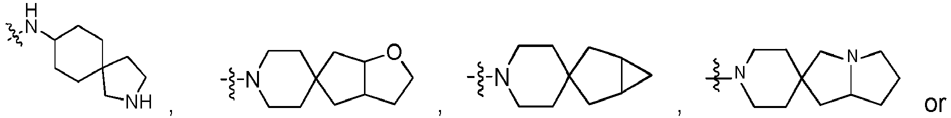

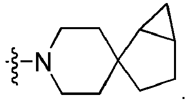

In some embodiments of Formula (I), each R₈ and R₉ is independently -H, halogen, CN, -OH, -NO₂, -C₁₋₆alkylene-NH₂, -C₁₋₆alkylene-NH-C₁₋₆alkyl, -C₁₋₆alkylene-N(C₁₋₆alkyl)₂, -CH₂NHBoc; -NH-C₁₋₆alkyl, -N(C₁₋₆alkyl)₂, -NH-C₁₋₆alkoxy, -N(C₁₋₆alkoxy)₂, C₁₋₆alkyl, C₁₋₆alkoxy, C₂₋₆alkenyl, C₅₋₁₀heterocyclic or C₅₋₁₀carbocyclic; each of which may be optionally substituted.

In some embodiments of Formula (I), each R₈ and R₉ is independently -H, -F, -CI, - CN, -OH, -NO₂, -C₁₋₃alkylene-NH₂, -C₁₋₃alkylene-NH-C₁₋₃alkyl, -C₁₋₃alkylene-N(C₁₋₃alkyl)₂, -CH₂NHBoc; -NH-C₁₋₃alkyl, -N(C₁₋₃alkyl)₂, -NH-C₁₋₃alkoxy, -N(C₁₋₃alkoxy)₂, C₁₋₄alkyl, C₁₋₃alkoxy, C₂₋₃alkenyl, 5-membered heterocyclic, 6-membered heterocyclic, 7-membered heterocyclic, 8-membered heterocyclic, 9-membered heterocyclic, 10-membered heterocyclic, 5-membered carbocyclic, 6-membered carbocyclic, 7-membered carbocyclic, 8-membered carbocyclic, 9-membered carbocyclic or 10-membered carbocyclic; and each of which may be independently optionally substituted with halogen, -CN, -OH, -NH₂, -N₃, -NO₂, -C₁₋₆alkylene-NH₂,-C₁₋₆alkylene-NH-C₁₋₆alkyl, -C₁₋₆alkylene-N(C₁₋₆alkyl)₂, -NHBoc, -CH₂NHBoc; -NH-C₁₋₆alkyl, -N(C₁₋₆alkyl)₂, -NH-C₁₋₆alkoxy, -N(C₁₋₆alkoxy)₂, substituted or unsubstituted C₁₋₆alkyl, or substituted or unsubstituted C₁₋₆alkoxy; and each heterocyclic contains 1, 2, 3 or 4 heteroatoms select from N, O or S.

In some embodiments of Formula (I), each R₈ and R₉ is independently -H, methyl, tert-butyl, -CH=CH₂, N(CH₃)₂,

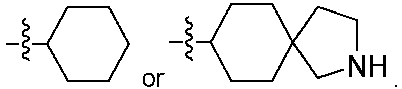

In some embodiments of Formula (I), the compound is of Formula II: 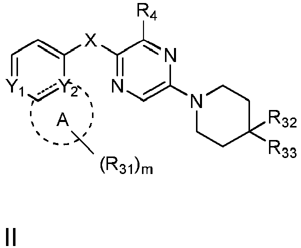 and
X is S;
Y₁ is N or CR₂₅;
Y₂ is C;
R₂₅ is H, halogen, C₁₋₃alkyl, C₁₋₃alkoxy, C₂₋₃alkenyl or C₂₋₃alkylnyl;
ring 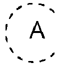 is 5-8 member heteroaryl containing 1, 2, 3 or 4 heteroatoms selected from N, O or S, 5-8 member carbocyclic or 5-8 member heterocyclic ring containing 1, 2, 3 or 4 heteroatoms selected from N, O or S;
R₃₁ is -H, halogen, -OH, -NH₂, -(C=O)C₁₋₃alkyl, -CN, -NO₂, carbonyl, =O, oxo, carboxyl, -C₁₋₃alkylene-NH₂; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy;
m is 0, 1, 2, 3 or 4;
R₄ is halogen, -NH₂, substituted or unsubstituted C₁₋₃alkoxy, or substituted or unsubstituted C₁₋₃alkyl;
each R₃₂ and R₃₃ is independently -H, halogen, -OH, -NH₂, -CN, -NO₂, -C₁₋₃alkylene-NH₂, -C₁₋₂alkylene-NH-C₁₋₃alkyl, -C₁₋₂alkylene-N(C₁₋₃alkyl)₂, -NHBoc, -CH₂NHBoc;-NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy;
or R₃₂ and R₃₃ together with the carbon atom to which they are both attached form a 5-8 member heteroaryl containing 1, 2 or 3 heteroatoms select from N, O or S, or 5-8 member heterocyclic ring containing 1, 2 or 3 heteroatoms select from N, O or S, wherein each of the ring systems is optionally substituted with halogen, -CN, -OH, - NH₂, carbonyl, =O, oxo, -CH₂NH₂, -C₁₋₃alkylene-NH₂, -C₁₋₂alkylene-NH-C₁₋₃alkyl, -C₁₋₂alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy.

The present invention further provides some preferred technical solutions with regard to compound of Formula (II).

In some embodiments of Formula (II), ring 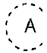 is 5-membered heteroaryl, 6-membered heteroaryl, 7-membered heteroaryl, 5-membered heterocyclic ring, 6-membered heterocyclic ring, 7-membered heterocyclic ring, 5-membered carbocyclic, 6-membered carbocyclic, 7-membered carbocyclic or 8-membered carbocyclic; and each of the ring systems contains 1, 2 or 3 heteroatoms selected from N, O or S.

In some embodiments of Formula (II), ring 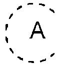 is 5-membered heterocyclic ring containing 1, 2 or 3 heteroatoms selected from N or O, 6-membered heterocyclic ring containing 1 or 2 heteroatoms selected from N or O, or 5-membered carbocyclic.

In some embodiments of Formula (II), R₃₁ is -H, -F, -CI, -Br, -I, -OH, -NH₂, carbonyl, =O, oxo, -(CO)C₁₋₃alkyl, -C₁₋₃alkylene-NH₂, -NH-C₁₋₃alkyl, -N(C₁₋₃alkyl)₂, substituted or unsubstituted C₁₋₃alkyl, or substituted or unsubstituted C₁₋₃alkoxy.

In some embodiments of Formula (II), R₃₁ is -F, -COCH₃, carbonyl, =O, oxo, -CH₃ or -CF₃.

In some embodiments of Formula (II), R₃₂ and R₃₃ together with the carbon atom to which they are both attached to form a 5-membered heteroaryl, 6-membered heteroaryl, 7-membered heteroaryl, 5-membered heterocyclic ring, 6-membered heterocyclic ring, or 7-membered heterocyclic ring; wherein each of the ring systems contains 1, 2, or 3 heteroatoms select from N, O or S, and is optionally substituted with halogen, -CN, -OH, -NH₂, carbonyl, =O, oxo, -CH₂NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy.

In some embodiments of Formula (II), R₃₂ and R₃₃ together with the carbon atom to which they are both attached form a 5-membered heterocyclic ring; 6-membered heterocyclic ring or 7-membered heterocyclic ring; wherein each of the ring system contains 1 or 2 heteroatoms independently select from O or N, and is optionally substituted with halogen, -CN, -OH, -NH₂, carbonyl, =O, oxo, -NO₂, C₁₋₃alkyl or C₁₋₃alkoxy.

In some embodiments of Formula (II), R₃₂ and R₃₃ together with the carbon atom to which they are both attached to form a 5-membered heterocyclic ring; and the heterocyclic ring contains 1 heteroatoms selected from O or N, and is optionally substituted with -F, -CI, -OH, -NH₂, carbonyl, =O, oxo, methyl or methoxy.

In some embodiments of Formula (II), each R₃₂ and R₃₃ is independently -CH₂NH₂,-CH₂NHBoc or methyl.

In some embodiments of Formula (II), Y₁ is N.

In some embodiments of Formula (II), Y₂ is C.

In some embodiments of Formula (II), is -H or -CI.

In some embodiments of Formula (II), R₄ is -NH₂.

In some embodiments of Formula (I), the compound is of Formula III: 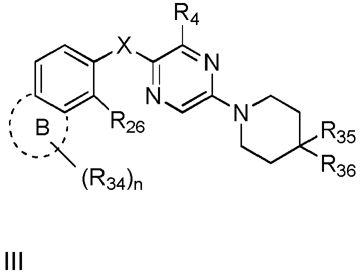 and
X is S;
R₂₆ is -H, halogen, substituted or unsubstituted C₁₋₃alkyl, or substituted or unsubstituted C₁₋₃alkoxy;
ring 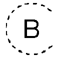 is 5-8 member heteroaryl or 5-8 member heterocyclic ring; and each ring system independently contains 1, 2, 3 or 4 heteroatoms selected from N, O or S;
R₃₄ is -H, halogen, -OH, -NR₃₅R₃₆, -CN, -NO₂, carbonyl, =O, oxo, substituted or unsubstituted C₁₋₃alkyl, or substituted or unsubstituted C₁₋₃alkoxy;
n is 0, 1, 2 or 3;
R₄ is halogen, -NH₂, substituted or unsubstituted C₁₋₆alkoxy, or substituted or unsubstituted C₁₋₆ alkyl;
each R₃₅ and R₃₆ is independently -H, halogen, -OH, -NH₂, -CN, -NO₂, -CH₂NH₂, substituted or unsubstituted C₁₋₃alkyl, or substituted or unsubstituted C₁₋₃alkoxy;
or R₃₅ and R₃₆ together with the carbon atom to which they are both attached to form a 5-8 member heteroaryl or 5-8 member heterocyclic ring, wherein each of the ring system independently contains 1, 2 or 3 heteroatoms selected from N, O or S, and is optionally substituted with halogen, -CN, -OH, -NH₂, carbonyl, =O, oxo, -CH₂NH₂,-C₁₋₃alkylene-NH₂, -C₁₋₂alkylene-NH-C₁₋₃alkyl, -C₁₋₂alkylene-N(C₁₋₃alkyl)₂, -NHBoc,-CH₂NHBoc; -NH-C₁₋₃alkyl, -N(C₁₋₃alkyl)₂, -NHC₁₋₃alkoxy, -N(C₁₋₃alkoxy)₂, substituted or unsubstituted C₁₋₃alkyl, or substituted or unsubstituted C₁₋₃alkoxy.

The present invention further provides some preferred technical solutions with regard to compound of Formula (III).

In some embodiments of Formula (III), ring 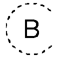 is 5-membered heteroaryl, 6-membered heteroaryl, 7-membered heteroaryl, 5-membered heterocyclic ring, 6-membered heterocyclic ring or 7-membered heterocyclic ring; and each of the ring system independently contains 1, 2 or 3 heteroatoms selected from N, O or S.

In some embodiments of Formula (III), ring 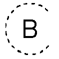 is 5-membered heterocyclic ring or 6-membered heterocyclic ring; and each of the ring system independently contains 1 or 2 heteroatoms selected from N or O.

In some embodiments of Formula (III), R₃₅ and R₃₆ together with the carbon atom to which they are both attached to form a 5-membered heteroaryl, 6-membered heteroaryl, 7-membered heteroaryl, 5-membered heterocyclic ring, 6-membered heterocyclic ring or 7-membered heterocyclic ring; wherein each of the ring system is independently contains 1, 2, or 3 heteroatoms select from N, O or S, and is optionally substituted with halogen, -CN, -OH, -NH₂, carbonyl, =O, oxo, -CH₂NH₂,-C₁₋₃alkylene-NH₂, -C₁₋₂alkylene-NH-C₁₋₃alkyl, -C₁₋₂alkylene-N(C₁₋₃alkyl)₂, -NHBoc,-CH₂NHBoc; -NH-C₁₋₃alkyl, -N(C₁₋₃alkyl)₂, -NHC₁₋₃alkoxy, -N(C₁₋₃alkoxy)₂, substituted or unsubstituted C₁₋₃alkyl, or substituted or unsubstituted C₁₋₃alkoxy.

In some embodiments of Formula (III), R₃₅ and R₃₆ together with the carbon atom to which they are both attached to form a 5-membered heterocyclic ring; 6-membered heterocyclic ring or 7-membered heterocyclic ring; wherein each of the ring system independently contains 1 or 2 heteroatoms independently select from O or N, and is optionally substituted with halogen, -CN, -OH, -NH₂, carbonyl, =O, oxo, -NO₂, C₁₋₃alkyl or C₁₋₃alkoxy.

In some embodiments of Formula (III), R₃₅ and R₃₆ together with the carbon atom to which they are both attached to form a 5-membered heterocyclic ring, wherein the ring system contains 1 heteroatom independently select from O or N, and is optionally substituted with -F, -CI, -OH, -NH₂, carbonyl, =O, oxo, methyl or methoxy.

In some embodiments of Formula (III), R₂₆ is -H or -Cl

In some embodiments of Formula (III), R₄ is -NH₂.

In some embodiments of Formula (III), R₃₄ is -F, -COCH₃, carbonyl, =O, oxo, -CH₃ or -CF₃.

In some embodiments of Formula (I), Formula (II) or Formula (III), each substituted or unsubstituted C₁₋₆alkyl is independently C₁₋₆alkyl, or C₁₋₆alkyl substituted with halogen, -OH, -CN, NH₂, -NO₂, carbonyl, =O, oxo, -C₁₋₆alkylene-NH₂, -C₁₋₆alkylene-NH-C₁₋₆alkyl, or -C₁₋₆alkylene-N(C₁₋₆alkyl)₂; each substituted or unsubstituted C₁₋₆alkoxy is independently C₁₋₆alkoxy, or C₁₋₆alkoxy substituted with halogen, -OH, -CN, NH₂, -NO₂, carbonyl, =O, oxo, -C₁₋₆alkylene-NH₂, -C₁₋₆alkylene-NH-C₁₋₃alkyl, or -C₁₋₆alkylene-N(C₁₋₆alkyl)₂.

In some embodiments of Formula (I), Formula (II) or Formula (III), each substituted or unsubstituted C₁₋₃alkyl is independently C₁₋₃alkyl, or C₁₋₃alkyl substituted with halogen, -OH, -CN, NH₂, -NO₂, carbonyl, =O, oxo, -C₁₋₃alkylene-NH₂, -C₁₋₃alkylene-NH-C₁₋₃alkyl, or -C₁₋₃alkylene-N(C₁₋₃alkyl)₂; each substituted or unsubstituted C₁₋₃alkoxy is independently C₁₋₃alkoxy, or C₁₋₃alkoxy substituted with halogen, -OH, -CN, NH₂, -NO₂, carbonyl, =O, oxo, -C₁₋₃alkylene-NH₂, -C₁₋₃alkylene-NH-C₁₋₃alkyl, or -C₁₋₃alkylene-N(C₁₋₃alkyl)₂.

In some embodiments of Formula (I), Formula (II) or Formula (III), each C₁₋₆alkyl is independently methyl, ethyl, propyl, isopropyl, cyclopropyl, n-butyl, isobutyl, tert-butyl, n-pentyl, neopentyl, isopentyl, cyclopentyl, n-hexyl or cyclohexyl.

In some embodiments of Formula (I), Formula (II) or Formula (III), each C₁₋₃alkyl is independently methyl, ethyl, propyl, isopropyl or cyclopropyl.

In some embodiments of Formula (I), Formula (II) or Formula (III), each C₁₋₃alkoxy is independently methoxy, ethoxy, propoxy, isopropoxy or cyclopropyloxy.

In some embodiments of Formula (I), Formula (II) or Formula (III), each C₂₋₃alkenyl is independently -CH=CH₂, -CH₂-CH=CH₂, or -CH=CH-CH₃.

In some embodiments of Formula (I), Formula (II) or Formula (III), each C₂₋₃alkylnyl is independently -C=CH, -CH₂-C=CH, or -C=C-CH₃.

In some embodiments of Formula (I), Formula (II) or Formula (III), each halogen is independently -F, -CI, -Br or -I.

In some embodiments of Formula (I), Formula (II) or Formula (III), each heterocyclic group and each carbocyclic group includes single ring, spiral ring, bridge ring, fused ring and all various combinations of spiral ring, bridge ring, and/or fused ring.

In some embodiments of Formula (I), Formula (II) or Formula (III), the said spiral ring includes 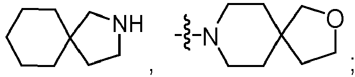 and the said various combinations of spiral ring, bridge ring, and/or fused ring include

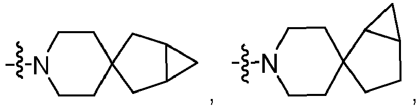

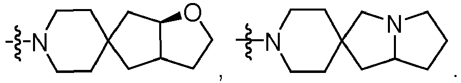

The present invention further provides some preferred technical solutions with regard to compound of Formula (I) or Formula (II), compound is
1) 6-((3-amino-5-(4-(aminomethyl)-4-methylpiperidin-1-yl)pyrazin-2-yl)thio)-2H-benzo[b][1,4]oxazin-3(4H)-one;
2) (S)-1-(4-((3-amino-5-(4-amino-2-oxa-8-azaspiro[4.5]decan-8-yl)pyrazin-2-yl)thio)-3,3-difluoroindolin-1-yl)ethanone;
3) (S)-4-((3-amino-5-(4-amino-2-oxa-8-azaspiro[4.5]decan-8-yl)pyrazin-2-yl)thio)-3,3-difluoroindolin-2-one;
4) 4-((3-amino-5-(4-(aminomethyl)-4-methylpiperidin-1-yl)pyrazin-2-yl)thio)indolin-2-one;
5) (S)-4-((3-amino-5-(4-amino-2-oxa-8-azaspiro[4.5]decan-8-yl)pyrazin-2-yl)thio)-3,3-difluoro-1H-pyrrolo[2,3-b]pyridin-2(3H)-one;
6) (S)-4-((3-amino-5-(4-amino-2-oxa-8-azaspiro[4.5]decan-8-yl)pyrazin-2-yl)thio)-3,3-difluoro-1-methylindolin-2-one;
7) (S)-4-((3-amino-5-(4-amino-2-oxa-8-azaspiro[4.5]decan-8-yl)pyrazin-2-yl)thio)indoline-2,3-dione hydrochloride;
8) 1-(4-((3-amino-5-(4-(aminomethyl)-4-methylpiperidin-1-yl)pyrazin-2-yl)thio)-3,3-difluoroindolin-1-yl)ethan-1-one;
9) 4-((3-amino-5-(4-(aminomethyl)-4-methylpiperidin-1-yl)pyrazin-2-yl)thio)indoline-2,3-dione;
10) 6-(4-(aminomethyl)-4-methylpiperidin-1-yl)-3-((3,4-dihydro-2H-benzo[b][1,4]oxazin-6-yl)thio)pyrazin-2-amine;
11) (S)-8-(5-((1H-pyrrolo[2,3-b]pyridin-4-yl)thio)-6-aminopyrazin-2-yl)-2-oxa-8-azaspiro[4.5]decan-4-amine;
12) (S)-8-(6-amino-5-((3,3-dimethylindolin-4-yl)thio)pyrazin-2-yl)-2-oxa-8-azaspiro[4.5]decan-4-amine;
13) (S)-8-(6-amino-5-((3-fluoro-1H-indol-4-yl)thio)pyrazin-2-yl)-2-oxa-8-azaspiro[4.5]decan-4-amine;
14) (S)-8-(5-((1H-indol-4-yl)thio)-6-aminopyrazin-2-yl)-2-oxa-8-azaspiro[4.5]decan-4-amine;
15) (S)-1-(4-((3-amino-5-(4-amino-2-oxa-8-azaspiro[4.5]decan-8-yl)pyrazin-2-yl)thio)-1H-indol-1-yl)ethanone;
16) (S)-6-((3-amino-5-(4-amino-2-oxa-8-azaspiro[4.5]decan-8-yl)pyrazin-2-yl)thio)-2H-benzo[b][1,4]oxazin-3(4H)-one;
17) (S)-4-((3-amino-5-(4-amino-2-oxa-8-azaspiro[4.5]decan-8-yl)pyrazin-2-yl)thio)-1,3,3-trimethylindolin-2-one;
18) (4S)-8-(6-amino-5-((3-fluoroindolin-4-yl)thio)pyrazin-2-yl)-2-oxa-8-azaspiro[4.5]decan-4-amine;
19) 1-(4-((3-amino-5-((S)-4-amino-2-oxa-8-azaspiro[4.5]decan-8-yl)pyrazin-2-yl)thio)-3-fluoro-3-methylindolin-1-yl)ethanone;
20) (S)-8-(6-amino-5-((3,3-difluoroindolin-4-yl)thio)pyrazin-2-yl)-2-oxa-8-azaspiro[4.5]decan-4-amine;
21) 1-(4-((3-amino-5-((S)-4-amino-2-oxaspiro[4.5]decan-8-yl)pyrazin-2-yl)thio)-3-methylindolin-1-yl)ethanone;
22) (S)-8-(6-amino-5-((8-chloro-4,4-difluoro-1,2,3,4-tetrahydroquinolin-5-yl)thio)pyrazin-2-yl)-2-oxa-8-azaspiro[4.5]decan-4-amine;
23) (4S)-8-(6-amino-5-((8-chloro-4-fluoro-1,2,3,4-tetrahydroquinolin-5-yl)thio)pyrazin-2-yl)-2-oxa-8-azaspiro[4.5]decan-4-amine;
24) (S)-8-(6-amino-5-((3,3-difluoro-1-methylindolin-4-yl)thio)pyrazin-2-yl)-2-oxa-8-azaspiro[4.5]decan-4-amine;
25) (S)-6-((3-amino-5-(4-amino-2-oxa-8-azaspiro[4.5]decan-8-yl)pyrazin-2-yl)thio)-3,3-difluoroindolin-2-one;
26) 4-((3-amino-5-((S)-4-amino-2-oxa-8-azaspiro[4.5]decan-8-yl)pyrazin-2-yl)thio)-3-fluoroindolin-2-one;
27) (S)-8-(6-amino-5-((3,3-difluoro-2,3-dihydrobenzofuran-4-yl)thio)pyrazin-2-yl)-2-oxa-8-azaspiro[4.5]decan-4-amine;
28) (S)-8-(6-amino-5-((4,4-difluorochroman-5-yl)thio)pyrazin-2-yl)-2-oxa-8-azaspiro[4.5]decan-4-amine;
29) 4-((3-amino-5-((S)-4-amino-2-oxa-8-azaspiro[4.5]decan-8-yl)pyrazin-2-yl)thio)-3-fluoro-1-methyl-3-(trifluoromethyl)indolin-2-one;
30) (S)-6-((3-amino-5-(4-amino-2-oxa-8-azaspiro[4.5]decan-8-yl)pyrazin-2-yl)thio)-7-chloroindolin-2-one;
31) (S)-8-(6-amino-5-((5-chloro-3,4-dihydro-2H-benzo[b][1,4]oxazin-6-yl)thio)pyrazin-2-yl)-2-oxa-8-azaspiro[4.5]decan-4-amine;
32) (S)-7-((3-amino-5-(4-amino-2-oxa-8-azaspiro[4.5]decan-8-yl)pyrazin-2-yl)thio)-8-chloro-3,4-dihydroquinolin-2(1H)-one;
33) (S)-6-((3-amino-5-(4-amino-2-oxa-8-azaspiro[4.5]decan-8-yl)pyrazin-2-yl)thio)-5-chloro-2H-benzo[b][1,4]oxazin-3(4H)-one;
34) 4-((3-amino-5-(4-(aminomethyl)-4-methylpiperidin-1-yl)pyrazin-2-yl)thio)-3,3-difluoro-1H-pyrrolo[2,3-b]pyridin-2(3H)-one;
35) (S)-4-((3-amino-5-(4-amino-2-oxa-8-azaspiro[4.5]decan-8-yl)pyrazin-2-yl)thio)-1H-benzo[d]imidazol-2(3H)-one;
36) (S)-4-((3-amino-5-(4-amino-2-oxa-8-azaspiro[4.5]decan-8-yl)pyrazin-2-yl)thio)-1,3-dimethyl-1H-benzo[d]imidazol-2(3H)-one;
37) (S)-8-(6-amino-5-((2,2-difluoro-2,3-dihydro-1H-benzo[d]imidazol-4-yl)thio)pyrazin-2-yl)-2-oxa-8-azaspiro[4.5]decan-4-amine;
38) (S)-8-(6-amino-5-((2,2-difluoro-1,3-dimethyl-2,3-dihydro-1H-benzo[d]imidazol-4-yl)thio)pyrazin-2-yl)-2-oxa-8-azaspiro[4.5]decan-4-amine;
39) (4S)-8-(6-amino-5-((1-amino-3,3-difluoro-2,3-dihydro-1H-inden-4-yl)thio)pyrazin-2-yl)-2-oxa-8-azaspiro[4.5]decan-4-amine;
40) (S)-1-(4-((3-amino-5-(4-amino-2-oxa-8-azaspiro[4.5]decan-8-yl)pyrazin-2-yl)thio)indolin-1-yl)ethanone.

Disclosed herein is a pharmaceutical composition comprising at least one compound described herein and at least one pharmaceutically acceptable excipient. In composition, the said compound is in a weight ratio to the said excipient within the range from about 0.0001 to about 10.

Disclosed herein is a use of the pharmaceutical composition of as described herein for use as a medicament.

As disclosed herein, the medicament can be used for the treatment or prevention of cancer, cancer metastasis, cardiovascular disease, an immunological disorder or an ocular disorder.

Disclosed herein is at least one compound described herein for use as a medicament.

As disclosed herein, the medicament may be used for the treatment or prevention of cancer, cancer metastasis, cardiovascular disease, an immunological disorder or an ocular disorder.

At least one compound for use described herein which is for use in the treatment of cancer, the prevention of cancer metastasis or the treatment of cardiovascular disease, an immunological disorder or an ocular disorder.

Disclosed herein is at least one compound described herein for use as an inhibitor of SHP2.

Disclosed herein is at least one compound described herein, or a pharmaceutically acceptable salt thereof for use in a method of treating a patient having a condition which is mediated by the activity of SHP2.

As disclosed herein, the condition mediated by the activity of SHP2 may be cancer.

As disclosed herein, the condition mediated by the activity of SHP2 may be noonan syndrome, leopard syndrome, juvenile myelomonocytic leukemias, liver cancer, neuroblastoma, melanoma, squamous-cell carcinoma of the head and neck, acute myeloid leukemia, breast cancer, esophageal cancer, lung cancer, colon cancer, head cancer, gastric carcinoma, neuroblastoma, anaplastic large-cell lymphoma and glioblastoma.

Disclosed herein is at least one compound described herein or a pharmaceutically acceptable salt thereof for use as a medicament.

Disclosed herein is at least one compound described herein or a pharmaceutically acceptable salt thereof for use in the treatment of cancer.

A cancer in a mammal, which as disclosed herein may be treated by administering to a mammal in need of such treatment an effective amount of at least one compound described herein or a pharmaceutically acceptable salt thereof, can be selected from the group consisting of noonan syndrome, leopard syndrome, juvenile myelomonocytic leukemias, liver cancer, neuroblastoma, melanoma, squamous-cell carcinoma of the head and neck, acute myeloid leukemia, breast cancer, esophageal cancer, lung cancer, colon cancer, head cancer, gastric carcinoma, neuroblastoma, anaplastic large-cell lymphoma and glioblastoma.

The term "halogen", as used herein, unless otherwise indicated, means fluoro, chloro, bromo or iodo. The preferred halogen groups include F, Cl and Br. The terms "haloC₁₋₆alkyl", "haloC₂₋₆alkenyl", "haloC₂₋₆alkynyl" and "haloC₁₋₆alkoxy" mean a C₁₋₆alkyl, C₂₋₆alkenyl, C₂₋₆alkynyl or C₁₋₆alkoxy in which one or more (in particular, 1 to 3) hydrogen atoms have been replaced by halogen atoms, especially fluorine or chlorine atoms. In some embodiments, preferred are fluoroC₁₋₆alkyl, fluoroC₂₋₆alkenyl, fluoroC₂₋₆alkynyl and fluoroC₁₋₆alkoxy groups, in particular fluoroC₁₋₃alkyl, for example, CF₃, CHF₂, CH₂F, CH₂CH₂F, CH₂CHF₂, CH₂CF₃ and fluoroC₁₋₃alkoxy groups, for example, OCF₃, OCHF₂, OCH₂F, OCH₂CH₂F, OCH₂CHF₂ or OCH₂CF₃, and most especially CF₃, OCF₃ and OCHF₂.

As used herein, unless otherwise indicated, alkyl includes saturated monovalent hydrocarbon radicals having straight, branched or cyclic moieties. For example, alkyl radicals include methyl, ethyl, propyl, isopropyl, cyclopropyl, n-butyl, isobutyl, sec-butyl, t-butyl, cyclobutyl, n-pentyl, 3- (2-methyl) butyl, 2-pentyl, 2-methylbutyl, neopentyl, cyclopentyl, n- hexyl, 2-hexyl, 2-methylpentyl and cyclohexyl. Similarly, C₁₋₈, as in C₁₋₈alkyl is defined to identify the group as having 1, 2, 3, 4, 5, 6, 7 or 8 carbon atoms in a linear or branched arrangement.

Alkylene means a difunctional group obtained by removal of a hydrogen atom from an alkyl group that is defined above. For example, methylene (i.e., -CH₂-), ethylene (i.e., -CH₂-CH₂- or -CH(CH₃)-) and propylene (i.e., -CH₂-CH₂- CH₂-, -CH(-CH₂-CH₃)- or ―CH₂-CH(CH₃)-).

Alkenyl and alkynyl groups include straight, branched chain or cyclic alkenes and alkynes. Likewise, "C₂₋₈alkenyl" and "C₂₋₈alkynyl" means an alkenyl or alkynyl radicals having 2, 3, 4, 5, 6, 7 or 8 carbon atoms in a linear or branched arrangement.

Alkoxy radicals are oxygen ethers formed from the previously described straight, branched chain or cyclic alkyl groups.

The term "aryl", as used herein, unless otherwise indicated, refers to an unsubstituted or substituted mono- or polycyclic ring system containing carbon ring atoms. The preferred aryls are monocyclic or bicyclic 6-10 membered aromatic ring systems. Phenyl and naphthyl are preferred aryls. The most preferred aryl is phenyl.

The term "heterocyclic", as used herein, unless otherwise indicated, refers to unsubstituted and substituted mono- or polycyclic non-aromatic ring system containing one or more heteroatoms. Preferred heteroatoms include N, O, and S, including N-oxides, sulfur oxides, and dioxides. Preferably the ring is three to eight membered and is either fully saturated or has one or more degrees of unsaturation. Multiple degrees of substitution, preferably one, two or three, are included within the present definition.

Examples of such heterocyclic groups include azetidinyl, pyrrolidinyl, piperidinyl, piperazinyl, oxopiperazinyl, oxopiperidinyl, oxoazepinyl, azepinyl, tetrahydrofuranyl, dioxolanyl, tetrahydroimidazolyl, tetrahydrothiazolyl, tetrahydrooxazolyl, tetrahydropyranyl, morpholinyl, thiomorpholinyl, thiamorpholinyl sulfoxide, thiamorpholinyl sulfone and oxadiazolyl.

The term "heteroaryl", as used herein, unless otherwise indicated, represents an aromatic ring system containing carbon(s) and at least one heteroatom. Heteroaryl may be monocyclic or polycyclic, substituted or unsubstituted. A monocyclic heteroaryl group may have 1 to 4 heteroatoms in the ring, while a polycyclic heteroaryl may contain 1 to 10 hetero atoms. A polycyclic heteroaryl ring may contain fused, spiro or bridged ring junction, for example, bicyclic heteroaryl is a polycyclic heteroaryl. Bicyclic heteroaryl rings may contain from 8 to 12 member atoms. Monocyclic heteroaryl rings may contain from 5 to 8 member atoms (carbons and heteroatoms). Examples of heteroaryl groups include thienyl, furanyl, imidazolyl, isoxazolyl, oxazolyl, pyrazolyl, pyrrolyl, thiazolyl, thiadiazolyl, triazolyl, pyridyl, pyridazinyl, indolyl, azaindolyl, indazolyl, benzimidazolyl, benzofuranyl, benzothienyl, benzisoxazolyl, benzoxazolyl, benzopyrazolyl, benzothiazolyl, benzothiadiazolyl, benzotriazolyl adeninyl, quinolinyl or isoquinolinyl.

The term "cycloalkyl" refers to a substituted or unsubstituted monocyclic, bicyclic or polycyclic non-aromatic saturated ring, which optionally includes an alkylene linker through which the cycloalkyl may be attached. Exemplary " cycloalkyl" groups include cyclopropyl, cyclobutyl, cyclopentyl, cyclohexyl and so on.

The term "carbonyl, =O or oxo "refers to the group C(O).

Whenever the term "alkyl" or "aryl" or either of their prefix roots appear in a name of a substituent (e.g., aralkyl or dialkylamino) it shall be interpreted as including those limitations given above for "alkyl" and "aryl". Designated numbers of carbon atoms (e.g., C_{I-6}) shall refer independently to the number of carbon atoms in an alkyl moiety or to the alkyl portion of a larger substituent in which alkyl appears as its prefix root.

The term "composition", as used herein, is intended to encompass a product comprising the specified ingredients in the specified amounts, as well as any product which results, directly or indirectly, from combinations of the specified ingredients in the specified amounts. Accordingly, pharmaceutical compositions containing the compounds of the present invention as the active ingredient as well as methods of preparing the instant compounds are also part of the present invention. Furthermore, some of the crystalline forms for the compounds may exist as polymorphs and as such are intended to be included in the present invention. In addition, some of the compounds may form solvates with water (i.e., hydrates) or common organic solvents and such solvates are also intended to be encompassed within the scope of this invention.

The compounds of the present invention may also be present in the form of pharmaceutically acceptable salts. For use in medicine, the salts of the compounds of this invention refer to non-toxic "pharmaceutically acceptable salts". The pharmaceutically acceptable salt forms include pharmaceutically acceptable acidic/anionic or basic/cationic salts. The pharmaceutically acceptable acidic/anionic salt generally takes a form in which the basic nitrogen is protonated with an inorganic or organic acid. Representative organic or inorganic acids include hydrochloric, hydrobromic, hydriodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, palmoic, 2-naphthalenesulfonic, p-toluenesulfonic, cyclohexanesulfamic, salicylic, saccharinic or trifluoroacetic. Pharmaceutically acceptable basic/cationic salts include aluminum, calcium, chloroprocaine, choline, diethanolamine, ethylenediamine, lithium, magnesium, potassium, sodium and zinc.

Disclosed herein but not part of the present invention are the prodrugs of the compounds claimed. In general, such prodrugs will be functional derivatives of the compounds that are readily converted in vivo into the required compound. Thus, in the methods of treatment disclosed herein the term "administering" shall encompass the treatment of the various disorders described with the compound specifically disclosed or with a compound which may not be specifically disclosed, but which converts to the specified compound in vivo after administration to the subject. Conventional procedures for the selection and preparation of suitable prodrug derivatives are described, for example, in "Design of Prodrugs", ed. H. Bundgaard, Elsevier, 1985.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques know in the art as well as those methods set forth herein.

The present invention includes compounds which can contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof.

The above Formula I is shown without a definitive stereochemistry at certain positions. The present invention includes all stereoisomers of Formula I and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

When a tautomer of the compound of Formula (I) exists, the present invention includes any possible tautomers and pharmaceutically acceptable salts thereof, and mixtures thereof, except where specifically stated otherwise.

When the compound of Formula (I) and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. When the compound of the present invention is basic, its corresponding salt can be conveniently prepared from pharmaceutically acceptable non-toxic acids, including inorganic and organic acids. Since the compounds of Formula (I) are intended for pharmaceutical use they are preferably provided in substantially pure form, for example at least 60% pure, more suitably at least 75% pure, especially at least 98% pure (% are on a weight for weight basis).

The pharmaceutical compositions disclosed herein comprise a compound represented by Formula I (or a pharmaceutically acceptable salt thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds represented by Formula I,
or pharmaceutically acceptable salts thereof, of this invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). Thus, the pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a nonaqueous liquid, as an oil-in-water emulsion, or as a water-in- oil liquid emulsion. In addition to the common dosage forms set out above, the compound represented by Formula I, or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions as disclosed herein may include a pharmaceutically acceptable carrier and a compound, or a pharmaceutically acceptable salt, of Formula I. The compounds of Formula I, or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen. In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient. For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1mg to about 2g of the active ingredient, typically 25mg, 50mg, 100mg, 200mg, 300mg, 400mg, 500mg, 600mg, 800mg, or 1000mg.

Pharmaceutical compositions disclosed herein suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions as disclosed herein suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions as disclosed herein can be in a form suitable for topical use such as, for example, an aerosol, cream, ointment, lotion, dusting powder, or the like. Further, the compositions can be in a form suitable for use in transdermal devices. These formulations may be prepared, utilizing a compound represented by Formula I of this invention, or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency. Pharmaceutical compositions disclosed herein can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including antioxidants) and the like. Furthermore, other adjuvants can be included to render the formulation isotonic with the blood of the intended recipient. Compositions containing a compound described by Formula I, or pharmaceutically acceptable salts thereof, may also be prepared in powder or liquid concentrate form.

Generally, dosage levels on the order of from about 0.01 mg/kg to about 150mg/kg of body weight per day are useful in the treatment of the above-indicated conditions, or alternatively about 0.5mg to about 7g per patient per day. For example, inflammation, cancer, psoriasis, allergy/asthma, disease and conditions of the immune system, disease and conditions of the central nervous system (CNS), may be effectively treated by the administration of from about 0.01 to 50mg of the compound per kilogram of body weight per day, or alternatively about 0.5mg to about 3.5g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

These and other aspects will become apparent from the following written description of the invention.

### Examples

The following Examples are provided to better illustrate the present invention. All parts and percentages are by weight and all temperatures are degrees Celsius, unless explicitly stated otherwise. The following abbreviations have been used in the examples:
DAST: Diethylaminosulfur trifluoride;
DCM: Dichloromethane;
DIEA: N,N-Diisopropylethylamine;
DMF: N,N-Dimethylformamide;
DMSO: Dimethyl sulfoxide;
EA: Ethyl acetate;
EtOH: Ethanol;
NMP: N-methyl-2-pyrrolidone;
TEA: Triethylamine;
THF: Tetrahydrofuran
TFA: Trifluoroacetic acid;
Xantphos: Dimethylbisdiphenylphosphinoxanthene;
min: Minute;
rt or RT: room temperature;
TLC: Thin layer chromatography;
Pre-TLC: Preparation by thin layer chromatography.

### Example 1 Synthesis of compound 1 (for reference only)

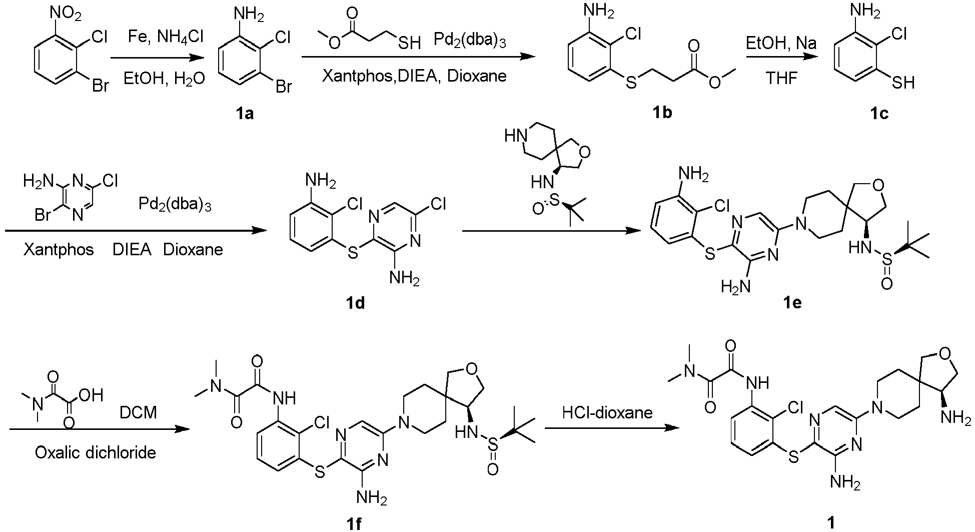

A mixture of 1-bromo-2-chloro-3-nitrobenzene (36.61g, 154.83mmol), iron powder (43.35g, 774.16mmol), NH₄Cl (8.28g, 154.83mmol), EtOH (100mL) and H₂O (50mL) was heated to 60°C for 4 hours, then cooled to 10°C. The reaction mixture was filtered through a pad of Celite and the filtrate was concentrated to remove the EtOH. The residual solution was extracted with EA (100mL×2). The combined organic extracts were washed with brine (100mL), dried over anhydrous Na₂SO₄ and concentrated under reduced pressure to afford the compound **1a** (30.01g,93.88%). MS: 206 (M+H)⁺.

A mixture of the compound **1a** (30.00g, 0.15mol), methyl 3-mercaptopropanoate (27.60g, 0.23mol), Pd₂(dba)₃ (1.37g, 1.5mmol), Xantphos (1.73g, 3.00mmol), DIEA (38.75g, 0.30mol) in dioxane (200mL) was stirred at 95°C under N₂ for 18 hours. After completion of the reaction, the reaction mixture was concentrated under reduced pressure and purified by column chromatography to afford the compound **1b** (10.00g, 27.13%). MS: 246 (M+H)⁺.

A mixture of Na (1.22g, 52.90mmol) and EtOH (25mL) was stirred at 20°C until the Na dissolved completely. The compound **1b** (10.00g, 40.70mmol) in THF (30mL) was added dropwise at -30°C~-20°C, then the mixture was stirred at 20°C for 3.5 hours, and concentrated under reduced pressure. The residue was added water (50mL), extracted with EA (50mL×2). The water phase was adjusted pH=2~3 with HCl solution (1mol/L) and extracted with EA (50mL×2). The combined organic extracts were washed with brine (50mL), dried over anhydrous Na₂SO₄ and filtered and concentrated under reduced pressure to afford the compound **1c** (6.02g, 92.66%). MS: 160 (M+H)⁺.

A mixture of the compound **1c** (6.02g, 37.71mol), 3-bromo-6-chloropyrazin-2-amine (7.86g, 37.71mol), Pd₂(dba)₃ (0.35g, 0.38mmol), Xantphos (0.43g, 0.75mmol), DIEA (9.74g, 75.42mol) in dioxane (70mL) was stirred at 95°C under N₂ for 17 hours. After completion of the reaction, the mixture was filtered and the filtrate was concentrated under reduced pressure. The residue was added EA (50mL) and stirred for 0.5 hour, then filtered to afford the compound **1d** (8.45, 78.03%). MS: 287 (M+H)⁺.

A solution of the compound **1d** (0.81g, 2.82mmol) , (R)-2-methyl-N-((S)-2-oxa-8-azaspiro [4.5]decan-4-yl)propane-2-sulfinamide (TFA salt, 1.26g, 3.38mmol) and K₂CO₃ (1.17g, 8.46mmol) in NMP (10mL) was stirred for 14 hours at 130°C. After cooling to RT, the resulting residue was dissolved in EA (100mL), washed with H₂O (30mL), dried over anhydrous Na₂SO₄, filtered and the volatiles were removed under reduced pressure and purified by column chromatography to afford the compound **1e** (0.55g, 38%). MS: 511(M+H)⁺.

Oxalyl chloride was added dropwise to a solution of 2-(dimethylamino)-2-oxoacetic acid (0.26 g, 2.15 mmol) in DCM (10mL),and stirred at RT for 2 hours, the volatiles were removed under reduced pressure, the residue was dissolved in DCM (10mL) and added dropwise to a solution of the compound **1e** (0.55g, 1.08mmol) in DCM (10mL), after completion of the reaction, the reaction mixture was quenched by addition of ice-water (20mL), washed with brine, dried over anhydrous Na₂SO₄, filtered and the volatiles were removed under reduced pressure and purified by column chromatography to afford the compound **1f** (0.42g, 64%). MS: 610(M+H)⁺.

The compound **1f** (0.41g, 0.67mmol) and HCl (4M in dioxane, 5mL) in DCM (10mL) was stirred for 20 min at 40°C. After cooling to RT, HCl (1M in H₂O) was added and the resulting aqueous mixture was extracted with DCM. The aqueous phase was basified with NH₄OH (28% in H₂O) until pH to 12 and extracted with DCM (3×20mL). The combined organic layers were washed with brine, dried over anhydrous Na₂SO₄, filtered, concentrated under reduced pressure, and purified by column chromatography to afford the compound 1 (100mg, 32%). MS: 506(M+H)⁺.

¹HNMR (DMSO-d6, 400MHz): δ 7.63 (s, 1H), 7.43-7.45 (d, 1H), 7.21-7.23 (d, 1H), 6.50-6.52 (d, 1H), 6.11 (s, 1H), 4.00-4.05 (m, 4H), 3.93-3.96 (dd, 2H), 2.93-3.29 (m, 4H), 1.54-1.98 (m, 4H).

### Example 2 Synthesis of compound 2 (for reference only)

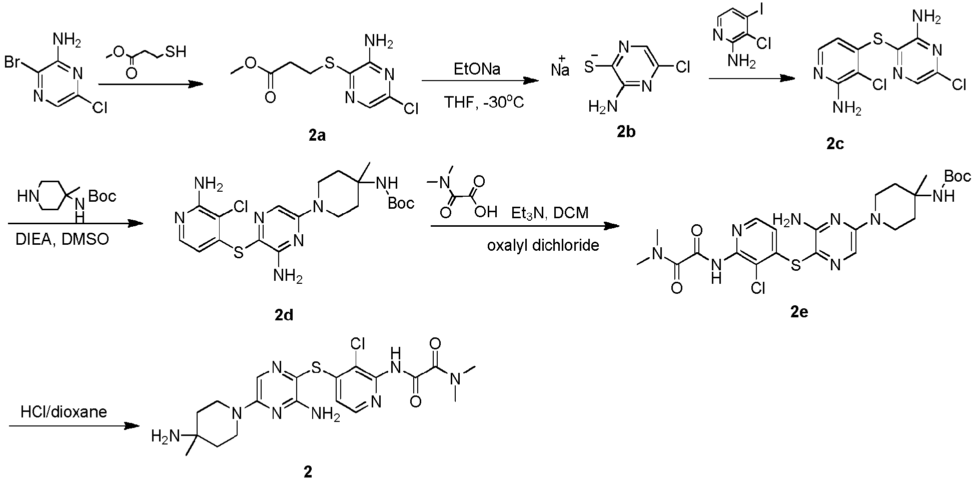

A mixture of 3-bromo-6-chloropyrazin-2-amine (20.02g, 96.05mmol), methyl 3-mercaptopropanoate (11.53g, 96.05mmol), DIEA (24.83g, 192.10mmol), Pd(OAc)₂ (0.30g, 1.34mmol), Xantphos (2.78g, 4.8mmol) in dixoane (200mL) was heated to 95°C under N₂ for 18 hours. The reaction mixture was filtered, concentrated under reduced pressure, and purified by column chromatography to afford the compound **2a** (18.83g, 79%). MS: 248(M+H)⁺.

A solution of the compound **2a** (18.33g, 76.02mmol) in THF (150mL) was cooled to - 30°C. Sodium ethoxide (6.72g, 98.82mmol) in ethanol (100mL) was added dropwise. The resulting mixture was stirred at -30 °C for 1 hours, then warmed to 25°C and stirred for another 2 hours. The volatiles were removed under reduced pressure and dissolved in DCM (100mL), the precipitate was filtered to give the compound **2b** as a brown solid (13.82g, 99%). MS: 162(M+H)⁺.

A mixture of the compound **2b** (9.98g, 54.36mmol), 3-chloro-4-iodopyridin-2-amine (13.83g, 54.36mmol), DIEA (14.04g, 108.72mmol), Pd₂(dba)₃ (1.00g, 1.09mmol), Xantphos (1.00g, 1.73mmol) in dioxane (200mL) was heated to 95°C under N₂ for 18 hours. The reaction mixture was filtered and concentrated under reduced pressure, the residue was dissolved in DCM (100mL), the precipitate was filtered to afford the compound **2c** as a brown solid (14.62g, 93.72%). MS: 288(M+H)⁺.

A mixture of the compound **2c** (2.31g, 8.05mmol), tert-butyl (4-methylpiperidin-4-yl)carbamate (3.45g, 16.10mmol), DIEA (3.12g, 24.15mmol) in DMSO (50mL) was stirred at 100°C for 3 hours. Water (100mL) was added to the reaction mixture and the precipitate was filtered to give the compound **2d** as a brown solid (2.19g, 58%). MS: 466(M+H)⁺.

To a solution of 2-(dimethylamino)-2-oxoacetic acid (350mg, 3mmol) in DCM (10mL) was added oxalyl dichloride (760mg, 6mmol) and DMF (2 drops). The resulting mixture was stirred at 20°C for 1 hour. The volatiles were removed under reduced pressure and the residue was dissolved in DCM (10mL), then the mixture was added to the solution of the compound **2d** (460mg, 1mmol) and TEA (1mL) in DCM (10mL). After completion of the reaction, the reaction mixture was concentrated in vacuo, the residue was purified by Pre-TLC to afford the compound **2e** as a yellow solid (142mg, 25%). MS: 565(M+H)⁺.

A mixture of the compound **2e** (142mg, 0.25mmol) in HCl/dioxane (10mL, 4M) was stirred for 1 hour. The precipitate was filtered to afford the compound **2** as a brown solid (70mg, 60%). MS: 465(M+H)⁺.

¹HNMR (DMSO-d6, 400MHz): δ 8.45-8.35 (m,2H), 8.06 (s, 1H), 7.71 (s, 1H), 6.45 (s, 1H), 4.06 (s, 6H), 3.38 (t, 2H), 2.93 (t, 2H), 2.86 (s, 2H), 1.84-1.71 (m, 4H), 1.39 (s, 3H).

### Example 3 Synthesis of compound 3 (for reference only)

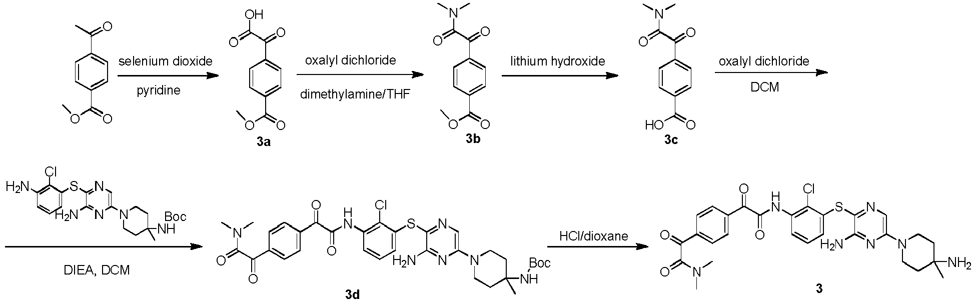

A mixture of methyl 4-acetylbenzoate (7.01g, 40.00mmol), selenium dioxide (8.95g, 80.00mmol) and pyridine (50mL) was heated to 100°C for 4 hours. Hydrochloric acid (70mL, 1M) was added to the reaction mixture to adjust pH=3, the aqueous solution was extracted with EA (70mL). The combined organic extracts were dried over anhydrous Na₂SO₄, concentrated in vacuo to afford the compound **3a** as a brown solid (6.12g, 74%). MS: m/z 207(M-H)⁻.

To a solution of the compound **3a** (2.51g, 12.07mmol) in DCM (30mL) was added oxalyl dichloride (15mL) and DMF (2 drops). The resulting mixture was stirred at 20°C for 1 hour. The volatiles were removed under reduced pressure and the residue was dissolved in DCM (10mL), then the mixture was added to the solution of dimethylamine/THF (10mL, 2M). After completion of the reaction, the reaction mixture was concentrated to afford the compound **3b** as a brown solid (2.35g, 83%). MS: 236(M+H)⁺.

A mixture of the compound **3b** (0.91g, 4.11mmol), lithium hydroxide monohydrate (0.85g, 20.55mmol), H₂O (10mL) in methanol (50mL) was stirred at 25°C for 1 hour. The volatiles were removed under reduced pressure and the residue was dissolved in hydrochloric acid (25mL, 1M), the aqueous solution was extracted with EA (20mL×2). The combined organic extracts were washed with brine (50mL), dried over anhydrous Na₂SO₄, filtered and the volatiles were removed under reduced pressure to afford the compound **3c** as a brown solid (0.85g, 93%). MS: 222(M+H)⁺.

To a solution of the compound **3c** (0.85g, 3.85mmol) in DCM (10mL) was added oxalyl dichloride (8mL) and DMF (2 drops). The resulting mixture was stirred at 20°C for 1 hour. The volatiles were removed under reduced pressure and the residue was dissolved in DCM (10mL), then the mixture was added to the solution of tert-butyl (1-(6-amino-5-((3-amino-2-chlorophenyl)thio)pyrazin-2-yl)-4-methylpiperidin-4-yl)carbamate (200mg, 0.43mmol) and DIEA (5mL) in DCM (10mL). After completion of the reaction, the reaction mixture was concentrated in vacuo, the residue was purified by Pre-TLC to afford the compound **3d** as a yellow solid (170mg, 57 %). MS: 696(M+H)⁺.

To a solution of HCl/dioxane (1mL, 4M) was added the compound **3d** (170mg, 0.24mmol), the resulting mixture was stirred at 25°C for 1 hour and the precipitate was filtered to give the crude product (107mg).The crude product was purified by Pre-TLC to afford the compound **3** as a brown solid (35mg, 24%). MS: 596(M+H)⁺.

¹HNMR (DMSO-d6, 400MHz): δ 10.44 (s, 1H), 8.39 (s, 1H), 8.18 (d, 2H), 8.02 (d, 2H), 7.68 (s, 1H), 7.36 (d, 1H), 7.26 (t, 1H), 6.58 (d, 1H), 6.18 (s, 1H), 4.03 (d, 2H), 3.37 (d, 2H), 3.04 (s, 3H), 2.90 (s, 3H), 1.83-1.71 (m, 4H), 1.39 (s, 3H).

### Example 4 Synthesis of compound 4 (for reference only)

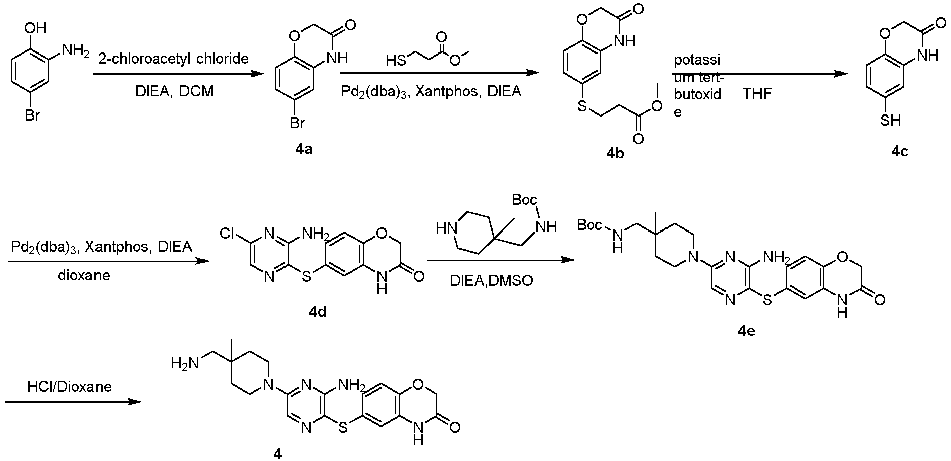

A mixture of 2-amino-4-bromophenol (10.15g, 354.29mmol), 2-chloroacetyl chloride (7.35g, 65.15mmol) and DCM (150mL) was cooled to 0°C. DIEA (35.06g, 271.45mmol) was added dropwise, the resulting mixture was stirred at 20°C for 5 hours. The reaction mixture was concentrated in vacuo and H₂O (70mL) was added, the precipitate was filtered to afford the compound **4a** as a red solid (7.61g,62%). MS: 228(M+H)⁺.

A solution of the compound **4a** (7.51g,33.09mmol), methyl 3-mercaptopropanoate (5.16g,43.02mmol), DIEA (8.55g,62.18mmol),Pd₂(dba)₃ (0.40g, 0.44mmol), Xantphos (0.40g, 0.69mmol) in dioxane (100mL) was heated to 100°C under N₂ for 8 hours. The reaction mixture was filtered and concentrated under reduced pressure, the residue was purified by column chromatography to afford the compound **4b** (5.12g, 58%). MS: 268(M+H)⁺.

A solution of the compound **4b** (3.75g, 14.04mmol) in THF (50mL) was cooled to - 70°C, potassium tert-butoxide/THF (28mL, 28.08mmol) was added dropwise, the resulting mixture was stirred at -70 °C for 0.5 hour. Hydrochloric acid (15mL, 1mol/L) was added to the reaction mixture, the aqueous solution was extracted with EA (20mL×2). The combined organic extracts were washed with brine (50mL), dried over anhydrous Na₂SO₄, filtered and the volatiles were removed under reduced pressure to afford the compound **4c** as a red solid (1.57g, 62%). MS: 182(M+H)⁺.

A mixture of the compound **4c** (3.22g, 17.79mmol), 3-bromo-6-chloropyrazin-2-amine (3.68g, 17.79mmol) , DIEA (4.60g, 35.58mmol), Pd₂(dba)₃ (0.15g, 0.16mmol), Xantphos (0.15g, 0.26mmol) in dioxane (30mL) was heated to 100°C under N₂ for 18 hours. The reaction mixture was filtered and concentrated under reduced pressure, the residue was dissolved in the solution of Hexane (10mL) and EA (10mL), the precipitate was filtered to afford the compound **4d** as a brown solid (2.88g, 52%). MS: 309(M+H)⁺.

A mixture of the compound **4d** (0.35g, 1.13mmol), tert-butyl ((4-methylpiperidin-4-yl)methyl)carbamate (0.39g, 1.70mmol), DIEA (0.36g, 2.83mmol) in DMSO (10mL) was stirred at 100°C for 2 hours. H₂O (10mL) was added to the reaction mixture, the aqueous solution was extracted with EA (10mL×2). The combined organic extracts were washed with brine (50mL), dried over anhydrous Na₂SO₄, filtered and the volatiles were removed under reduced pressure. The residue was purified by column chromatography to afford the compound **4e** (0.15g, 27%). MS: 501 (M+H)⁺.

To a solution of HCl/Dixoane (1mL, 4mol/L) was added the compound **4e** (150mg, 0.30mmol). The resulting mixture was stirred at 25°C for 1 hour and the precipitate was filtered to afford the compound **4** as a white solid (60mg, 50%). MS: 401 (M+H)⁺.

¹HNMR(DMSO-d6, 400MHz): δ 7.5 (s, 1H), 6.87 (d, 1H), 6.75 (d, 1H), 6.73 (s, 1H), 6.00 (s, 1H), 4.52 (s, 2H), 3.78-3.74 (m, 2H), 3.30-3.27 (m, 2H), 2.39 (s, 2H), 1.43-1.39 (m, 2H), 1.27-1.25 (m, 2H), 0.91 (s, 3H).

### Example 5 Synthesis of compound 5 (for reference only)

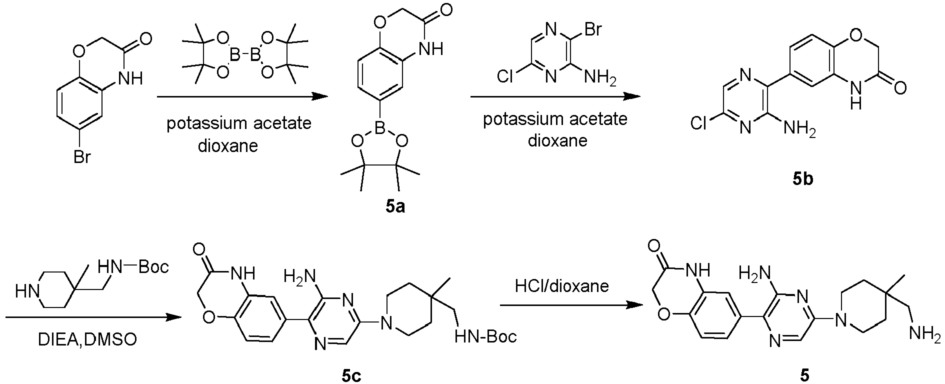

A solution of the compound **4a** (3.33g, 14.67mmol), 4,4,4',4',5,5,5',5'-octamethyl-2,2'-bi(1,3,2-dioxaborolane) (4.48g, 17.61mmol), potassium acetate (2.88g, 29.34mmol), Pd(dppf)Cl₂ (0.15g, 0.20mmol) in dioxane (40mL) was heated to 100°C under N₂ for 24 hours. The reaction mixture was filtered and concentrated under reduced pressure, the residue was purified by column chromatography to afford the compound **5a** (2.25g,56%). MS: 276(M+H)⁺.

A solution of the compound **5a** (2.20g, 8.00mmol), 3-bromo-5-chloropyrazin-2-amine (1.54g,7.27mmol), potassium acetate (2.00g, 14.54mmol), Pd(dppf)Cl₂ (0.15g, 0.20mmol), H₂O (2mL) in dioxane (40mL) was heated to 75°C under N₂ for 4 hours. Hexane (50mL) was added, the precipitate was filtered to afford the compound **5b** as a brown solid (0.79g, 36%). MS: 277(M+H)⁺.

A mixture of the compound **5b** (230mg, 0.83mmol), tert-butyl ((4-methylpiperidin-4-yl)methyl)carbamate (0.29g, 1.25mmol), DIEA (0.43g, 3.33mmol) in DMSO (10mL) was stirred at 100°C for 18 hours. H₂O (20mL) was added to the reaction mixture, the aqueous solution was extracted with EA (20mL×2). The combined organic extracts were washed with brine (20mL), dried over anhydrous Na₂SO₄, filtered and the volatiles were removed under reduced pressure, the residue was purified by column chromatography to afford the compound **5c** 157mg, 40%). MS: 469(M+H)⁺.

To a solution of HCl/dioxane (1mL, 4M) was added the compound **5c** (150mg, 0.34mmol). the resulting mixture was stirred at 25°C for 1 hour and the precipitate was filtered to afford the compound 5 as a brown solid (72mg, 56%). MS: 369 (M+H)⁺.

¹HNMR(DMSO-d6, 400MHz): δ 7.55 (s, 1H), 7.21 (d, 1H), 7.17 (d, 1H), 7.15 (d, 1H), 4.57 (s, 2H), 3.77 (t, 2H), 3.30 (t, 2H), 2.73 (s, 2H), 1.53-1.38 (m, 4H), 1.07 (s, 3H).

### Example 6 Synthesis of compound 6 (for reference only)

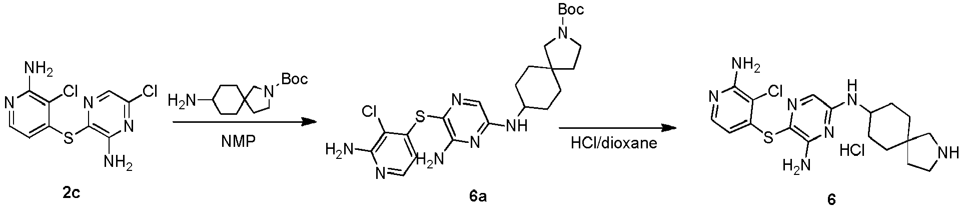

The oil bath was heated to 160 °C, a mixture of the compound 2c (80mg, 0.28mmol) in NMP (5mL) was stirred at 160°C, then tert-butyl 8-amino-2-azaspiro[4.5]decane-2-Carboxylate (190mg, 0.75mmol) was added. 1.5 hours later. The reaction mixture was cooled and water (40mL) was added, the resulting mixture was extracted with EA (20mL×2). The combined organic extracts were washed with brine (30mL), dried over anhydrous Na₂SO₄ filtered and the volatiles were removed under reduced pressure. The residue was purified by Pre-TLC to afford the compound **6a** (15mg, 10.59%). MS: 506 (M+H)⁺.

A mixture of the compound **6a** (39mg, 0.08mmol), HCl/dioxane (4mol/L, 1mL), and dioxane (2mL) was stirred at 20°C for 1 hour. The mixture was concentrated under reduced pressure, the residue was added EA (5mL) and stirred for 5 mins. The solid was filtered to afford the compound **6** as an HCl salt (30mg, 84.76%). MS: 203.7 (M+2H)²⁺.

### Example 7 Synthesis of compound 7

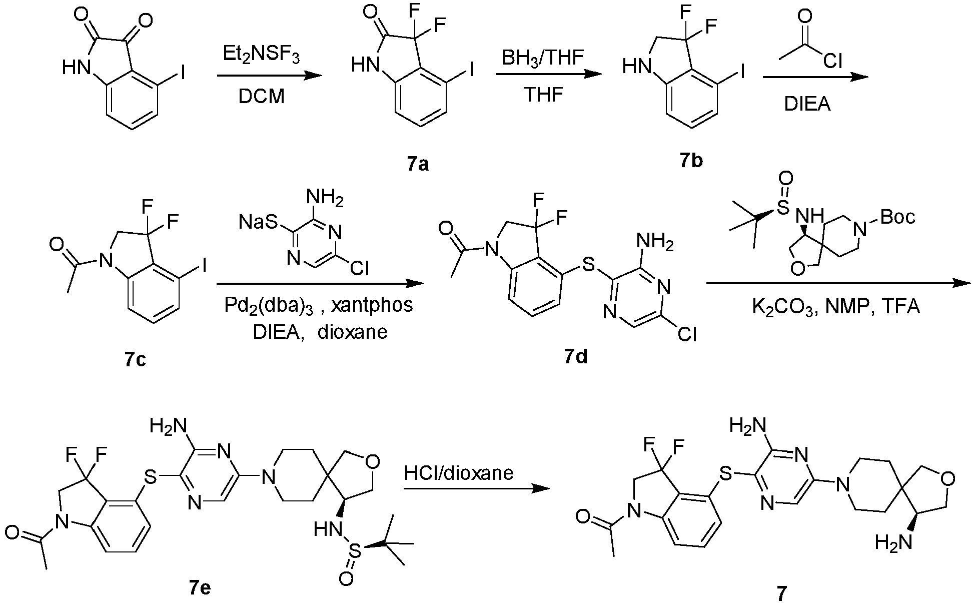

A solution of 4-iodoindoline-2,3-dione (35.00g, 128.19mmol) in DCM (300mL) was added diethylaminosulfurtrifluoride (62.00g, 387.12mmol) dropwise at 0°C. The mixture was stirred overnight at RT. After completion of the reaction, the mixture was drop wised into a solution of sodium hydrogen carbonate in water (85g/400mL), the two layers were separated and aqueous layer was extracted with DCM (150mL×2), The combined organic phases were dried over anhydrous Na₂SO₄ ,filtered and the volatiles were removed under reduced pressure. The residue was purified by column chromatography to afford the compound 7a as a off white solid (24.01g, 63.4%). MS: 296(M+H)⁺.

A solution of the compound 7a (24.00g, 81.34mmol) in THF (100mL) was stirred at 0°C, BH₃/THF (290mL, 1M) was added dropwise, the ice-water bath was removed after the adding was completed and the mixture was stirred at RT for another 1 hour, TLC showed the reaction was completed. The mixture was quenched with 10% citric acid solution (50mL) at 0°C,water (200mL) was added, extracted with EA (200mL×2), the organic phase was washed with brine (200mL×2), dried over anhydrous Na₂SO₄, filtered and the solvent was removed until about 300mL remained under reduced pressure, and compound 7b was used immediately for the next step without further purification. MS: 282(M+H)⁺.

A mixture of the compound 7b in EA obtained from the last step and DIEA (19mL, 161.71mmol) was added acetyl chloride (12mL, 169.68mmol) dropwise at 0°C. After completion of the reaction, water was added (100mL), the organic layer was separated and aqueous layer was extracted with EA (50mL). The combined organic layers were washed with brine (200mLx2), dried over anhydrous Na₂SO₄, filtered and the volatiles were removed under reduced pressure, and the resultant residue was washed with EA-hexane=1:10 (80mL) to afford the compound 7c (21.30g, 81.0%). MS: 324(M+H)⁺.

A mixture of the compound 7c (10.00g, 30.95mol), sodium 3-amino-5-chloropyrazine-2-thiolate (6.28g, 34.21mmol), Pd₂(dba)₃ (1.40g, 1.55mmol), Xantphos (1.80g, 3.11mmol) and DIEA (8.00g, 62.13mmol) in dioxane (120mL) was stirred at 70°C under N₂ for about 5 hours. The solution was cooled to RT and filtered, the filtrate was removed under reduced pressure, and the resultant residue was washed with EA (50mL), and filtrated to afford the compound **7d** (9.92g, 89.8%). MS: 357 (M+H)⁺.

A mixture of (S)-tert-butyl 4-((R)-1,1-dimethylethylsulfinamido)-2-oxa-8-azaspiro[4.5]decane-8-carboxylate (508mg, 1.41mmol) and TFA (1mL) in DCM (5mL)was stirred at RT for 1 hour, the solvent was removed under reduced pressure, the resultant residue was added K₂CO₃ (0.81g, 5.86mmol), NMP (5mL)and stirred for 5 min, then the compound **7d** (250mg, 0.70mmol) was added, the mixture was heated to 75°C for 2 hours, cooled and concentrated under reduced pressure, the residue was purified by column chromatography to afford the compound **7e** (84mg, 20.7%). MS: 581(M+H)⁺.

A solution of the compound **7e** (185mg, 0.32mmol) in 3mL of dioxane was added HCl/dioxane (1.2mL, 4M). The mixture was stirred at RT. After completion of the reaction, the solution was removed under reduced pressure and the residue was washed with EA (5mL) to afford the compound 7 (136mg, 82.8%) as an HCI salt. MS: 477 (M+H)⁺.

### Example 8 Synthesis of compound 8

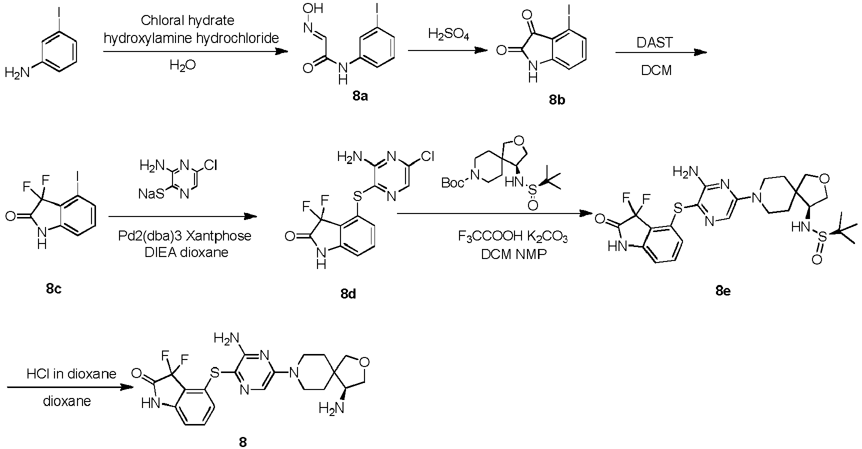

Chloral hydrate (50.02g) and Na₂SO₄ (350.21g) were dissolved in water (700mL) in a 3 L beaker and warmed to 35°C. A warm solution of the appropriate commercial aniline derivative (60.45g, 0.276mol) in water (200mL), and an aqueous solution of concentrated HCI (30mL) was added (a white precipitate of the amine sulfate was formed), followed by a warm solution of hydroxylamine hydrochloride (61.18g, 0.88mol) in water (275mL). The mixture was stirred by hand and heated on a hot plate (a thick paste formed at 75-70 °C) at 80-90 °C for 2 hours and then allowed to cool for 1 hour, by which time the temperature had fallen to 50°C and filtered. The pale cream product was washed by stirring with water (1L) and filtered. Drying overnight at 40 °C gave the compound **8a** (69.74g,87%). MS: 291(M+H)⁺⁻.

Sulfuric acid (1L) was heated in a 3L beaker on a hot plate to 60°C and then removed. The compound **8a** was added in portion with stirring over 30 mins so that the temperature did not exceed 65°C. The mixture was then heated to 80°C for 15 mins, allowed to cool to 70°C, and cooled on ice. The solution was poured on to crushed ice (5L) and left to stand for 1 hour before filtering the orange-red precipitate. The product was washed by stirring with water (400mL) and filtered to give a mixture of the compound **8b** and 6-iodoindoline-2,3-dione. The crude product was dissolved in a solution of NaOH (20g) in water (200mL) at 60 °C and then acidified with acetic acid to pH=4.9. After standing 0.5 hour and cooling to 35°C, the compound 8b precipitate was filtered and washed with water (50mL) (38.37g, 59%). MS: 274(M+H)⁺.

A mixture of the compound **8b** (2.31g, 8.46mmol), DAST (4.10g, 25.43mmol) and DCM (100mL) was stirred at RT for 24 hours. The mixture was quenched by addition of NaHCO₃ solution and then filtered to give the crude product, the crude product was washed with Hexane to afford the compound **8c** (2.14g,86%). MS: 294(M-H)⁻.

A mixture of the compound **8c** (1.01g, 3.42mmol), Pd₂(dba)₃ (100mg, 0.34mmol), Xantphos (100mg, 0.34mmol), DIEA (883mg, 6.84mmol) and dioxane (30mL) was stirred at 80°C for 30 mins. sodium 3-amino-5-chloropyrazine-2-thiolate (628mg, 3.42mmol) was added then stirred for another 3 hours at 80°C. The mixture was cooled and the volatiles were removed under reduced pressure, the residue was purified by column chromatography to afford the compound **8d** (545mg, 48%). MS: 329(M+H)⁺.

A mixture of (S)-tert-butyl 4-((R)-1,1-dimethylethylsulfinamido)-2-oxa-8-azaspiro[4.5]decane-8-carboxylate (1.18g,3.27mmol), TFA (5mL) and DCM (20mL) was stirred at room temperature for 2 hours. The volatiles were removed under reduced pressure. The residue was added the compound **8d** (542mg, 1.65mmol), K₂CO₃ (1.82g, 13.20mmol) and NMP (12mL) and stirred at 80°C for 10 hours. It was quenched by addition of water (40mL), then exacted with EA (30mL×5), the combined organic phases were washed with brine (100mL), dried over anhydrous Na₂SO₄, filtered and the volatiles were removed under reduced pressure, the residue was purified by column chromatography to afford the compound **8e** (138mg, 15%). MS: 553(M+H)⁺.

The compound **8e** (138mg, 0.25mmol) was dissolved in dioxane (3mL) and stirred. A solution of HCI/dioxane (0.5mL, 4M) was added then the mixture was stirred at room temperature for 0.5 hour and the volatiles were removed under reduced pressure. The residue was washed with EA (10mL) and filtered to afford the compound 8 as a yellow solid, HCI salt. MS: 449(M+H)⁺.

### Example 9 Synthesis of compound 9

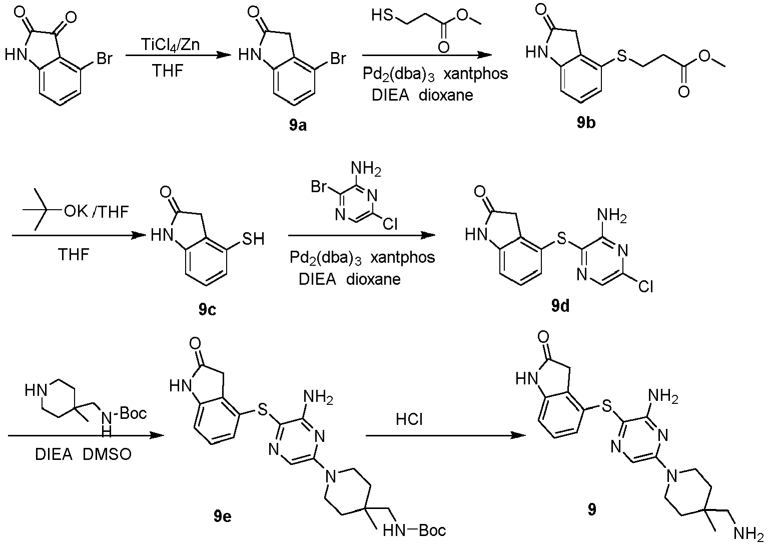

A mixture of Zn power (8.64g, 132.13mmol) and TiCI4 (12.60g, 66.42mmol) in THF (100mL) was stirred at 80°C for 2 hours, then it was cooled to RT, a solution of 4-bromoindoline-2,3-dione (5.01g, 22.16mmol) in THF (100mL) was added dropwise under N₂. After completion of the reaction, Hydrochloric acid solution (100mL, 3M) was added, the mixture was extracted with DCM (50mLx3), the organic phase was washed with brine (50mLx2), dried over anhydrous Na₂SO₄, filtered and the solvent was removed under reduced pressure, the residue was purified by column chromatography to afford the compound **9a** (2.63g, 56.0%). MS: 212(M+H)⁺.

A mixture of the compound **9a** (1.00g, 4.72mmol), methyl 3-mercaptopropanoate (1.13g, 9.40mmol), Pd₂(dba)₃ (0.15g, 0.16mmol), Xantphos (0.20g, 0.35mmol) and DIEA (1.23g, 9.52mmol) in dioxane (25mL) was stirred overnight at 100°C under N₂.the solvent was removed under reduced pressure, the residue was purified by column chromatography to afford the compound **9b** (0.73g, 61.5%). MS: 252(M+H)⁺.

A mixture of the compound **9b** (1.65g, 6.56mmol) in THF (50mL) was added t-BuOK/THF (15mL, 1M) dropwise at -70°C, After completion of the reaction, it was quenched with Hydrochloric acid solution (20mL, 1M) and extracted with EA (50mLx3), the organic phase was washed with brine (100mL×2), dried over anhydrous Na₂SO₄, filtered and the solvent was removed under reduced pressure, and the residue of the compound **9c** was used immediately for the next step without further purification (1.08g, 100%). MS: 166 (M+H)⁺.

A mixture of the compound **9c** (1.0g, 6.54mmol), 3-bromo-6-chloropyrazin-2-amine (1.37g, 6.57mmol), Pd₂(dba)₃ (0.31g, 0.34mmol), Xantphos (0.40g, 0.69mmol) and DIEA (1.70g, 13.16mmol) in dioxane (80mL) was stirred at 100°C under N₂ for 5 hours. The mixture was cooled to RT and filtered, the filtrate was removed under reduced pressure, the residue was purified by column chromatography to afford the compound **9d** (0.53g, 27.7%). MS: 293 (M+H)⁺.

A mixture of the compound **9d** (146mg, 0.50mmol), tert-butyl ((4-methylpiperidin-4-yl)methyl)carbamate (240mg, 1.05mmol) and DIEA (203mg, 1.57mmol) in DMSO (5mL) was stired at 80°C , after the reaction was completed, cooled to RT, water (20mL) was added, the mixture was extracted with EA (20mL×2), the organic phase was washed with brine (50mL×2) , dried over anhydrous Na₂SO₄, filtered and the solvent was removed under reduced pressure, the residue was purified by column chromatography to afford the compound **9e** (99mg, 40.8 %). MS: 485(M+H)⁺.

A solution of the compound **9e** (24mg, 0.049mmol) in 5mL of DCM was bubbled into HCI gas at RT. After completion of the reaction, water (20mL) was added, the mixture was washed with EA (20mL×2), then the aqueous layer was adjusted to pH=11, extracted with DCM (20mL×2), dried over anhydrous Na₂SO₄, filtered and the filtrate was removed under reduced pressure to afford the compound 9 (16mg, 84.9%). MS: 385(M+H)⁺.

### Example 10 Synthesis of compound 10

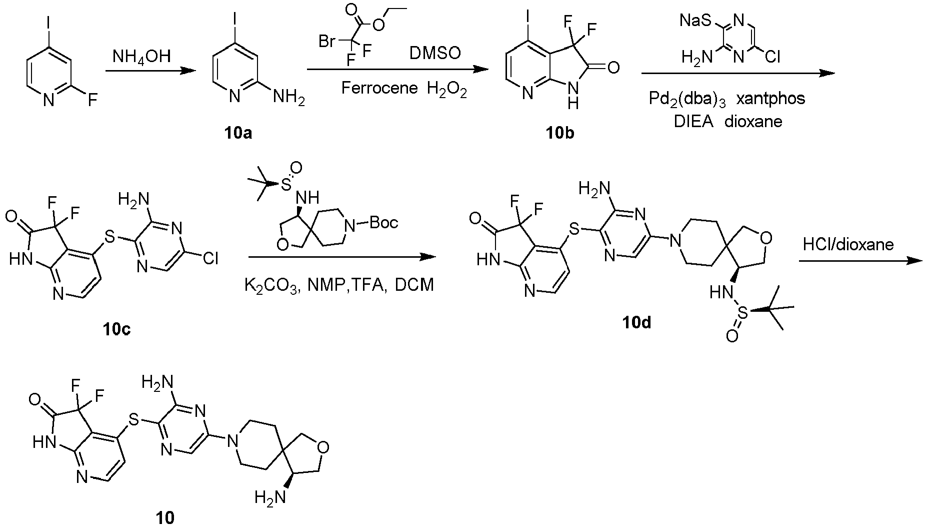

A mixture of 2-fluoro-4-iodopyridine (10.00g, 43.50mmol), ammonium hydroxide (10mL) in DMSO (20mL) was stirred at 100°C for 40 hours. H₂O (100mL) was added to the reaction mixture and the precipitate was filtered to afford the compound 10a as a brown solid (8.62g, 90%). MS: 221 (M+H)⁺.

To a solution of the compound **10a** (8.00g, 36.36mmol), ethyl 2-bromo-2,2-difluoroacetate (18.46g, 90.91mmol) and Ferrocene (0.68g, 3.64mmol) in DMSO (70mL) was added H₂O₂ (8mL) at -5°C. The resulting mixture was stirred at 25°C for 24 hours. H₂O (100mL) was added to the reaction mixture, the aqueous solution was extracted with EA (100mL×2). The combined organic extracts were washed with brine (50mL), dried over anhydrous Na₂SO₄, filtered and the volatiles were removed under reduced pressure in vacuo. The residue was purified by column chromatography to afford the compound **10b** as a yellow solid (3.41g, 32%). MS: 297(M+H)⁺.

A mixture of the compound **10b** (1.48g, 5.00mmol), sodium 3-amino-5-chloropyrazine-2-thiolate (0.92g, 5.00mmol) , DIEA (1.29g, 10.00mmol), Pd₂(dba)₃ (0.15g, 0.16mmol), Xantphos (0.15g, 0.26mmol) in dioxane (20mL) was heated to 95°C under N₂ for 18 hours. The reaction mixture was filtered and concentrated under reduced pressure, the residue was purified by column chromatography afford the compound **10c** (0.59g, 36%). MS: 330(M+H)⁺.

A mixture of (S)-tert-butyl 4-((R)-1,1-dimethylethylsulfinamido)-2-oxa-8-azaspiro[4.5]decane-8-carboxylate (361mg, 1.00mmol) , TFA (1mL) in DCM (5mL) was stirred at 25°C for 1 hour. The reaction mixture was concentrated under reduced pressure, the residue was dissolved in NMP (8mL), then the compound **10c** (330g, 1.00mmol), K₂CO₃ (1.10g, 8.00mmol) was added to mixture and stirred at 80°C for 1 hour. The reaction mixture was filtered and concentrated under reduced pressure, the residue was purified by Pre-TLC to afford the compound **10d** (80mg,14%). MS: 554(M+H)⁺.

To a solution of the compound **10d** (80mg, 0.14mmol) in DCM (10mL) was added HCI/dioxane (1mL, 4M). The resulting mixture was stirred at 25°C for 1 hour and the precipitate was filtered to afford the compound **10** as a brown solid (10mg, 16%). MS: 450 (M+H)⁺.

### Example 11 Synthesis of compound 11

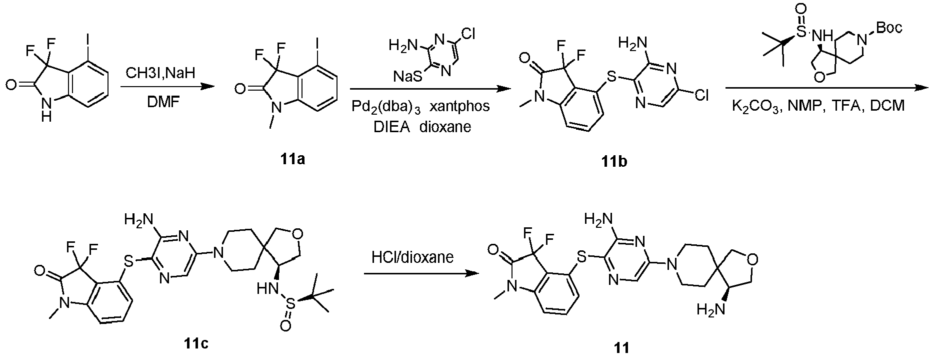

A mixture of the compound **7a** (2.30g, 7.80mmol), NaH (0.94g, 23.39mmol, 60%) in DMF (30mL) was stirred at 25°C for 0.5 hour. Methyl iodide (3.32g, 23.39mmol) was added to the reaction mixture and stirred at 25°C for 1 hour. Reaction was quenched with water (100mL), the aqueous solution was extracted with EA (100mL×2). The combined organic extracts were washed with brine (50mL), dried over anhydrous Na₂SO₄,filtered and the volatiles were removed under reduced pressure in vacuo. The residue was purified by column chromatography to afford the compound **11a** as a yellow solid (1.10g, 46%). MS: 310(M+H)⁺.

A mixture of the compound **11a** (1.10g, 3.56mmol), sodium 3-amino-5-chloropyrazine-2-thiolate (0.65g, 3.56mmol), DIEA (0.92g, 7.12mmol),Pd₂(dba)₃ (0.10g, 0.11mmol), Xantphos (0.10g, 0.18mmol) in dioxane (30mL) was heated to 95°C under N₂ for 18 hours. The reaction mixture was concentrated under reduced pressure, the residue was purified by column chromatography to afford the compound **11b** (1.06g, 87%). MS: 343(M+H)⁺.

A mixture of (S)-tert-butyl 4-((R)-1,1-dimethylethylsulfinamido)-2-oxa-8-azaspiro[4.5]decane-8-carboxylate (200mg, 0.55mmol), TFA (2mL) in DCM (10mL) was stirred at 25°C for 1 hour. The reaction mixture was concentrated under reduced pressure. The residue was dissolved in NMP (8mL), then the compound **11b** (190mg, 0.55mmol), K₂CO₃ (613mg, 4.44mmol) was added to mixture and stirred at 90°C for 24 hours. H₂O (50mL) was added to the reaction. The aqueous solution was extracted with EA (50mLx3), The combined organic extracts were washed with brine (50mL), dried over anhydrous Na₂SO₄, filtered and the volatiles were removed in vacuo, the residue was purified by Pre-TLC to afford the compound **11c** as a yellow solid (50mg, 16%). MS: 567(M+H)⁺.

To a solution of the compound **11c** (50mg, 0.14mmol) in DCM (5mL) was added HCI/dioxane (1mL, 4M). The resulting mixture was stirred at 25°C for 1 hour. The reaction mixture was concentrated under reduced pressure, the residue was purified by Pre-TLC to afford the compound **11** (3mg, 7%). MS: 463 (M+H)⁺.

### Example 12 Synthesis of compound 12

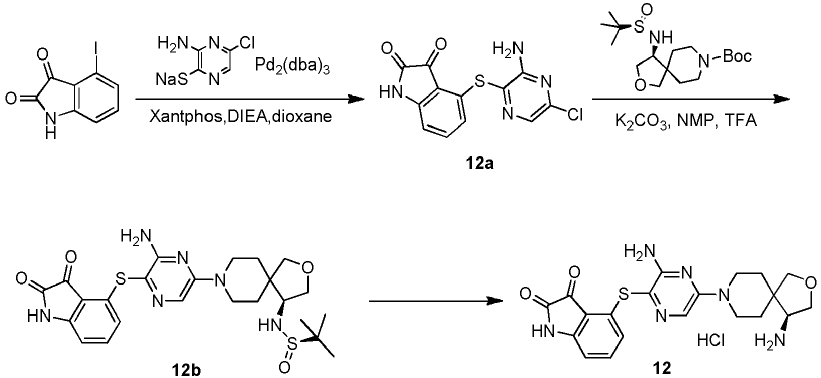

A mixture of 4-iodoindoline-2,3-dione (200mg, 0.73mmol), sodium 3-amino-5-chloropyrazine-2-thiolate (0.13g, 0.73mol), Pd₂(dba)₃ (20mg, 0.02mmol), Xantphos (20mg, 0.035mmol), DIEA (0.19g, 1.46mmol) in dioxane (10mL) was stirred at 95°C under N₂ for 2 hours. After completion of the reaction, the reaction mixture was concentrated under reduced pressure and the residue was purified by column chromatography to afford the compound **12a** (0.19g, 84.86%). MS: 307 (M+H)⁺.

A mixture of (S)-tert-butyl 4-((R)-1,1-dimethylethylsulfinamido)-2-oxa-8-azaspiro[4.5] decane-8-carboxylate (0.28g, 0.74mmol), TFA (1mL), and DCM (5mL) was stirred at 20°C for 1 hour. The mixture was concentrated under reduced pressure, and the residue was added DCM (10mL), concentrated under reduced pressure again. A mixture of the residue, K₂CO₃ (0.68g, 4.96mmol), the compound **12a** (0.19g, 0.62mmol) and NMP (5mL) was heated to 80°C for 18 hours. The reaction mixture was cooled and water (40mL) was added, the resulting mixture was extracted with EA (20mL×2). The combined organic extracts were washed with brine (30mL), dried over anhydrous Na₂SO₄ and evaporated to dryness. The residue was purified by Pre-TLC to afford the compound **12b** (18mg, 5.47 %). MS: 531 (M+H)⁺.

A mixture of the compound **12b** (39mg, 0.08mmol), HCI/dioxane (4mol/L, 1mL), and dioxane (2mL) was stirred at 20°C for 2 hours. The mixture was concentrated under reduced pressure, the residue was added EA (10mL) and stirred for 5 mins. The precipitate was filtered to afford the compound 12 (4mg, 25.47 %) as an HCI salt. MS: 427 (M+H)⁺.

### Example 13 Synthesis of compound 13

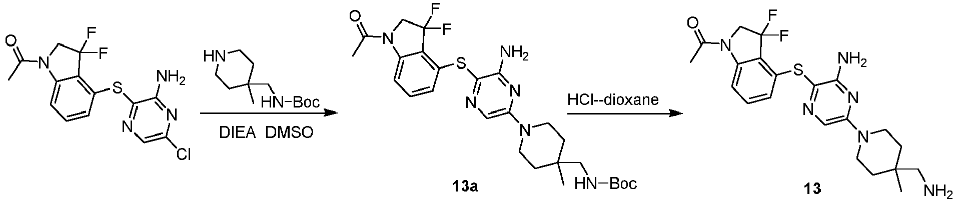

A mixture of the compound **7d** (80mg, 0.22mol), DIEA (101mg, 0.78mmol) and tert-butyl ((4-methylpiperidin-4-yl)methyl)carbamate (200mg, 0.88mmol) in DMSO (5mL) was stirred at 80°C , after the reaction was completed, the mixture was cooled to RT, water (20mL) was added, the mixture was extracted with EA (20mL×2), the organic phase was washed with brine (50mL×2) , dried over anhydrous Na₂SO₄, filtered and the solvent was removed under reduced pressure, the residue was purified by column chromatography to afford the compound **13a** (120mg, 100%). MS: 549(M+H)⁺.

A solution of the compound **13a** (120mg, 0.22mmol) in dioxane (4mL) was added HCI/dioxane (5mL, 4M). The mixture was stirred at RT under ultrasonic for about 5 mins. After completion of the reaction, the solution was removed under reduced pressure and the residue was washed with EA (5mL) to afford the compound **13** (85mg, 79.7%) as an HCI salt. MS: 449(M+H)⁺.

### Example 14 Synthesis of compound 14

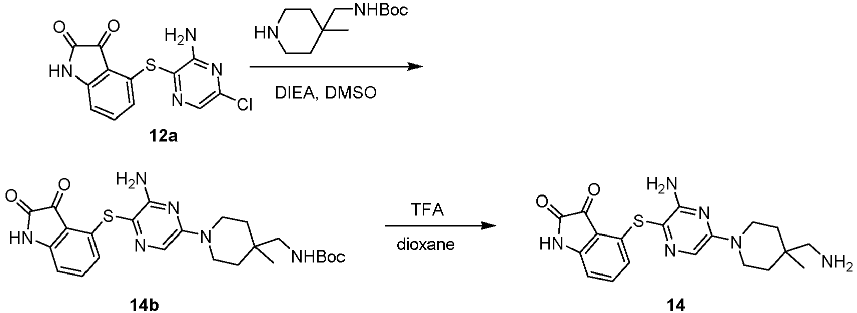

A mixture of the compound **12a** (0.11g, 0.36mmol), tert-butyl ((4-methylpiperidin-4-yl)methyl)Carbamate (0.25g, 1.08mmol), DIEA (93mg, 0.72mmol) and DMSO (10mL) was heated to 80°C for 17 hours. The reaction mixture was cooled and water (50mL) was added, the resulting mixture was extracted with EA (30mL×2). The combined organic extracts were washed with brine (60mL), dried over anhydrous Na₂SO₄, filtered and the solvent was removed under reduced pressure. The residue was purified by Pre-TLC to afford the compound **14b** (0.13g, 72.43%). MS: 499 (M+H)⁺.

A mixture of the compound **14b** (0.13g,0.26mmol), TFA (1mL) and DCM (5mL) was stirred at 20°C for 2 hours. The mixture was concentrated under reduced pressure, the residue was added EA (10mL), stirred for 5min. The precipitate was filtered to afford the compound **14** (25mg, 18.76%) as an TFA salt. MS: 399 (M+H)⁺.

| Ex No | Chemical Name | Structure | Physical Data (MS) (M+H)⁺ |
|---|---|---|---|
| 15 | 5-((2-amino-3-chloropyridin-4-yl)thio)-6-(4-(aminomethyl)-4-methylpiperidin-1-yl)pyrazin-2-amine (for reference only) | 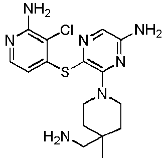 | 380 |
| 16 | N¹-(4-((3-amino-5-(4-(am inomethyl)-4-methylpiperidin-1-yl)pyrazin-2-yl)thio)-3-chloropyridin-2-yl)-N2, N2-dimethyloxalamide (for reference only) | 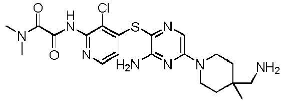 | 479 |
| 17 | tert-butyl ((1-(6-amino-5-((2-amino-3-chloropyridin-4-yl)thio)pyrazin-2-yl)-4-methylpiperidin-4-yl)methyl)carbamate (for reference only) | 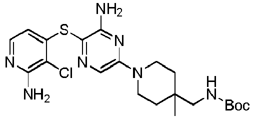 | 480 |
| 18 | (S)-N1-(4-((3-amino-5-(4-amino-2-oxa-8-azaspiro[4.5]decan-8-yl)pyrazin-2-yl)thio)-3-chloropyridin-2-yl)-N2, N2-dimethyloxalamide (for reference only) | 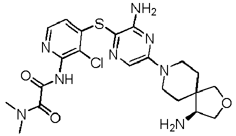 | 507 |
| 19 | (S)-N¹-(3-((3-amino-5-(4-amino-2-oxa-8-azaspiro[4.5]decan-8-yl)pyrazin-2-yl)thio)-2-chlorophenyl)-N²,N²-dimethyloxalamide (for reference only) | 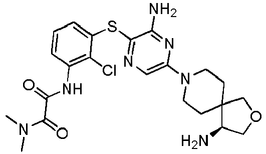 | 506 |
| 20 | N¹-(3-((3-amino-5-(4-(am inomethyl)-4-methylpiperidin-1-yl)pyrazin-2-yl)thio)-2-chlorophenyl)-N²,N²-dimethyloxalamide (for reference only) | 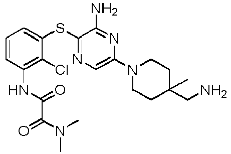 | 478 |
| 21 | N-(3-((3-amino-5-(4-(am inomethyl)-4-methylpiperidin-1-yl)pyrazin-2-yl)thio)-2-chlorophenyl)-3-(2-(dimethylamino)-2-oxoacetyl)benzamide (for reference only) | 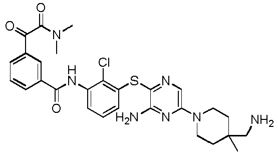 | 582 |
| 22 | 2-(3-(3-(3-((3-amino-5-(4-(am inomethyl)-4-methylpiperidin-1-yl)pyrazin-2-yl)thio)-2-chlorophenyl)ureido)phenyl)-N,N-dimethyl-2-oxoacetamide (for reference only) | 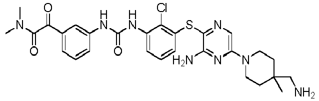 | 597 |
| 23 | 2-(4-(3-(3-((3-amino-5-(4-(aminomethyl)-4-methylpiperidin-1 -yl)pyrazin-2-yl)thio)-2-chlorophenyl)ureido)phenyl)-N,N-dimethyl-2-oxoacetamide (for reference only) | 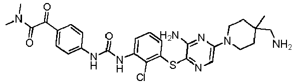 | 597 |
| 24 | 6-(4-(aminomethyl)-4-methylpiperidin-1-yl)-3-((3,4-dihydro-2H-benzo[b][1,4]oxazin-6-yl)thio)pyrazin-2-amine | 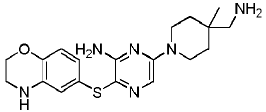 | 387 |
| 25 | tert-butyl (1-(6-amino-5-((2-amino-3-chloropyridin-4-yl)thio)pyrazin-2-yl)-4-methylpiperidin-4-yl)carbamate (for reference only) | 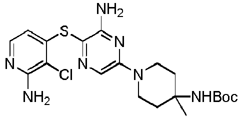 | 466 |
| 26 | tert-butyl (1-(5-((2-acrylamido-3-chloropyridin-4-yl)thio)-6-am inopyrazin-2-yl)-4-methylpiperidin-4-yl)carbamate (for reference only) | 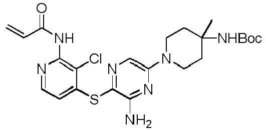 | 520 |
| 27 | tert-butyl (1-(6-amino-5-((3-chloro-2-(2-(dimethylam ino)-2-oxoacetam ido)pyridin-4-yl)thio)pyrazin-2-yl)-4-methylpiperidin-4-yl)carbamate (for reference only) | 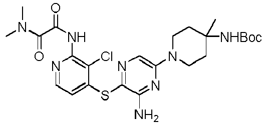 | 565 |
| 28 | tert-butyl (1-(6-amino-5-((2-chloro-3-(2-(dimethylamino)-2-oxoacetam ido)phenyl)thio)pyrazin -2-yl)-4-methylpiperidin-4-yl)carbamate (for reference only) | 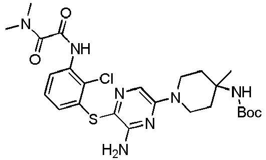 | 564 |
| 29 | N¹-(3-((3-amino-5-(4-amino-4-methylpiperidin-1-yl)pyrazin-2-yl)thio)-2-chlorophenyl)-N²,N²-dimethyloxalamide (for reference only) | 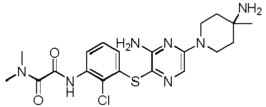 | 464 |
| 30 | tert-butyl (1-(6-amino-5-((3-amino-2-chlorophenyl)thio)pyrazin-2-yl)-4-methylpiperidin-4-yl)carbamate (for reference only) | 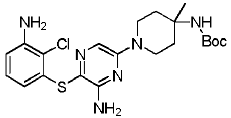 | 465 |
| 31 | N-(3-((3-amino-5-(4-amino-4-methylpiperidin-1-yl)pyrazin-2-yl)thio)-2-chlorophenyl)-2-oxo-2-(p-tolyl)acetamide (for reference only) | 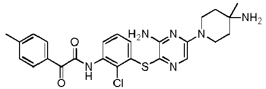 | 511 |
| 32 | tert-butyl (1-(5-((3-acrylamido-2-chlorophenyl)thio)-6-am inopyrazin-2-yl)-4-methylpiperidin-4-yl)carbamate (for reference only) | 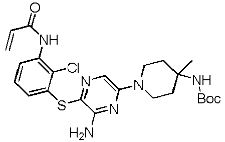 | 519 |
| 33 | 6-(3-amino-5-(4-amino-4-methylpiperidin-1-yl)pyrazin-2-yl)-2H-benzo[b][1,4]oxazin-3(4H)-one (for reference only) | 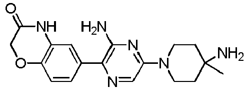 | 355 |
| 34 | N-(4-((3-amino-5-(4-amino-4-methylpiperidin-1-yl)pyrazin-2-yl)thio)-3-chloropyridin-2-yl)-4-(2-(dimethylamino)-2-oxoacetyl)benzamide (for reference only) | 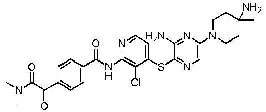 | 569 |
| 35 | N-(4-((3-amino-5-(4-(am inomethyl)-4-methylpiperidin-1-yl)pyrazin-2-yl)thio)-3-chloropyridin-2-yl)-4-(2-(dimethylamino)-2-oxoacetyl)benzamide (for reference only) | 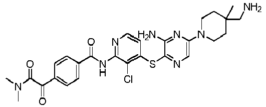 | 583 |
| 36 | N-(3-((3-amino-5-(4-(am inomethyl)-4-methylpiperidin-1-yl)pyrazin-2-yl)thio)-2-chlorophenyl)-4-(2-(dimethylamino)-2-oxoacetyl)benzamide (for reference only) | 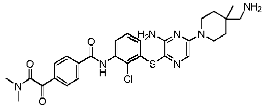 | 582 |
| 37 | 5-((2-amino-3-chloropyridin-4-yl)thio)-N2-cyclohexylpyrazine-2,6-diamine (for reference only) | 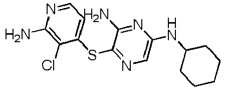 | 351 |
| 38 | (S)-8-(5-((1H-pyrrolo[2,3-b]pyridin-4-yl)thio)-6-aminopyrazin-2-yl)-2-oxa-8-azaspiro[4.5]decan-4-amine | 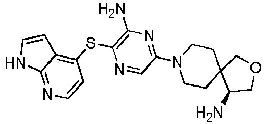 | 398 |
| 39 | (S)-8-(6-amino-5-((3,3-dimethylindolin-4-yl)thio)pyrazin-2-yl)-2-oxa-8-azaspiro[4.5]decan-4-amine | 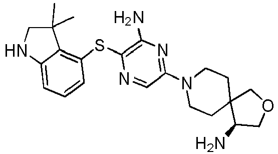 | 427 |
| 40 | (S)-8-(6-amino-5-((3-fluoro-1H-indol-4-yl)thio)pyrazin-2-yl)-2-oxa-8-azaspiro[4.5]decan-4-amine | 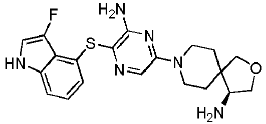 | 415 |
| 41 | 5-((2-amino-3-chloropyridin-4-yl)thio)-N2-(4-(aminomethyl)-4-methylcyclohexyl)pyrazine-2,6-diamine (for reference only) | 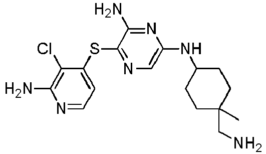 | 394 |
| 42 | (S)-8-(5-((1H-indol-4-yl)thio)-6-aminopyrazin-2-yl)-2-oxa-8-azaspiro[4.5]decan-4-amine | 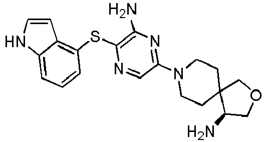 | 397 |
| 43 | (S)-1-(4-((3-amino-5-(4-amino-2-oxa-8-azaspiro[4.5]decan-8-yl)pyrazin-2-yl)thio)-1H-indol-1-yl)ethanone | 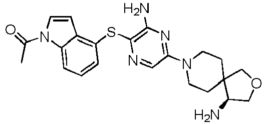 | 439 |
| 44 | 5-((2-amino-3-chloropyridin-4-yl)thio)-N2-(4-amino-4-methylcyclohexyl)pyrazine-2,6-diamine (for reference only) | 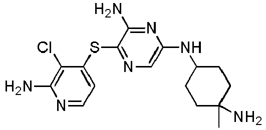 | 380 |
| 45 | (S)-6-((3-amino-5-(4-amino-2-oxa-8-azaspiro[4.5]decan-8-yl)pyrazin-2-yl)thio)-2H-benzo[b][1,4]oxazin-3(4H)-one | 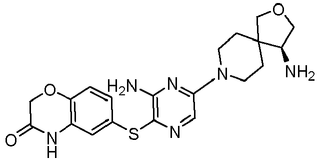 | 429 |
| 46 | (S)-4-((3-amino-5-(4-amino-2-oxa-8-azaspiro[4.5]decan-8-yl)pyrazin-2-yl)thio)-1,3,3-trimethyl indol in-2-one | 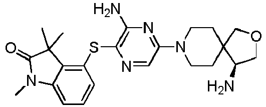 | 455 |
| 47 | (4S)-8-(6-amino-5-((3-fluoroindolin-4-yl)thio)pyrazin-2-yl)-2-oxa-8-azaspiro[4.5]decan-4-amine | 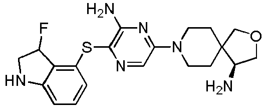 | 417 |
| 48 | 1-(4-((3-amino-5-((S)-4-amino-2-oxa-8-azaspiro[4.5]decan-8-yl)pyrazin-2-yl)thio)-3-fluoro-3-methylindolin-1-yl)ethanone | 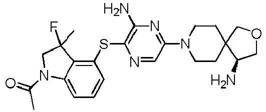 | 473 |
| 49 | (S)-8-(6-amino-5-((3,3-difluoroindolin-4-yl)thio)pyrazin-2-yl)-2-oxa-8-azaspiro[4.5]decan-4-amine | 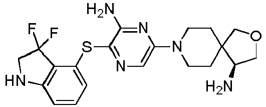 | 435 |
| 50 | 1-(4-((3-amino-5-((S)-4-amino-2-oxaspiro[4.5]decan-8-yl)pyrazin-2-yl)thio)-3-methylindolin-1-yl)ethanone | 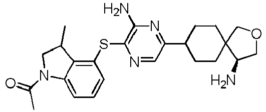 | 454 |
| 51 | (S)-8-(6-amino-5-((8-chloro-4,4-difluoro-1,2,3,4-tetrahydroquinolin-5-yl)thio)pyrazin-2-yl)-2-oxa-8-azaspiro[4.5]decan-4-amine | 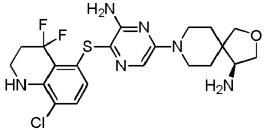 | 483 |
| 52 | (4S)-8-(6-amino-5-((8-chloro-4-fluoro-1,2,3,4-tetrahydroquinolin-5-yl)thio)pyrazin-2-yl)-2-oxa-8-azaspiro[4.5]decan-4-amine | 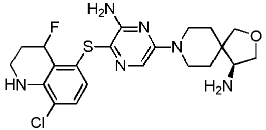 | 465 |
| 53 | (S)-8-(6-amino-5-((3,3-difluoro-1-methylindolin-4-yl)thio)pyrazin-2-yl)-2-oxa-8-azaspiro[4.5]decan-4-amine | 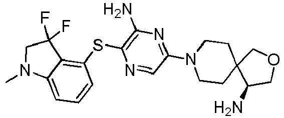 | 449 |
| 54 | (S)-6-((3-amino-5-(4-amino-2-oxa-8-azaspiro[4.5]decan-8-yl)pyrazin-2-yl)thio)-3,3-difluoroindolin-2-one | 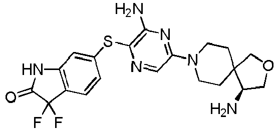 | 449 |
| 55 | 4-((3-amino-5-((S)-4-amino-2-oxa-8-azaspiro[4.5]decan-8-yl)pyrazin-2-yl)thio)-3-fluoroindolin-2-one | 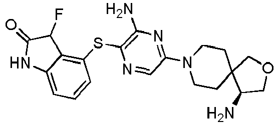 | 431 |
| 56 | (S)-8-(6-amino-5-((3,3-difluoro-2,3-dihydrobenzofuran-4-yl)thio)pyrazin-2-yl)-2-oxa-8-azaspiro[4.5]decan-4-amine | 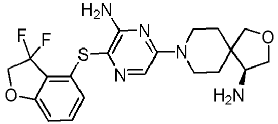 | 436 |
| 57 | (S)-8-(6-amino-5-((4,4-difluorochroman-5-yl)thio)pyrazin-2-yl)-2-oxa-8-azaspiro[4.5]decan-4-amine | 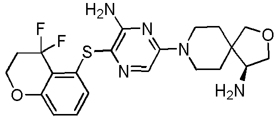 | 450 |
| 58 | 4-((3-amino-5-((S)-4-amino-2-oxa-8-azaspiro[4.5]decan-8-yl)pyrazin-2-yl)thio)-3-fluoro-1-methyl-3-(trifluoromethyl)indolin-2-one | 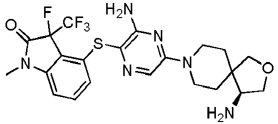 | 513 |
| 59 | (S)-6-((3-amino-5-(4-amino-2-oxa-8-azaspiro[4.5]decan-8-yl)pyrazin-2-yl)thio)-7-chloroindol in-2-one | 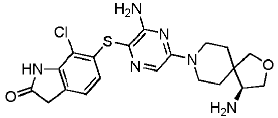 | 447 |
| 60 | (S)-8-(6-amino-5-((5-chloro-3,4-dihydro-2H-benzo[b][1,4]oxazin-6-yl)thio)pyrazin-2-yl)-2-oxa-8-azaspiro[4.5]decan-4-amine | 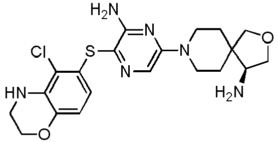 | 449 |
| 61 | (S)-7-((3-amino-5-(4-amino-2-oxa-8-azaspiro[4.5]decan-8-yl)pyrazin-2-yl)thio)-8-chloro-3,4-dihydroquinolin-2(1H)-one | 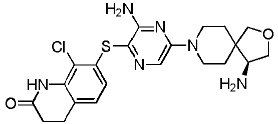 | 461 |
| 62 | (S)-6-((3-amino-5-(4-amino-2-oxa-8-azaspiro[4.5]decan-8-yl)pyrazin-2-yl)thio)-5-chloro-2H-benzo[b][1,4]oxazin-3(4H)-one | 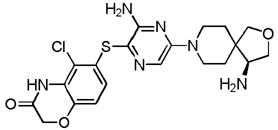 | 463 |
| 63 | (S)-2-(3-((3-amino-5-(4-amino-2-oxa-8-azaspiro[4.5]decan-8-yl)pyrazin-2-yl)thio)-2-chlorophenyl)-N, N-dimethyl-2-oxoacetamide (for reference only) | 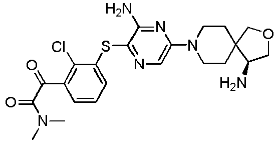 | 491 |
| 64 | 4-((3-amino-5-(4-(aminomethyl)-4-methylpiperidin-1-yl)pyrazin-2-yl)thio)-3,3-difluoro-1H-pyrrolo[2,3-b]pyridin-2(3H)-one | 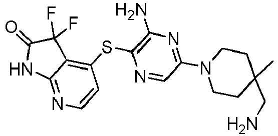 | 422 |
| 65 | (S)-4-((3-amino-5-(4-amino-2-oxa-8-azaspiro[4.5]decan-8-yl)pyrazin-2-yl)thio)-1H-benzo[d]imidazol-2(3H)-one | 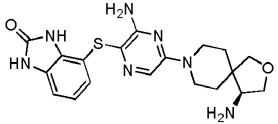 | 414 |
| 66 | (S)-4-((3-amino-5-(4-amino-2-oxa-8-azaspiro[4.5]decan-8-yl)pyrazin-2-yl)thio)-1,3-dimethyl-1H-benzo[d]imidazol-2(3H)-one | 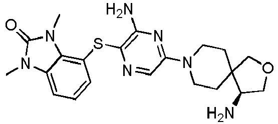 | 442 |
| 67 | (S)-8-(6-amino-5-((2,2-difluoro-2,3-dihydro-1H-benzo[d]imidazol-4-yl)thio)pyrazin-2-yl)-2-oxa-8-azaspiro[4.5]decan-4-amine | 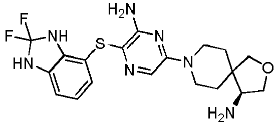 | 436 |
| 68 | (S)-8-(6-amino-5-((2,2-difluoro-1,3-dimethyl-2,3-dihydro-1H-benzo[d]imidazol-4-yl)thio)pyrazin-2-yl)-2-oxa-8-azaspiro[4.5]decan-4-amine | 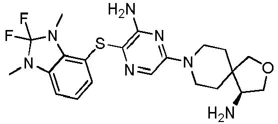 | 464 |
| 69 | (4S)-8-(6-amino-5-((1-amino-3,3-difluoro-2,3-dihydro-1H-inden-4-yl)thio)pyrazin-2-yl)-2-oxa-8-azaspiro[4.5]decan-4-amine | 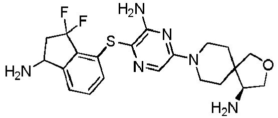 | 449 |
| 70 | (S)-5-((3-amino-5-(4-amino-2-oxa-8-azaspiro[4.5]decan-8-yl)pyrazin-2-yl)thio)-2-methyl-[1,2,4]triazolo[4,3-a]pyridin-3(2H)-one (for reference only) | 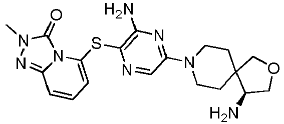 | 429 |
| 71 | (S)-8-(6-amino-5-((3,3-difluoro-2-methyl-2,3-dihydro-[1,2,4]triazolo[4,3-a]pyridin-5-yl)thio)pyrazin-2-yl)-2-oxa-8-azaspiro[4.5]decan-4-amine (for reference only) | 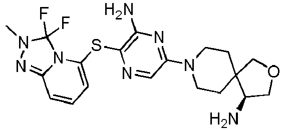 | 451 |
| 72 | (S)-1-(4-((3-amino-5-(4-amino-2-oxa-8-azaspiro[4.5]decan-8-yl)pyrazin-2-yl)thio)indolin-1-yl)ethanone | 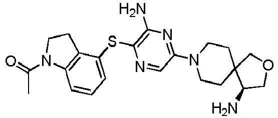 | 441 |
| 73 | 1 '-(6-amino-5-((2-amino-3-chloropyridin-4-yl)thio)pyrazin-2-yl)hexahydrospiro[cyclopenta[b]fu ran-5,4'-piperidin]-4-amine (for reference only) | 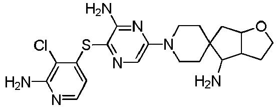 | 448 |
| 74 | 1'-(6-amino-5-((2-amino-3-chloropyridin-4-yl)thio)pyrazin-2-yl)spiro[bicyclo[3.1.0]hexane-3,4'-piperidin]-2-amine (for reference only) | 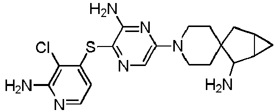 | 418 |
| 75 | 1'-amino-1-(6-amino-5-((2-amino-3-chloropyridin-4-yl)thio)pyrazin-2-yl)tetrahydrospiro[piperidine-4,2'-pyrrolizin]-3'(1'H)-one(for reference only) | 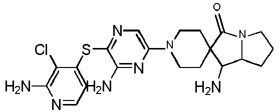 | 461 |
| 76 | 1'-(6-amino-5-((2-amino-3-chloropyridin-4-yl)thio)pyrazin-2-yl)spiro[bicyclo[3.1.0]hexane-2,4'-piperidin]-3-amine (for reference only) | 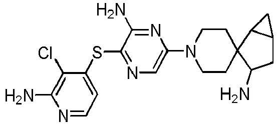 | 418 |

### PHARMACOLOGICAL TESTING

### Example A. Phosphatase Assay (single dose inhibition)

### Assay Protocol:

For single dose inhibition assays using 6,8-difluoro-4-methylumbelliferyl phosphate (DiFMUP) as a substrate, SHP2 samples (diluted to 0.5 nM in reaction buffer) were incubated with dPEG8 peptide for 30 min in reaction buffer[60 mM 3,3-dimethyl glutarate (pH7.2), 75 mM NaCI, 75 mM KCI, and 1 mM EDTA, 0.05% Tween 20, 2mM dithiothreitol (DTT) ] to active the PTP. DMSO [0.5% (v/v)] or compounds (100nM) were added to the mixture and incubated for 30 min at room temperature. Reactions were initiated by the addition of DiFMUP (12 µM; total reaction volume of 100 µL), and the fluorescence (excitation at 340 nm, emission at 450 nm) of the resulting solutions was measured on a 2104-0020 EnVision Xcite Multilabel Reader (PerkinElmer) after 30min. The experiment is carried out in triplicate. The value for the control sample (DMSO) was set to 100%, and the values for the compound-treated samples were expressed as activity relative to the control sample. The inhibition of SHP2 by compounds of the invention were shown in table 1.

**Table 1**

| Example | SHP2 inhibition(%) | Example | SHP2 inhibition(%) |
|---|---|---|---|
| 3@0.1µM | 60 | 7@0.1µM | 79 |
| 8@0.1µM | 76 | 10@0.1µM | 81 |
| 20@0.1µM | 57 | 29@0.1µM | 47 |
| 31 @0.1µM | 53 | 32 @0.1µM | 49 |
| 34 @0.1µM | 37 | 35 @0.1µM | 30 |
| 38 @0.1µM | 30 | 40 @0.1µM | 71 |
| 42@0.1µM | 58 | 43@0.1µM | 43 |
| 58 @0.1µM | 61 | 72@0.1µM | 31 |
| 74@0.1µM | 75 | 75@0.1µM | 35 |

### Example B.Phosphatase Assays (IC50)

IC₅₀ values were estimated using 6,8-difluoro-4-methylumbelliferyl phosphate (DiFMUP) as a substrate, SHP2 samples (diluted to 0.5 nM in reaction buffer) were incubated with dPEG8 peptide for 30 min in reaction buffer [60 mM 3,3-dimethyl glutarate (pH7.2), 75 mM NaCI, 75 mM KCI, and 1 mM EDTA, 0.05% Tween 20, 2mM dithiothreitol (DTT) ] to active the PTP. DMSO [0.5% (v/v)] or compounds (concentrations ranging from 0.3 nM to 1 µM) were added to the mixture and incubated for 30 min at room temperature. Reactions were initiated by the addition of DiFMUP (12 µM; total reaction volume of 100 µL), and the fluorescence (excitation at 340 nm, emission at 450 nm) of the resulting solutions was measured on a 2104-0020 EnVision Xcite Multilabel Reader (PerkinElmer) after 30min. The IC₅₀ results of the compounds of the invention were shown by table 2.

**Table 2**

| Example | IC₅₀(nM) | Example | IC₅₀(nM) |
|---|---|---|---|
| 7@ | 25.8 | 8@ | 21.4 |
| 10@ | 30 | 40@ | 40.6 |
| 74@ | 8.3 | SHP099 | 84 |

### Example C. Cell Proliferation Assay

KYSE-520 (1500 cells/well) were plated onto 96-well plates in 100µL medium (RPMI-1640 containing 3% FBS for KYSE-520 cells, Gibco). For drug treatment, compounds of the invention at various concentrations were added 24 hours after cell plating. At day 8, 50µL MTS/PMS reagents (Promega/Sigma) were added, and the absorbance value was determined according to the supplier's instruction (Promega). The IC₅₀ results of the compounds of the invention were shown by table 3.

**Table 3**

| Example | IC₅₀((µM) | Example | IC₅₀((µM) |
|---|---|---|---|
| 1 | 15.94 | 7 | 2.17 |
| 8 | 26.38 | 11 | 12.04 |
| 13 | 20.57 | 74 | 3.38 |

The compounds of the present invention are preferably formulated as pharmaceutical compositions administered by a variety of routes. Most preferably, such compositions are for oral administration. Such pharmaceutical compositions and processes for preparing the same are well known in the art. See, e.g., REMINGTON: THE SCIENCE AND PRACTICE OF PHARMACY (A. Gennaro, et al, eds., 19th ed., Mack Publishing Co., 1995). The compounds of Formula I are generally effective over a wide dosage range.

For example, dosages per day normally fall within the range of about 1 mg to about 200 mg total daily dose, preferably 1 mg to 150 mg total daily dose, more preferably 1 mg to 50 mg total daily dose. In some instances, dosage levels below the lower limit of the aforesaid range may be more than adequate, while in other cases still larger doses may be employed. It will be understood that the amount of the compound actually administered will be determined by a physician, in the light of the relevant circumstances, including the condition to be treated, the chosen route of administration, the actual compound or compounds administered, the age, weight, and response of the individual patient, and the severity of the patient's symptoms.

## Claims

1. A compound of Formula I or a pharmaceutically acceptable salt: 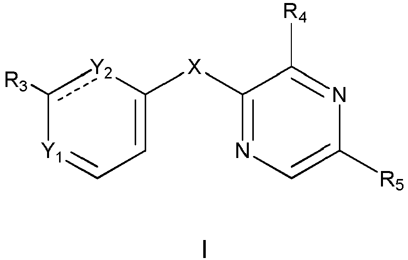
X is S;
Y₁ is N or CR₁;
Y₂ is N or CR₂;
R₁ combines with R₃, or R₂ combines with R₃, to form a 5-10 member heteroaryl, 5-10 member carbocyclic ring or 5-10 member heterocyclic ring, wherein each of the ring systems is independently optionally substituted with halogen, -CN, -OH, -NR₈R₉, -N₃, -NO₂, carbonyl, =O, oxo, substituted or unsubstituted C₁₋₆alkyl, substituted or unsubstituted C₁₋₆alkoxy, or C(O)R₈;
when R₁ combines with R₃ to form a 5-10 member heteroaryl, 5-10 member carbocyclic ring or 5-10 member heterocyclic ring, R₂ is -H, halogen, -CN, -OH, -NH₂, -N₃, -NO₂, substituted or unsubstituted C₁₋₆alkoxy, or substituted or unsubstituted C₁₋₆alkyl;
when R₂ combines with R₃ to form a 5-10 member heteroaryl, 5-10 member carbocyclic ring or 5-10 member heterocyclic ring, R₁ is -H, halogen, -CN, -OH, -NH₂, -N₃, -NO₂, substituted or unsubstituted C₁₋₆alkoxy, or substituted or unsubstituted C₁-₆alkyl;
R₄ is halogen, -CN, -OH, -NR₈R₉, -N₃, -NO₂, substituted or unsubstituted C₁₋₆alkoxy, substituted or unsubstituted C₁₋₆alkyl, C₅₋₁₈heterocyclic ring or C₅₋₁₈carbocyclic ring; wherein each of the ring systems is independently optionally substituted with halogen, -CN, -OH, -NO₂, carbonyl, =O, oxo, substituted or unsubstituted C₁₋₆alkyl, substituted or unsubstituted C₁₋₆alkoxy, -NR₈R₉ or -CH₂NR₈R₉;
R₅ is -H, halogen, -CN, -OH, -NR₈R₉, -N₃, -NO₂, C₁₋₆alkyl, C₁₋₆alkoxy, C₆₋₁₀aryl, C₆₋₁₀arylalkyl, C₆₋₁₀heteroaryl, C₅₋₁₈heterocyclic ring or C₅₋₁₈carbocyclic ring; and each of which is independently optionally substituted with halogen, -CN, -OH, -N₃, -NO₂, -NH₂, carbonyl, =O, oxo, substituted or unsubstituted C₁₋₆alkyl, substituted or unsubstituted C₁₋₆alkoxy, (CH₂)ₖNR₈R₉, (CH₂)ₖNHC(O)OR₆ or C(O)R₈; and k is 0, 1 or 2;
each R₈ and R₉ is independently -H, halogen, -CN, -OH, -N₃, -NO₂, C₁₋₆alkyl, C₁₋₆alkoxy, C₂₋₆alkenyl, NH(C₁₋₆alkyl), N(C₁₋₆alkyl)₂, C₅₋₁₀heterocyclic ring or C₅₋₁₀carbocyclic ring; and each of which may be independently optionally substituted with halogen, -CN, -OH, -NH₂, -N₃, -NO₂, -C₁₋₆alkylene-NH₂, -C₁₋₆alkylene-NH-C₁₋₆alkyl, -C₁₋₆alkylene-N(C₁₋₆alkyl)₂, -NHBoc, -CH₂NHBoc, -NH-C₁₋₆alkyl, -N(C₁₋₆alkyl)₂, -NH-C₁₋₆alkoxy, -N(C₁₋₆alkoxy)₂, substituted or unsubstituted C₁₋₆alkyl, or substituted or unsubstituted C₁₋₆alkoxy; and each heterocyclic ring contains 1, 2, 3 or 4 heteroatoms selected from N, O or S;
wherein alkyl is saturated monovalent hydrocarbon radicals having straight, branched or cyclic moieties, alkoxy is straight, branched chain or cyclic alkyl group, and heterocyclic is unsubstituted and substituted mono- or polycyclic non-aromatic ring system containing one or more heteroatoms.

2. The compound of claim 1, wherein
when R₂ combines with R₃ to form a 5-10 member heteroaryl, 5-10 member carbocyclic ring or 5-10 member heterocyclic ring, R₁ is -H, -F, -CI, -Br, -I, -CN, -OH, - NH₂, substituted or unsubstituted C₁₋₃alkoxy, or substituted or unsubstituted C₁₋₃alkyl;
when R₁ combines with R₃ to form a 5-10 member heteroaryl, 5-10 member carbocyclic ring or 5-10 member heterocyclic ring, R₂ is -H, -F, -CI, -Br, -I, -CN, -OH, - NH₂, substituted or unsubstituted C₁₋₃alkoxy, or substituted or unsubstituted C₁₋₃alkyl;
R₄ is -F, -CI, -Br, -CN, -OH, -NR₈R₉, substituted or unsubstituted C₁₋₆alkyl, substituted or unsubstituted C₁₋₆alkoxy, C₅₋₁₈heterocyclic ring or C₅₋₁₀carbocyclic ring, wherein each of the ring systems is optionally substituted with -F, -CI, -Br, -CN, -OH, -NO₂, carbonyl, =O, oxo, substituted or unsubstituted C₁₋₆alkyl, substituted or unsubstituted C₁₋₆alkoxy, -NH₂, -NH-C₁₋₆alkyl, -NH-C₁₋₆alkoxy, -C₁₋₆alkylene-NH₂ or -C₁₋₆alkylene-NH-C₁₋₆alkyl-,
R₅ is -H, -F, -CI, -Br, -I, -NR₈R₉, C₁₋₃alkyl, C₁₋₃alkoxy, C₆₋₉aryl, C₆₋₉arylalkyl, C₆₋₉heteroaryl, C₆₋₁₇heterocyclic ring or C₆₋₁₇carbocyclic ring, wherein each of which is independently optionally substituted with halogen, -CN, -OH, -N₃, -NO₂, -NH₂, carbonyl, =O, oxo, substituted or unsubstituted C₁₋₆alkyl, substituted or unsubstituted C₁₋₆alkoxy, (CH₂)ₖNR₈R₉, (CH₂)ₖNHC(O)OR₆ or C(O)R₈; and k is 0, 1 or 2; wherein the C₆₋₉ heteroaryl contains 1, 2, 3 or 4 heteroatoms selected from N, O or S, and the C₆₋₉heteroaryl is 6-membered heteroaryl, 7-membered heteroaryl, 8-membered heteroaryl or 9-membered heteroaryl; the C₆₋₁₇heterocyclic ring contains 1, 2, 3, 4, 5, or 6 heteroatoms selected from N, O, or S, and the C₆₋₁₇heterocyclic ring is 6-membered heterocyclic ring, 7-membered heterocyclic ring, 8-membered heterocyclic ring, 9-membered heterocyclic ring, 10-membered heterocyclic ring, 11-membered heterocyclic ring, 12-membered heterocyclic ring, 13-membered heterocyclic ring, 14-membered heterocyclic ring, 15-membered heterocyclic ring, 16-membered heterocyclic ring or 17-membered heterocyclic ring;
each R₈ and R₉ is independently -H, halogen, CN, -OH, -NO₂, -C₁₋₆alkylene-NH₂, -C₁-₆alkylene-NH-C₁₋₆alkyl, -C₁₋₆alkylene-N(C₁₋₆alkyl)₂, -CH₂NHBoc, -NH-C₁₋₆alkyl, -N(C₁-₆alkyl)₂, -NH-C₁₋₆alkoxy, -N(C₁₋₆alkoxy)₂, C₁₋₆alkyl, C₁₋₆alkoxy, C₂₋₆alkenyl, C₅₋₁₀heterocyclic ring or C₅₋₁₀carbocyclic ring; each of which may be optionally substituted with halogen, -CN, -OH, -NH₂, -N₃, -NO₂, -C₁₋₆alkylene-NH₂, -C₁₋₆alkylene-NH-C₁₋₆alkyl, -C₁₋₆alkylene-N(C₁₋₆alkyl)₂, -NHBoc, -CH₂NHBoc, -NH-C₁-₆alkyl, -N(C₁₋₆alkyl)₂, -NH-C₁₋₆alkoxy, -N(C₁₋₆alkoxy)₂, substituted or unsubstituted C₁₋₆alkyl, or substituted or unsubstituted C₁₋₆alkoxy; and each heterocyclic ring contains 1, 2, 3 or 4 heteroatoms selected from N, O or S.

3. The compound of claims 1 or 2, wherein
when R₂ combines with R₃ to form a 5-10 member heteroaryl, 5-10 member carbocyclic ring or 5-10 member heterocyclic ring, R₁ is -H, -F, -CI, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with halogen, OH or NH₂;
when R₁ combines with R₃ to form a 5-10 member heteroaryl, 5-10 member carbocyclic ring or 5-10 member heterocyclic ring, R₂ is -H, -F, -CI, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with halogen, OH or NH₂;
R₄ is -F, -CI, -CN, -OH, -NH₂, substituted or unsubstituted C₁₋₃alkyl, substituted or unsubstituted C₁₋₃alkoxy, 5-membered heterocyclic ring containing 1, 2 or 3 heteroatoms selected from N or O, 6-membered heterocyclic ring containing 1, 2 or 3 heteroatoms selected from N or O, 5-membered carbocyclic ring or 6-membered carbocyclic ring; wherein each of the ring systems is optionally substituted with -F, - Cl, -Br, -CN, -OH, carbonyl, =O, oxo, substituted or unsubstituted C₁₋₃alkyl, substituted or unsubstituted C₁₋₃alkoxy, -NH₂, -NH-C₁₋₃alkyl, -NH-C₁₋₃alkoxy, -C₁₋₃alkylene-NH₂ or -C₁₋₃alkylene-NH-C₁₋₃alkyl;
R₅ is -F, -CI, -Br, -NR₈R₉, methyl, ethyl, propyl, isopropyl, methoxy, ethoxy, propoxy, isopropoxy, 6-membered aryl, 7-membered aryl, 8-membered aryl, 9-membered aryl, 6-membered arylalkyl, 7-membered arylalkyl, 8-membered arylalkyl, 9-membered arylalkyl, 6-membered heteroaryl, 7-membered heteroaryl, 8-membered heteroaryl, 9-membered heteroaryl, 6-membered heterocyclic ring, 7-membered heterocyclic ring, 8-membered heterocyclic ring, 9-membered heterocyclic ring, 10-membered heterocyclic ring, 11-membered heterocyclic ring, 12-membered heterocyclic ring, 13-membered heterocyclic ring, 14-membered heterocyclic ring, 15-membered heterocyclic ring, 16-membered heterocyclic ring, 6-membered carbocyclic ring, 7-membered carbocyclic ring, 8-membered carbocyclic ring, 9-membered carbocyclic ring, 10-membered carbocyclic ring, 11-membered carbocyclic ring, 12-membered carbocyclic ring, 13-membered carbocyclic ring, 14-membered carbocyclic ring, 15-membered carbocyclic ring or 16-membered carbocyclic ring; and each heteroaryl contains 1, 2 or 3 heteroatoms selected from N, O or S, and each heterocyclic ring contains 1, 2, 3, or 4 heteroatoms selected from N, O or S; wherein each of which is independently optionally substituted with -F, -CI, -Br, -I, -CN, -OH, -NO₂, -NH₂, carbonyl, =O, oxo, substituted or unsubstituted C₁₋₃alkyl, substituted or unsubstituted C₁₋₃alkoxy, (CH₂)ₖNR₈R₉, (CH₂)ₖNHC(O)OR₈, or substituted or unsubstituted C(O)R₈; and k is 0, 1 or 2; and/or
each R₈ and R₉ is independently -H, -F, -CI, -CN, -OH, -NO₂, -C₁₋₃alkylene-NH₂, -C₁₋₃alkylene-NH-C₁₋₃alkyl, -C₁₋₃alkylene-N(C₁₋₃alkyl)₂, -CH₂NHBoc, -NH-C₁₋₃alkyl, -N(C₁₋₃alkyl)₂, -NH-C₁₋₃alkoxy, -N(C₁₋₃alkoxy)₂, C₁₋₄alkyl, C₁₋₃alkoxy, C₂₋₃alkenyl, 5-membered heterocyclic ring, 6-membered heterocyclic ring, 7-membered heterocyclic ring, 8-membered heterocyclic ring, 9-membered heterocyclic ring, 10-membered heterocyclic ring, 5-membered carbocyclic ring, 6-membered carbocyclic ring, 7-membered carbocyclic ring, 8-membered carbocyclic ring, 9-membered carbocyclic ring, 10-membered carbocyclic ring; and each of which may be independently optionally substituted with halogen, -CN, -OH, -NH₂, -N₃, -NO₂, -C₁₋₆alkylene-NH₂, -C₁₋₆alkylene-NH-C₁₋₆alkyl, -C₁₋₆alkylene-N(C₁₋₆alkyl)₂, -NHBoc, - CH₂NHBoc, -NH-C₁₋₆alkyl, -N(C₁₋₆alkyl)₂, -NH-C₁₋₆alkoxy, -N(C₁₋₆alkoxy)₂, substituted or unsubstituted C₁₋₆alkyl, or substituted or unsubstituted C₁₋₆alkoxy; and each heterocyclic ring contains 1, 2, 3 or 4 heteroatoms selected from N, O or S.

4. The compound of any one of claims 1-3 wherein R₁ combines with R₃, or R₂ combines with R₃, to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring, wherein each of the ring systems contains 1, 2, 3 or 4 heteroatoms selected from N, O or S, and is optionally substituted with -F, -CI, -Br, -I, -CN, -OH, - NH₂, carbonyl, =O, oxo, substituted or unsubstituted C₁₋₃alkyl, substituted or unsubstituted C₁₋₃alkoxy or C(O)R₈.

5. The compound of any one of claims 1-4, wherein R₁ combines with R₃ to form a 5-membered heteroaryl, 6-membered heteroaryl, 5-membered heterocyclic ring or 6-membered heterocyclic ring, wherein each of the ring systems contains 1 or 2 heteroatoms selected from N or O, and is optionally substituted with -F, -CI, -Br, -OH, -NH₂, carbonyl, =O, oxo, methyl, ethyl, propyl, isopropyl, methoxy, CHF₂, CH₂F, CF₃ or C(O)R₈; and each methyl, ethyl, propyl, isopropyl, methoxy, CHF₂, CH₂F or C(O)R₈ is independently optionally substituted with -F, -CI, -Br or -I

6. The compound of any one of claims 1-5, wherein R₂ combines with R₃ to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring, wherein each of the ring systems contains 1, 2, 3 or 4 heteroatoms selected from N, O or S, and is optionally substituted with -F, -CI, -Br, -I, -CN, -OH, -NH₂, carbonyl, =O, oxo, substituted or unsubstituted C₁₋₃alkyl, substituted or unsubstituted C₁₋₃alkoxy or C(O)R₈; and the C₁₋₃alkyl is independently methyl, ethyl, propyl, isopropyl or cyclopropyl; the C₁₋₃alkoxy is independently methoxy, ethoxy, propoxy, isopropoxy or cyclopropyloxy.

7. The compound of any one of claims 1-6, wherein R₄ is -CI, -NH₂, methyl or piperidinyl, wherein the piperidinyl is optionally substituted with methyl, -NH₂ or - CH₂NH₂.

8. The compound of any one of claims 1-7, wherein:
R₅ is 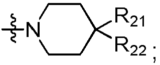
each R₂₁ and R₂₂ is independently halogen, C₁₋₃alkyl, -NH₂, -C₁₋₃alkylene-NH₂, -C₁-₂alkylene-NH-C₁₋₃alkyl, -C₁₋₂alkylene-N(C₁₋₃alkyl)₂, -NHBoc or -CH₂NHBoc,
or R₂₁ and R₂₂ together with the carbon atom to which they are both attached form a 5-10 member heteroaryl, 5-10 member carbocyclic ring or a 5-10 member heterocyclic ring, wherein each of the ring system is optionally substituted with halogen, -CN, -OH, carbonyl, =O, oxo, -C₁₋₃alkylene-NH₂, -C₁₋₂alkylene-NH-C₁₋₃alkyl, -C₁₋₂alkylene-N(C₁₋₃alkyl)₂, -NHBoc, -CH₂NHBoc, -NH₂, C₁₋₃alkoxy or C₁₋₃alkyl.

9. The compound of claim 8, wherein, R₂₁ and R₂₂ together with the carbon atom to which they are both attached form a 5-membered heteroaryl, 6-membered heteroaryl, 7-membered heteroaryl, 8-membered heteroaryl, 9-membered heteroaryl, 10-membered heteroaryl, 5-membered heterocyclic ring, 6-membered heterocyclic ring, 7-membered heterocyclic ring, 8-membered heterocyclic ring, 9-membered heterocyclic ring or 10-membered heterocyclic ring; wherein each of the ring system contains 1, 2 or 3 heteroatoms selected from N, O or S, and is independently optionally substituted with halogen, -CN, -OH, carbonyl, =O, oxo, -NH₂, C₁₋₃alkoxy or C₁₋₃alkyl; and the C₁₋₃alkoxy is independently methoxy, ethoxy, propoxy, isopropoxy or cyclopropyloxy; the C₁₋₃alkyl is independently methyl, ethyl, propyl, isopropyl or cyclopropyl.

10. The compound of any one of claims 1-9, wherein R₅ is -NH₂,

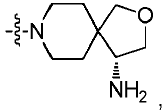

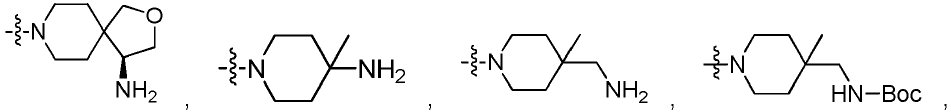

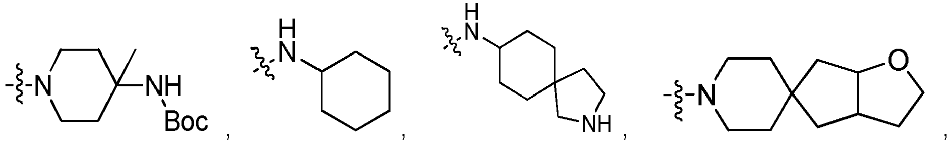

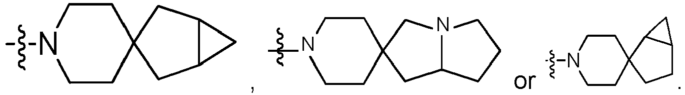

11. The compound of any one of claims 1-10, wherein R₈ and R₉ is independently -H, methyl, tert-butyl, -CH=CH₂, N(CH₃)₂,

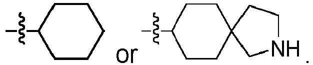

12. The compound of any one of claims 1-11, wherein
X is S;
Y₁ is N and Y₂ is CR₂;
Y₂ is N and Y₁ is CR₁; and/or
R₄ is -NH₂.

13. The compound of any one of claims 1-12, wherein the compound is of Formula II: 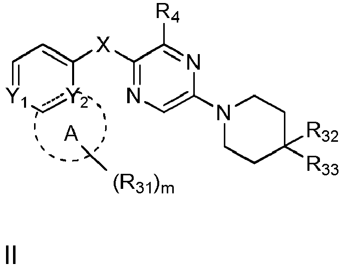 wherein,
X is S;
Y₁ is N or CR₂₅;
Y₂ is C;
R₂₅ is H, halogen, C₁₋₃alkyl, or C₁₋₃alkoxy-,
ring 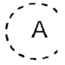 is 5-8 member heteroaryl containing 1,2,3 or 4 heteroatoms selected form N, O or S, 5-8 member carbocyclic ring or 5-8 member heterocyclic ring containing 1,2,3 or 4 heteroatoms selected form N, O or S;
R₃₁ is -H, halogen, -OH, -NH₂, -(C=O)C₁₋₃alkyl, -CN, -NO₂, carbonyl, =O, oxo, carboxyl, -C₁₋₃alkylene-NH₂, -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy;
m is 0, 1, 2, 3 or 4;
R₄ is -H, halogen, -NH₂, substituted or unsubstituted C₁₋₃alkoxy, or substituted or unsubstituted C₁₋₃alkyl;
each R₃₂ and R₃₃ is independently -H, halogen, -OH, -NH₂, -CN, -NO₂, -C₁₋₃alkylene-NH₂, -C₁₋₂alkylene-NH-C₁₋₃alkyl, -C₁₋₂alkylene-N(C₁₋₃alkyl)₂, -NHBoc, -CH₂NHBoc, - NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy-,
or R₃₂ and R₃₃ together with the carbon atom to which they are both attached form a 5-8 member heteroaryl containing 1, 2 or 3 heteroatoms selected from N, O or S, or 5-8 member heterocyclic ring containing 1, 2 or 3 heteroatoms selected from N, O or S, wherein each of the ring systems is optionally substituted with halogen, -CN, -OH, -NH₂, carbonyl, =O, oxo, -CH₂NH₂, -C₁₋₃alkylene-NH₂, -C₁₋₂alkylene-NH-C₁₋₃alkyl, -C₁₋₂alkylene-N(C₁₋₃alkyl)₂, -NHBoc, -CH₂NHBoc, -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy.

14. The compound of claim 13, wherein
ring 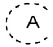 is 5-membered heteroaryl, 6-membered heteroaryl, 7-membered heteroaryl, 5-membered heterocyclic ring, 6-membered heterocyclic ring, 7-membered heterocyclic ring, 5-membered carbocyclic ring, 6-membered carbocyclic ring, 7-membered carbocyclic ring or 8-membered carbocyclic ring; and each the ring systems contains 1, 2 or 3 heteroatoms selected from N, O or S;
R₃₁ is -H, -F, -CI, -Br, -I, -OH, -NH₂, carbonyl, =O, oxo, -(CO)C₁₋₃alkyl, -C₁₋₃alkylene-NH₂, -NH-C₁₋₃alkyl, -N(C₁₋₃alkyl)₂, substituted or unsubstituted C₁₋₃alkyl, or substituted or unsubstituted C₁₋₃alkoxy-,
R₃₂ and R₃₃ together with the carbon atom to which they are both attached to form a 5-membered heterocyclic ring, 6-membered heterocyclic ring or 7-membered heterocyclic ring; wherein each of the ring system containing 1 or 2 heteroatoms independently selected from O or N, and is optionally substituted with halogen, -CN, - OH, -NH₂, carbonyl, =O, oxo, -NO₂, C₁₋₃alkyl or C₁₋₃alkoxy; and/or
each R₃₂ and R₃₃ is independently -CH₂NH₂, -CH₂NHBoc or methyl.

15. The compound of claim 13 or 14, wherein
ring 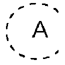 is 5-membered heterocyclic ring containing 1, 2 or 3 heteroatoms selected form N or O, 6-membered heterocyclic ring containing 1or 2 heteroatoms selected form N or O or 5-membered carbocyclic ring;
R₃₁ is -F, -COCH₃, carbonyl, =O, oxo, -CH₃ or -CF₃; and/or
R₃₂ and R₃₃ together with the carbon atom to which they are both attached to form a 5-membered heterocyclic ring; and the heterocyclic ring contains 1 heteroatoms selected from O or N, and is optionally substituted with -F, -CI, -OH, -NH₂, carbonyl, =O, oxo, methyl or methoxy.

16. The compound of any one of claims 13-15, wherein
Y₁ is N;
Y₂ is C;
R₂₅ is -H or -CI; and/or
R₄ is -NH₂.

17. The compound of any one of claims 1-12, wherein the compound is of Formula III: 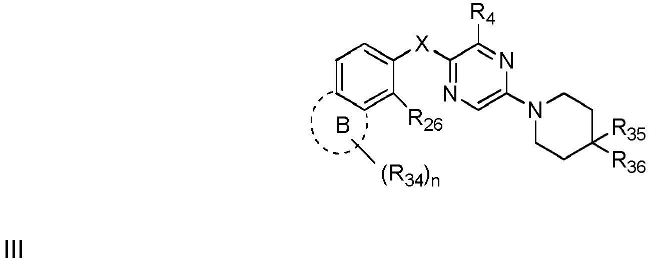 wherein,
X is S;
R₂₆ is -H, halogen, substituted or unsubstituted C₁₋₃alkyl, or substituted or unsubstituted C₁₋₃alkoxy-,
ring 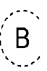 is 5-8 member heteroaryl or 5-8 member heterocyclic ring; and each the ring system independently contains 1, 2, 3 or 4 heteroatoms selected form N, O or S;
R₃₄ is -H, halogen, -OH, -NR₃₅R₃₆, -CN, -NO₂, carbonyl, =O, oxo, substituted or unsubstituted C₁₋₃alkyl, or substituted or unsubstituted C₁₋₃alkoxy-,
n is 0, 1, 2 or 3_{;}
R₄ is halogen, -NH₂, substituted or unsubstituted C₁₋₆alkoxy, or substituted or unsubstituted C₁₋₆ alkyl;
each R₃₅ and R₃₆ is independently -H, halogen, -OH, -NH₂, -CN, -NO₂, -CH₂NH₂, substituted or unsubstituted C₁₋₃alkyl, or substituted or unsubstituted C₁₋₃alkoxy-,
or R₃₅ and R₃₆ together with the carbon atom to which they are both attached to form a 5-8 member heteroaryl or 5-8 member heterocyclic ring, wherein each of the ring system independently contains 1, 2 or 3 heteroatoms selected from N, O or S, and is optionally substituted with halogen, -CN, -OH, -NH₂, carbonyl, =O, oxo, -CH₂NH₂, - C₁₋₃alkylene-NH₂, -C₁₋₂alkylene-NH-C₁₋₃alkyl, -C₁₋₂alkylene-N(C₁₋₃alkyl)₂, -NHBoc, - CH₂NHBoc, -NH-C₁₋₃alkyl, -N(C₁₋₃alkyl)₂, -NHC₁₋₃alkoxy, -N(C₁₋₃alkoxy)₂, substituted or unsubstituted C₁₋₃alkyl, or substituted or unsubstituted C₁₋₃alkoxy.

18. The compound of claim 17, wherein
ring 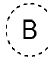 is 5-membered heteroaryl, 6-membered heteroaryl, 7-membered heteroaryl, 5-membered heterocyclic ring, 6-membered heterocyclic ring or 7-membered heterocyclic ring; and each of the ring system independently contains 1, 2 or 3 heteroatoms selected form N, O or S, and/or
R₃₅ and R₃₆ together with the carbon atom to which they are both attached to form a 5-membered heterocyclic ring, 6-membered heterocyclic ring or 7-membered heterocyclic ring; wherein each of the ring system is independently contains 1 or 2 heteroatoms independently selected from O or N, and is optionally substituted with halogen, -CN, -OH, -NH₂, carbonyl, =O, oxo, -NO₂, C₁₋₃alkyl or C₁₋₃alkoxy.

19. The compound of claim 17 or 18, wherein
ring 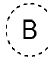 is 5-membered heterocyclic ring or 6-membered heterocyclic ring; and each of the ring system independently contains 1 or 2 heteroatoms selected form N or O; and/or
R₃₅ and R₃₆ together with the carbon atom to which they are both attached to form a 5-membered heterocyclic ring; wherein the ring system contains 1 heteroatom independently selected from O or N, and is optionally substituted with -F, -CI, -OH, - NH₂, carbonyl, =O, oxo, methyl or methoxy.

20. The compound of any one of claims 17-19, wherein
R₂₆ is -H or -CI;
R₄ is -NH₂; and/or
R₃₄ is -F, -COCH₃, =O, -CH₃ or -CF₃.

21. The compound of any one of claims 1-20, wherein each substituted or unsubstituted C₁₋₆alkyl is independently C₁₋₆alkyl, or C₁₋₆alkyl substituted with halogen, -OH, -CN, NH₂, -NO₂, carbonyl, =O, oxo, -C₁₋₆alkylene-NH₂, -C₁₋₆alkylene-NH-C₁₋₆alkyl. or -C₁₋₆alkylene-N(C₁₋₆alkyl)₂; each substituted or unsubstituted C₁₋₆alkoxy is independently C₁₋₆alkoxy, or C₁₋₆alkoxy substituted with halogen, -OH, -CN, NH₂, -NO₂, carbonyl, =O, oxo, -C₁₋₆alkylene-NH₂, -C₁₋₆alkylene-NH-C₁₋₃alkyl or -C₁₋₆alkylene-N (C₁₋₆alkyl)₂.

22. The compound of any one of claims 1-21, wherein
each C₁₋₆alkyl is independently methyl, ethyl, propyl, isopropyl, cyclopropyl, n-butyl, isobutyl, tert-buyl, n-pentyl, neopentyl, isopentyl, cyclopentyl, n-hexyl or cyclohexyl;
each C₁₋₃alkoxy is independently methoxy, ethoxy, propoxy, isopropoxy or cyclopropyloxy;
each C₂₋₃alkenyl is independently -CH=CH₂, -CH₂-CH=CH₂ or -CH=CH-CH₃;
each C₂₋₃alkylnyl is independently -C=CH, -CH₂-C≡CH or -C≡C-CH₃; and/or
each halogen is independently -F, -CI, -Br or -I.

23. The compound of any one of claims 1-22, wherein each heterocyclic ring group and each carbocyclic ring group includes single ring, spiral ring, bridge ring, fused ring and all various combinations of spiral ring, bridge ring, and/or fused ring.

24. The compound of claim 23, the said spiral ring includes 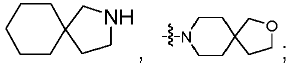 and the said various combinations of spiral ring, bridge ring, and/or fused ring include

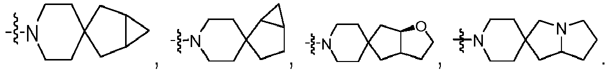

25. The compound of claim 1, wherein the compound is
(S)-1-(4-((3-amino-5-(4-amino-2-oxa-8-azaspiro[4.5]decan-8-yl)pyrazin-2-yl)thio)-3,3-difluoroindolin-1-yl)ethanone.

## Patentansprüche

1. Eine Verbindung der Formel I oder ein pharmazeutisch akzeptables Salz, wobei: 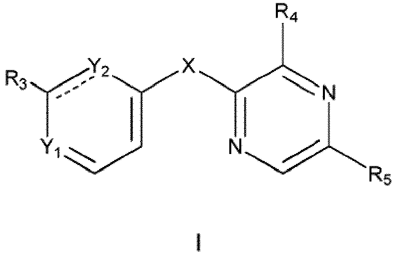
X S ist;
Y₁ N oder CR₁ ist;
Y₂ N oder CR₂ ist;
R₁ sich mit R₃ vereinigt oder R₂ sich mit R₃ vereinigt, um ein 5-10-gliedriges Heteroaryl, einen 5-10-gliedrigen carbocyclischen Ring oder einen 5-10-gliedrigen heterocyclischen Ring zu bilden, wobei jedes der Ringsysteme unabhängig optional mit Halogen, -CN, -OH, -NR₈R₉, -N₃, -NO₂, Carbonyl, =O, Oxo, substituiertem oder unsubstituiertem C₁₋₆-Alkyl, substituiertem oder unsubstituiertem C₁₋₆-Alkoxy oder C(O)R₈ substituiert ist;
wenn sich R₁ mit R₃ vereinigt, um ein 5-10-gliedriges Heteroaryl, einen 5-10-gliedrigen carbocyclischen Ring oder 5-10-gliedrigen heterocyclischen Ring zu bilden, R₂ -H, Halogen, -CN, -OH, -NH₂, -N₃, -NO₂, substituiertes oder unsubstituiertes C₁₋₅-Alkoxy oder substituiertes oder unsubstituiertes C₁₋₆-Alkyl ist;
wenn sich R₂ mit R₃ vereinigt, um ein 5-10-gliedriges Heteroaryl, einen 5-10-gliedrigen carbocyclischen Ring oder 5-10-gliedrigen heterocyclischen Ring zu bilden, R₁ -H, Halogen, -CN, -OH, -NH₂, -N₃, -NO₂, substituiertes oder unsubstituiertes C₁₋₅-Alkoxy oder substituiertes oder unsubstituiertes C₁₋₅-Alkyl ist;
R₄ Halogen, -CN, -OH, -NR₈R₉, -N₃, -NO₂, substituiertes oder unsubstituiertes C₁₋₆-Alkoxy, substituiertes oder unsubstituiertes C₁₋₅-Alkyl, ein heterocyclischer C₅₋₁₈-Ring oder carbocyclischer C₅₋₁₈-Ring ist; wobei jedes der Ringsysteme unabhängig optional mit Halogen, -CN, -OH, -NO₂, Carbonyl, =O, Oxo, substituiertem oder unsubstituiertem C₁₋₅-Alkyl, substituiertem oder unsubstituiertem C₁₋₆-Alkoxy, -NR₈R₉ oder -CH₂NR₈R₉ substituiert ist;
R₅ -H, Halogen, -CN, -OH, -NR₈R₉, -N₃, -NO₂, C₁₋₅-Alkyl, C₁₋₅-Alkoxy, C₆₋₁₀-Aryl, C₆₋₁₀-Arylalkyl, C₆₋₁₀-Heteroaryl, ein heterocyclischer C₅₋₁₃-Ring oder carbocyclischer C₅₋₁₈-Ring ist; und jedes von denen unabhängig optional mit Halogen, -CN, -OH, -N₃, -NO₂, -NH₂, Carbonyl, =O, Oxo, substituiertem oder unsubstituiertem C₁₋₅-Alkyl, substituiertem oder unsubstituiertem C₁₋₅-Alkoxy, (CH₂)ₖNR₈R₉, (CH₂)ₖNHC(O)OR₈ oder C(O)R₈ substituiert ist; und k 0, 1 oder 2 ist;
jedes R₈ und R₉ unabhängig -H, Halogen, -CN, -OH, -N₃, -NO₂, C₁₋₆-Alkyl, C₁₋₅-Alkoxy, C₂₋₆-Alkenyl, NH(C₁₋₆-alkyl), N(C₁₋₆-alkyl)₂, ein heterocyclischer C₅₋₁₀-Ring oder carbocyclischer C₅₋₁₀-Ring ist; und jedes davon unabhängig optional mit Halogen, - CN, -OH, -NH₂, -N₃, -NO₂, -C₁₋₆-Alkylen-NH₂, -C₁₋₆-Alkylen-NH-C₁₋₅-alkyl, -C₁₋₆-Alkylen-N(C₁₋₆-alkyl)₂, -NHBoc, -CH₂NHBoc, -NH-C₁₋₆-alkyl, -N(C₁₋₆-alkyl)₂, -NH-C₁₋₆-alkoxy, -N(C₁₋₆-alkoxy)₂, substituiertem oder unsubstituiertem C₁₋₅-Alkyl oder substituiertem oder unsubstituiertem C₁₋₅-Alkoxy substituiert sein kann; und jeder heterocyclische Ring 1, 2, 3 oder 4 Heteroatome, ausgewählt aus N, O oder S, enthält;
wobei Alkyl gesättigte einwertige Kohlenwasserstoffreste mit geraden, verzweigten oder cyclischen Molekülteilen ist, Alkoxy eine gerade, verzweigtkettige oder cyclische Alkylgruppe ist und heterocyclisch ein unsubstituiertes und substituiertes mono- oder polycyclisches nichtaromatisches Ringsystem ist, das ein oder mehrere Heteroatome enthält.

2. Verbindung nach Anspruch 1, wobei
wenn sich R₂ mit R₃ vereinigt, um ein 5-10-gliedriges Heteroaryl, einen 5-10-gliedrigen carbocyclischen Ring oder einen 5-10-gliedrigen heterocyclischen Ring zu bilden, R₁ -H, -F, -Cl, -Br, -I, -CN, -OH, -NH₂, substituiertes oder unsubstituiertes C₁₋₃-Alkoxy oder substituiertes oder unsubstituiertes C₁₋₃-Alkyl ist;
wenn sich R₁ mit R₃ vereinigt, um ein 5-10-gliedriges Heteroaryl, einen 5-10-gliedrigen carbocyclischen Ring oder einen 5-10-gliedrigen heterocyclischen Ring zu bilden, R₂ -H, -F, -Cl, -Br, -I, -CN, -OH, -NH₂, substituiertes oder unsubstituiertes C₁₋₃-Alkoxy oder substituiertes oder unsubstituiertes C₁₋₃-Alkyl ist;
R₄ -F, -Cl, -Br, -CN, -OH, -NR₈R₉, substituiertes oder unsubstituiertes C₁₋₅-Alkyl, substituiertes oder unsubstituiertes C₁₋₅-Alkoxy, ein heterocyclischer C₅₋₁₃-Ring oder carbocyclischer C₅₋₁₀-Ring ist, wobei jedes der Ringsysteme optional mit -F, -Cl, -Br, -CN, -OH, -NO₂, Carbonyl, =O, Oxo, substituiertem oder unsubstituiertem C₁₋₅-Alkyl, substituiertem oder unsubstituiertem C₁₋₅-Alkoxy, -NH₂, -NH-C₁₋₆-Alkyl, -NH-C₁₋₆-alkoxy, -C₁₋₆-Alkylen-NH₂ oder -C₁₋₆-Alkylen-NH-C₁₋₆-alkyl substituiert ist;
R₅ -H, -F, -Cl, -Br, -I, -NR₈R₉, C₁₋₃-Alkyl, C₁₋₃-Alkoxy, C₆₋₉-Aryl, C₆₋₉-Arylalkyl, C₆₋₉-Heteroaryl, ein heterocyclischer C₆₋₁₇-Ring oder carbocyclischer C₆₋₁₇-Ring ist, wobei jedes davon unabhängig optional mit Halogen, -CN, -OH, -N₃, -NO₂, -NH₂, Carbonyl, =O, Oxo, substituiertem oder unsubstituiertem C₁₋₅-Alkyl, substituiertem oder unsubstituiertem C₁₋₅-Alkoxy, (CH₂)ₖNR₈R₉, (CH₂)ₖNHC(O)OR₈ oder C(O)R₈ substituiert ist; und k 0, 1 oder 2 ist; wobei das C₆₋₉-Heteroaryl 1, 2, 3 oder 4 Heteroatome, ausgewählt aus N, O oder S, enthält und das C₆₋₉-Heteroaryl 6-gliedriges Heteroaryl, 7-gliedriges Heteroaryl, 8-gliedriges Heteroaryl oder 9-gliedriges Heteroaryl ist; der heterocyclische C₆₋₁₇-Ring 1, 2, 3, 4, 5 oder 6 Heteroatome, ausgewählt aus N, O, oder S, enthält und der heterocyclische C₆₋₁₇-Ring ein 6-gliedriger heterocyclischer Ring, 7-gliedriger heterocyclischer Ring, 8-gliedriger heterocyclischer Ring, 9-gliedriger heterocyclischer Ring, 10-gliedriger heterocyclischer Ring, 11-gliedriger heterocyclischer Ring, 12-gliedriger heterocyclischer Ring, 13-gliedriger heterocyclischer Ring, 14-gliedriger heterocyclischer Ring, 15-gliedriger heterocyclischer Ring, 16-gliedriger heterocyclischer Ring oder 17-gliedriger heterocyclischer Ring ist;
jedes R₈ und R₉ unabhängig -H, Halogen, CN, -OH, -NO₂, -C₁₋₅-Alkylen-NH₂, -C₁₋₆-Alkylen-NH-C₁₋₆-alkyl, -C₁₋₆-Alkylen-N(C₁₋₆-alkyl)₂, -CH₂NHBoc, -NH-C₁₋₆-alkyl, -N(C₁₋₆-alkyl)₂, -NH-C₁₋₆-alkoxy, -N(C₁₋₆-alkoxy)₂, C₁₋₅-Alkyl, C₁₋₅-Alkoxy, C₂₋₆-Alkenyl, ein heterocyclischer C₅₋₁₀-Ring oder carbocyclischer C₅₋₁₀-Ring ist; von dem jedes optional mit Halogen, -CN, -OH, -NH₂, -N₃, -NO₂, -C₁₋₅-Alkylen-NH₂, -C₁₋₆-Alkylen-NH-C₁₋₆-alkyl, -C₁₋₆-Alkylen-N(C₁₋₆-alkyl)₂, -NHBoc, -CH₂NHBoc, -NH-C₁₋₆-alkyl, -N(C₁₋₆-alkyl)₂, -NH-C₁₋₆-alkoxy, -N(C₁₋₆-alkoxy)₂, substituiertem oder unsubstituiertem C₁₋₆-Alkyl oder substituiertem oder unsubstituiertem C₁₋₅-Alkoxy substituiert sein kann; und jeder heterocyclische Ring 1, 2, 3 oder 4 Heteroatome, ausgewählt aus N, O oder S, enthält.

3. Verbindung nach Ansprüchen 1 oder 2, wobei
wenn sich R₂ mit R₃ vereinigt, um ein 5-10-gliedriges Heteroaryl, einen 5-10-gliedrigen carbocyclischen Ring oder einen 5-10-gliedrigen heterocyclischen Ring zu bilden, R₁ -H, -F, -Cl, -NH₂, Methyl, Ethyl, Propyl, Isopropyl, Methoxy, Ethoxy, Propoxy oder Isopropoxy ist; und jedes Methyl, Ethyl, Propyl, Isopropyl, Methoxy, Ethoxy, Propoxy oder Isopropoxy unabhängig optional mit Halogen, OH oder NH₂ substituiert ist;
wenn sich R₁ mit R₃ vereinigt, um ein 5-10-gliedriges Heteroaryl, einen 5-10-gliedrigen carbocyclischen Ring oder einen 5-10-gliedrigen heterocyclischen Ring zu bilden, R₂ -H, -F, -Cl, -NH₂, Methyl, Ethyl, Propyl, Isopropyl, Methoxy, Ethoxy, Propoxy oder Isopropoxy ist; und jedes Methyl, Ethyl, Propyl, Isopropyl, Methoxy, Ethoxy, Propoxy oder Isopropoxy unabhängig optional mit Halogen, OH oder NH₂ substituiert ist;
R₄ -F, -Cl, -CN, -OH, -NH₂, substituiertes oder unsubstituiertes C₁₋₃-Alkyl, substituiertes oder unsubstituiertes C₁₋₃-Alkoxy, ein 5-gliedriger heterocyclischer Ring, der 1, 2 oder 3 Heteroatome, ausgewählt aus N oder O, enthält, ein 6-gliedriger heterocyclischer Ring, der 1, 2 oder 3 Heteroatome, ausgewählt aus N oder O, enthält, ein 5-gliedriger carbocyclischer Ring oder 6-gliedriger carbocyclischer Ring ist; wobei jedes der Ringsysteme optional mit -F, - Cl, -Br, -CN, -OH, Carbonyl, =O, Oxo, substituiertem oder unsubstituiertem C₁₋₃-Alkyl, substituiertem oder unsubstituiertem C₁₋₃-Alkoxy, -NH₂, -NH-C₁₋₃-alkyl, -NH-C₁₋₃-alkoxy, -C₁₋₃-Alkylen-NH₂ oder -C₁₋₃-Alkylen-NH-C₁₋₃-alkyl substituiert ist;
R₅ -F, -Cl, -Br, -NR₈R₉, Methyl, Ethyl, Propyl, Isopropyl, Methoxy, Ethoxy, Propoxy, Isopropoxy, 6-gliedriges Aryl, 7-gliedriges Aryl, 8-gliedriges Aryl, 9-gliedriges Aryl, 6-gliedriges Arylalkyl, 7-gliedriges Arylalkyl, 8-gliedriges Arylalkyl, 9-gliedriges Arylalkyl, 6-gliedriges Heteroaryl, 7-gliedriges Heteroaryl, 8-gliedriges Heteroaryl, 9-gliedriges Heteroaryl, ein 6-gliedriger heterocyclischer Ring, 7-gliedriger heterocyclischer Ring, 8-gliedriger heterocyclischer Ring, 9-gliedriger heterocyclischer Ring, 10-gliedriger heterocyclischer Ring, 11-gliedriger heterocyclischer Ring, 12-gliedriger heterocyclischer Ring, 13-gliedriger heterocyclischer Ring, 14-gliedriger heterocyclischer Ring, 15-gliedriger heterocyclischer Ring, 16-gliedriger heterocyclischer Ring, 6-gliedriger carbocyclischer Ring, 7- gliedriger carbocyclischer Ring, 8-gliedriger carbocyclischer Ring, 9-gliedriger carbocyclischer Ring, 10-gliedriger carbocyclischer Ring, 11-gliedriger carbocyclischer Ring, 12-gliedriger carbocyclischer Ring, 13-gliedriger carbocyclischer Ring, 14-gliedriger carbocyclischer Ring, 15-gliedriger carbocyclischer Ring oder 16-gliedriger carbocyclischer Ring ist; und jedes Heteroaryl 1, 2 oder 3 Heteroatome, ausgewählt aus N, O oder S, enthält und jeder heterocyclische Ring 1, 2, 3 oder 4 Heteroatome, ausgewählt aus N, O oder S, enthält; wobei jedes davon unabhängig optional mit -F, -Cl, -Br, -I, -CN, -OH, -NO₂, - NH₂, Carbonyl, =O, Oxo, substituiertem oder unsubstituiertem C₁₋₃-Alkyl, substituiertem oder unsubstituiertem C₁₋₃-Alkoxy, (CH₂)ₖNR₈R₉, (CH₂)ₖNHC(O)OR₈ oder substituiertem oder unsubstituiertem C(O)R₈ substituiert ist; und k 0, 1 oder 2 ist; und/oder
jedes R₈ und R₉ unabhängig -H, -F, -Cl, -CN, -OH, -NO₂, -C₁₋₃-Alkylen-NH₂, -C₁₋₃-Alkylen-NH-Ci-s-alkyl, -C₁₋₃-Alkylen-N(C₁₋₃-alkyl)₂, -CH₂NHBoc, -NH-C₁₋₃-alkyl, -N(C₁₋₃-alkyl)₂, -NH-C₁₋₃-alkoxy, -N(C₁₋₃-alkoxy)₂, C₁₋₄-Alkyl, C₁₋₃-Alkoxy, C₂₋₃-Alkenyl, ein 5-gliedriger heterocyclischer Ring, 6-gliedriger heterocyclischer Ring, 7-gliedriger heterocyclischer Ring, 8-gliedriger heterocyclischer Ring, 9-gliedriger heterocyclischer Ring, 10-gliedriger heterocyclischer Ring, 5-gliedriger carbocyclischer Ring, 6-gliedriger carbocyclischer Ring, 7-gliedriger carbocyclischer Ring, 8-gliedriger carbocyclischer Ring, 9-gliedriger carbocyclischer Ring, 10-gliedriger carbocyclischer Ring ist; und jedes davon unabhängig optional mit Halogen, -CN, -OH, -NH₂, -N₃, -NO₂, -C₁₋₅-Alkylen-NH₂, -C₁₋₆-Alkylen-NH-C₁₋₆-alkyl, - C₁₋₆-Alkylen-N(C₁₋₆-alkyl)₂, -NHBoc, - CH₂NHBoc, -NH-C₁₋₆-alkyl, -N(C₁₋₆-alkyl)₂, -NH-C₁₋₅-Alkoxy, -N(C₁₋₆-Alkoxy)₂, substituiertem oder unsubstituiertem C₁₋₅-Alkyl, oder substituiertem oder unsubstituiertem C₁₋₅-Alkoxy substituiert sein kann; und jeder heterocyclische Ring 1, 2, 3 oder 4 Heteroatome, ausgewählt aus N, O oder S, enthält.

4. Verbindung nach einem der Ansprüche 1-3, wobei sich R₁ mit R₃ vereinigt oder sich R₂ mit R₃ vereinigt, um ein 5-gliedriges Heteroaryl, 6-gliedriges Heteroaryl, 7-gliedriges Heteroaryl, 8-gliedriges Heteroaryl, einen 5-gliedrigen heterocyclischen Ring, 6- gliedrigen heterocyclischen Ring, 7-gliedrigen heterocyclischen Ring oder 8-gliedrigen heterocyclischen Ring zu bilden, wobei jedes der Ringsysteme 1, 2, 3 oder 4 Heteroatome, ausgewählt aus N, O oder S, enthält und optional mit -F, -Cl, -Br, -I, -CN, -OH, -NH₂, Carbonyl, =O, Oxo, substituiertem oder unsubstituiertem C₁₋₃-Alkyl, substituiertem oder unsubstituiertem C₁₋₃-Alkoxy oder C(O)R₈ substituiert ist.

5. Verbindung nach einem der Ansprüche 1-4, wobei sich R₁ mit R₃ vereinigt, um ein 5- gliedriges Heteroaryl, 6-gliedriges Heteroaryl, einen 5-gliedrigen heterocyclischen Ring oder 6-gliedrigen heterocyclischen Ring zu bilden, wobei jedes der Ringsysteme 1 oder 2 Heteroatome, ausgewählt aus N oder O, enthält und optional mit -F, -Cl, - Br, -OH, -NH₂, Carbonyl, =O, Oxo, Methyl, Ethyl, Propyl, Isopropyl, Methoxy, CHF₂, CH₂F, CF₃ oder C(O)R₈ substituiert ist; und jedes Methyl, Ethyl, Propyl, Isopropyl, Methoxy, CHF₂, CH₂F oder C(O)R₈ unabhängig optional mit -F, -Cl, -Br oder -I substituiert ist.

6. Verbindung nach einem der Ansprüche 1-5, wobei sich R₂ mit R₃ vereinigt, um ein 5-gliedriges Heteroaryl, 6-gliedriges Heteroaryl, 7-gliedriges Heteroaryl, 8-gliedriges Heteroaryl, einen 5-gliedrigen heterocyclischen Ring, 6-gliedrigen heterocyclischen Ring, 7-gliedrigen heterocyclischen Ring oder 8-gliedrigen heterocyclischen Ring zu bilden, wobei jedes der Ringsysteme 1, 2, 3 oder 4 Heteroatome, ausgewählt aus N, O oder S, enthält und optional mit -F, -Cl, -Br, -I, -CN, -OH, -NH₂, Carbonyl, =O, Oxo, substituiertem oder unsubstituiertem C₁₋₃-Alkyl, substituiertem oder unsubstituiertem C₁₋₃-Alkoxy oder C(O)R₈ substituiert ist; und das C₁₋₃-Alkyl unabhängig Methyl, Ethyl, Propyl, Isopropyl oder Cyclopropyl ist; das C₁₋₃-Alkoxy unabhängig Methoxy, Ethoxy, Propoxy, Isopropoxy oder Cyclopropyloxy ist.

7. Verbindung nach einem der Ansprüche 1-6, wobei R₄ -Cl, -NH₂, Methyl oder Piperidinyl ist, wobei das Piperidinyl optional mit Methyl, -NH₂ oder -CH₂NH₂ substituiert ist.

8. Verbindung nach einem der Ansprüche 1-7, wobei: 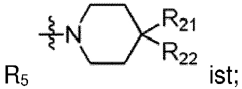 jedes R₂₁ und R₂₂ unabhängig Halogen, C₁₋₃-Alkyl, -NH₂, -C₁₋₃-Alkylen-NH₂, -C₁₋₂-Alkylen-NH-C₁₋₃-alkyl, -C1-2-Alkylen-N(C₁₋₃-alkyl)₂, -NHBoc oder -CH₂NHBoc ist,
oder R₂₁ und R₂₂ zusammen mit dem Kohlenstoffatom, an dem sie beide hängen, ein 5-10-gliedriges Heteroaryl, einen 5-10-gliedrigen carbocyclischen Ring oder einen 5-10-gliedrigen heterocyclischen Ring bilden, wobei jedes der Ringsysteme optional mit Halogen, -CN, -OH, Carbonyl, =O, Oxo, -C₁₋₃-Alkylen-NH₂, -C₁₋₂-Alkylen-NH-C₁₋₃-alkyl, -C1-2-Alkylen-N(C₁₋₃-alkyl)₂, -NHBoc, -CH₂NHBoc, -NH₂, C₁₋₃-Alkoxy oder C₁₋₃-Alkyl substituiert ist.

9. Verbindung nach Anspruch 8, wobei R₂₁ und R₂₂ zusammen mit dem Kohlenstoffatom, an dem sie beide hängen, ein 5-gliedriges Heteroaryl, 6-gliedriges Heteroaryl, 7-gliedriges Heteroaryl, 8-gliedriges Heteroaryl, 9-gliedriges Heteroaryl, 10-gliedriges Heteroaryl, einen 5-gliedrigen heterocyclischen Ring, 6-gliedrigen heterocyclischen Ring, 7-gliedrigen heterocyclischen Ring, 8-gliedrigen heterocyclischen Ring, 9-gliedrigen heterocyclischen Ring oder 10-gliedrigen heterocyclischen Ring bilden; wobei jedes der Ringsysteme 1, 2 oder 3 Heteroatome, ausgewählt aus N, O oder S, enthält und unabhängig optional mit Halogen, -CN, - OH, Carbonyl, =O, Oxo, -NH₂, C₁₋₃-Alkoxy oder C₁₋₃-Alkyl substituiert ist; und das C₁₋₃-Alkoxy unabhängig Methoxy, Ethoxy, Propoxy, Isopropoxy oder Cyclopropyloxy ist; das C₁₋₃-Alkyl unabhängig Methyl, Ethyl, Propyl, Isopropyl oder Cyclopropyl ist.

10. Verbindung nach einem der Ansprüche 1-9, wobei R₅ -NH₂,

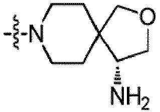

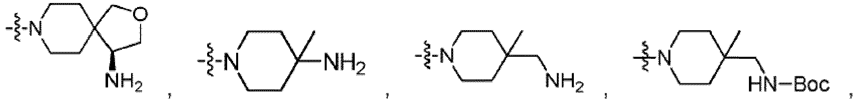

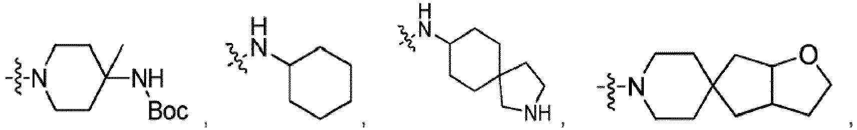

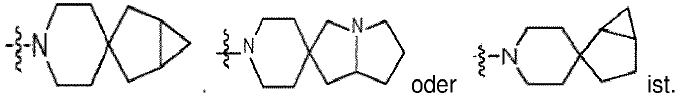

11. Verbindung nach einem der Ansprüche 1-10, wobei R₈ und R₉ unabhängig -H, Methyl, tert-Butyl, -CH=CH₂, N(CH₃)₂, 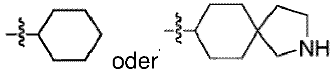 ist.

12. Verbindung nach einem der Ansprüche 1-11, wobei
X S ist;
Y₁ N ist und Y₂ CR₂ ist;
Y₂ N ist und Y₁ CR₁ ist; und/oder
R₄ -NH₂ ist.

13. Verbindung nach einem der Ansprüche 1-12, wobei die Verbindung eine mit der Formel II ist: 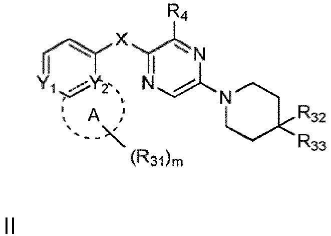 wobei
X S ist;
Y₁ N oder CR₂₅ ist;
Y₂ C ist;
R₂₅ H, Halogen, C₁₋₃-Alkyl oder C₁₋₃-Alkoxy ist;
Ring 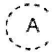 5-8-gliedriges Heteroaryl, das 1, 2, 3 oder 4 Heteroatome, ausgewählt aus N, O oder S, enthält, ein 5-8-gliedriger carbocyclischer Ring oder 5-8-gliedriger heterocyclischer Ring, der 1, 2, 3 oder 4 Heteroatome, ausgewählt aus N, O oder S, enthält, ist;
R₃₁ -H, Halogen, -OH, -NH₂, -(C=O)C₁₋₃-alkyl, -CN, -NO₂, Carbonyl, =O, Oxo, Carboxyl, -C₁₋₃-Alkylen-NH₂, -NH-C₁₋₃-alkyl, -N(C₁₋₃-alkyl)₂, -NH-C₁₋₃-alkoxy, -N(C₁₋₃-alkoxy)₂, substituiertes oder unsubstituiertes C₁₋₃-Alkyl oder substituiertes oder unsubstituiertes C₁₋₃-Alkoxy ist;
m 0, 1, 2, 3 oder 4 ist;
R₄ -H, Halogen, -NH₂, substituiertes oder unsubstituiertes C₁₋₃-Alkoxy oder substituiertes oder unsubstituiertes C₁₋₃-Alkyl ist;
jedes R₃₂ und R₃₃ unabhängig -H, Halogen, -OH, -NH₂, -CN, -NO₂, -C₁₋₃-Alkylen-NH₂, -C₁₋₂-Alkylen-NH-C₁₋₃-alkyl, -C1-2-Alkylen-N(C₁₋₃-alkyl)₂, -NHBoc, -CH₂NHBoc, -NH-C₁₋₃-alkyl, -N(C₁₋₃-alkyl)₂, -NH-C₁₋₃-alkoxy, -N(C₁₋₃-alkoxy)₂, substituiertes oder unsubstituiertes C₁₋₃-Alkyl oder substituiertes oder unsubstituiertes C₁₋₃-Alkoxy ist;
oder R₃₂ und R₃₃ zusammen mit dem Kohlenstoffatom, an dem sie beide hängen, ein 5-8-gliedriges Heteroaryl, das 1, 2 oder 3 Heteroatome, ausgewählt aus N, O oder S, enthält, oder einen 5-8-gliedrigen heterocyclischen Ring, der 1, 2 oder 3 Heteroatome, ausgewählt aus N, O oder S, enthält, bilden, wobei jedes der Ringsysteme optional mit Halogen, -CN, -OH, -NH₂, Carbonyl, =O, Oxo, -CH₂NH2, - C₁₋₃-Alkylen-NH₂, -C₁₋₂-Alkylen-NH-C₁₋₃-alkyl, -C1-2-Alkylen-N(C₁₋₃-alkyl)₂, -NHBoc, - CH₂NHBoc, -NH-C₁₋₃-alkyl, -N(C₁₋₃-alkyl)₂, -NH-C₁₋₃-alkoxy, -N(C₁₋₃-alkoxy)₂, substituiertem oder unsubstituiertem C₁₋₃-Alkyl oder substituiertem oder unsubstituiertem C₁₋₃-Alkoxy substituiert ist.

14. Verbindung nach Anspruch 13, wobei
Ring 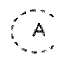 5-gliedriges Heteroaryl, 6-gliedriges Heteroaryl, 7-gliedriges Heteroaryl, ein 5-gliedriger heterocyclischer Ring, 6-gliedriger heterocyclischer Ring, 7-gliedriger heterocyclischer Ring, 5-gliedriger carbocyclischer Ring, 6-gliedriger carbocyclischer Ring, 7- gliedriger carbocyclischer Ring oder 8-gliedriger carbocyclischer Ring ist; und jedes der Ringsysteme 1, 2 oder 3 Heteroatome, ausgewählt aus N, O oder S, enthält;
R₃₁ -H, -F, -Cl, -Br, -I, -OH, -NH₂, Carbonyl, =O, Oxo, -(CO)C₁₋₃-alkyl, -C₁₋₃-Alkylen-NH₂, -NH-C₁₋₃-alkyl, -N(C₁₋₃-alkyl)₂, substituiertes oder unsubstituiertes C₁₋₃-Alkyl oder substituiertes oder unsubstituiertes C₁₋₃-Alkoxy ist;
R₃₂ und R₃₃ zusammen mit dem Kohlenstoffatom, an dem sie beide hängen, einen 5-gliedrigen heterocyclischen Ring, 6-gliedrigen heterocyclischen Ring oder 7-gliedrigen heterocyclischen Ring bilden; wobei jedes der Ringsysteme 1 oder 2 Heteroatome, unabhängig ausgewählt aus O oder N, enthält und optional mit Halogen, -CN, -OH, -NH₂, Carbonyl, =O, Oxo, -NO₂, C₁₋₃-Alkyl oder C₁₋₃-Alkoxy substituiert ist; und/oder
jedes R₃₂ und R₃₃ unabhängig -CH₂NH₂, -CH₂NHBoc oder Methyl ist.

15. Verbindung nach Anspruch 13 oder 14, wobei
Ring 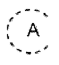 ein 5-gliedriger heterocyclischer Ring, der 1, 2 oder 3 Heteroatome, ausgewählt aus N oder O, ein 6-gliedriger heterocyclischer Ring, der 1 oder 2 Heteroatome, ausgewählt aus N oder O, oder ein 5-gliedriger carbocyclischer Ring ist;
R₃₁ -F, -COCH₃, Carbonyl, =O, Oxo, -CH₃ oder -CF₃ ist; und/oder
R₃₂ und R₃₃ zusammen mit dem Kohlenstoffatom, an dem sie beide hängen, einen 5-gliedrigen heterocyclischen Ring bilden; und der heterocyclische Ring 1 Heteroatom, ausgewählt aus O oder N, enthält und optional mit -F, -Cl, -OH, -NH₂, Carbonyl, =O, Oxo, Methyl oder Methoxy substituiert ist.

16. Verbindung nach einem der Ansprüche 13-15, wobei
Y₁ N ist;
Y₂ C ist;
R₂₅ -H oder -Cl ist; und/oder
R₄ -NH₂ ist.

17. Verbindung nach einem der Ansprüche 1-12, wobei die Verbindung eine mit der Formel III ist: 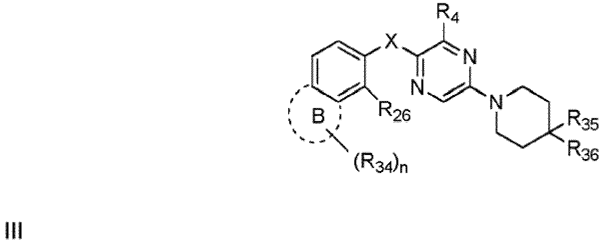 wobei
X S ist;
R₂₆ -H, Halogen, substituiertes oder unsubstituiertes C₁₋₃-Alkyl oder substituiertes oder unsubstituiertes C₁₋₃-Alkoxy ist;
Ring 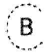 5-8-gliedriges Heteroaryl oder ein 5-8-gliedriger heterocyclischer Ring ist; und jedes der Ringsysteme unabhängig 1, 2, 3 oder 4 Heteroatome, ausgewählt aus N, O oder S, enthält;
R₃₄ -H, Halogen, -OH, -NR₃₅R₃₆, -CN, -NO₂, Carbonyl, =O, Oxo, substituiertes oder unsubstituiertes C₁₋₃-Alkyl oder substituiertes oder unsubstituiertes C₁₋₃-Alkoxy ist;
n 0, 1, 2 oder 3 ist;
R₄ Halogen, -NH₂, substituiertes oder unsubstituiertes C₁₋₅-Alkoxy oder substituiertes oder unsubstituiertes C₁₋₅-Alkyl ist;
jedes R₃₅ und R₃₆ unabhängig -H, Halogen, -OH, -NH₂, -CN, -NO₂, -CH₂NH2, substituiertes oder unsubstituiertes C₁₋₃-Alkyl oder substituiertes oder unsubstituiertes C₁₋₃-Alkoxy ist;
oder R₃₅ und R₃₆ zusammen mit dem Kohlenstoffatom, an dem sie beide hängen, ein 5-8-gliedriges Heteroaryl oder einen 5-8-gliedrigen heterocyclischen Ring bilden, wobei jedes der Ringsysteme unabhängig 1, 2 oder 3 Heteroatome, ausgewählt aus N, O oder S, enthält und optional mit Halogen, -CN, -OH, -NH₂, Carbonyl, =O, Oxo, - CH₂NH₂, -C₁₋₃-Alkylen-NH₂, -C₁₋₂-Alkylen-NH-C₁₋₃-alkyl, -C1-2-Alkylen-N(C₁₋₃-alkyl)₂, - NHBoc, -CH₂NHBoc, -NH-C₁₋₃-alkyl, -N(C₁₋₃-alkyl)₂, -NHC₁₋₃-alkoxy, -N(C₁₋₃-alkoxy)₂, substituiertem oder unsubstituiertem C₁₋₃-Alkyl oder substituiertem oder unsubstituiertem C₁₋₃-Alkoxy substituiert ist.

18. Verbindung nach Anspruch 17, wobei
Ring 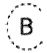 5-gliedriges Heteroaryl, 6-gliedriges Heteroaryl, 7-gliedriges Heteroaryl, ein 5-gliedriger heterocyclischer Ring, 6-gliedriger heterocyclischer Ring oder 7-gliedriger heterocyclischer Ring ist; und jedes der Ringsysteme unabhängig 1, 2 oder 3 Heteroatome, ausgewählt aus N, O oder S, enthält und/oder
R₃₅ und R₃₆ zusammen mit dem Kohlenstoffatom, an dem sie beide hängen, einen 5-gliedrigen heterocyclischen Ring, 6-gliedrigen heterocyclischen Ring oder 7-gliedrigen heterocyclischen Ring bilden; wobei jedes der Ringsysteme unabhängig 1 oder 2 Heteroatome, unabhängig ausgewählt aus O oder N, enthält und optional mit Halogen, -CN, -OH, -NH₂, Carbonyl, =O, Oxo, -NO₂, C₁₋₃-Alkyl oder C₁₋₃-Alkoxy substituiert ist.

19. Verbindung nach Anspruch 17 oder 18, wobei
Ring 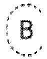 ein 5-gliedriger heterocyclischer Ring oder 6-gliedriger heterocyclischer Ring ist; und jedes der Ringsysteme unabhängig 1 oder 2 Heteroatome, ausgewählt aus N oder O, enthält; und/oder
R₃₅ und R₃₆ zusammen mit dem Kohlenstoffatom, an dem sie beide hängen, einen 5-gliedrigen heterocyclischen Ring bilden; wobei das Ringsystem 1 Heteroatom, unabhängig ausgewählt aus O oder N, enthält und optional mit -F, -Cl, -OH, -NH₂, Carbonyl, =O, Oxo, Methyl oder Methoxy substituiert ist.

20. Verbindung nach einem der Ansprüche 17-19, wobei
R₂₆ -H oder -Cl ist;
R₄ -NH₂ ist; und/oder
R₃₄ -F, -COCH₃, =O, -CH₃ oder -CF₃ ist.

21. Verbindung nach einem der Ansprüche 1-20, wobei jedes substituierte oder unsubstituierte C₁₋₅-Alkyl unabhängig C₁₋₅-Alkyl, oder C₁₋₅-Alkyl, substituiert mit Halogen, -OH, -CN, NH₂, -NO₂, Carbonyl, =O, Oxo, -C₁₋₅-Alkylen-NH₂, -C₁₋₆-Alkylen-NH-C₁₋₆-alkyl oder -C₁₋₆-Alkylen-N(C₁₋₆-alkyl)₂ ist; jedes substituierte oder unsubstituierte C₁₋₅-Alkoxy unabhängig C₁₋₅-Alkoxy oder C₁₋₅-Alkoxy, substituiert mit Halogen, -OH, -CN, NH₂, -NO₂, Carbonyl, =O, Oxo, -C₁₋₅-Alkylen-NH₂, -C₁₋₆-Alkylen-NH-C₁₋₃-alkyl oder -C₁₋₆-Alkylen-N(C₁₋₆-alkyl)₂ ist.

22. Verbindung nach einem der Ansprüche 1-21, wobei
jedes C₁₋₅-Alkyl unabhängig Methyl, Ethyl, Propyl, Isopropyl, Cyclopropyl, n-Butyl, Isobutyl, tert-Butyl, n-Pentyl, Neopentyl, Isopentyl, Cyclopentyl, n-Hexyl oder Cyclohexyl ist;
jedes C₁₋₃-Alkoxy unabhängig Methoxy, Ethoxy, Propoxy, Isopropoxy oder Cyclopropyloxy ist;
jedes C₂₋₃-Alkenyl unabhängig -CH=CH₂, -CH₂-CH=CH₂ oder -CH=CH-CH₃ ist;
jedes C₂₋₃-Alkinyl unabhängig -C=CH, -CH₂-C=CH oder -C=C-CH₃ ist; und/oder
jedes Halogen unabhängig -F, -Cl, -Br oder -I ist.

23. Verbindung nach einem der Ansprüche 1-22, wobei jede heterocyclische Ringgruppe und jede carbocyclische Ringgruppe einen einzelnen Ring, spiralförmigen Ring, Brückenring, kondensierten Ring und alle verschiedenen Kombinationen von spiralförmigem Ring, Brückenring und/oder kondensiertem Ring umfasst.

24. Verbindung nach Anspruch 23, wobei der genannte spiralförmige Ring 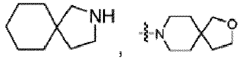 umfasst; und die genannten verschiedenen Kombinationen von spiralförmigem Ring, Brückenring und/oder kondensiertem Ring 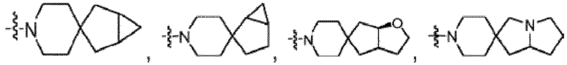 umfassen.

25. Verbindung nach Anspruch 1, wobei die Verbindung (S)-1-(4-((3-Amino-5-(4-amino-2-oxa-8-azaspiro[4.5]decan-8-yl)pyrazin-2-yl)thio)-3,3-difluorindolin-1-yl)ethanon ist.

## Revendications

1. Composé ayant la Formule I ou sel pharmaceutiquement acceptable, dans lequel : 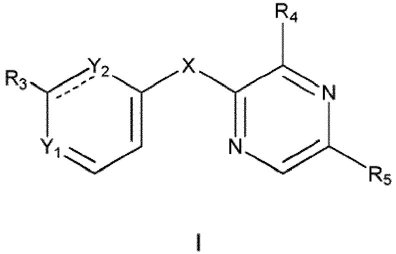
X est S ;
Y₁ est N ou un groupe CR₁ ;
Y₂ est N ou un groupe CR₂ ;
R₁ se combine avec R₃, ou R₂ se combine avec R₃, pour former un groupe hétéroaryle à 5 à 10 chaînons, un cycle carbocyclique à 5 à 10 chaînons ou un cycle hétérocyclique à 5 à 10 chaînons, dans lequel chacun des systèmes cycliques est optionnellement substitué indépendamment avec un atome d'halogène ou un groupe -CN, -OH, -NR₈R₉, -N₃, -NO₂, carbonyle, =O, oxo, alkyle C₁₋₆ substitué ou non substitué, alcoxy C₁₋₆ substitué ou non substitué, ou C(O)R₈ ;
lorsque R₁ se combine avec R₃ pour former un groupe hétéroaryle à 5 à 10 chaînons, un cycle carbocyclique à 5 à 10 chaînons ou un cycle hétérocyclique à 5 à 10 chaînons, R₂ est -H, un atome d'halogène ou un groupe -CN, -OH, -NH₂, -N₃, -NO₂, alcoxy C₁₋₆ substitué ou non substitué, ou alkyle C₁₋₆ substitué ou non substitué ;
lorsque R₂ se combine avec R₃ pour former un groupe hétéroaryle à 5 à 10 chaînons, un cycle carbocyclique à 5 à 10 chaînons ou un cycle hétérocyclique à 5 à 10 chaînons, R₁ est -H, un atome d'halogène ou un groupe -CN, -OH, -NH₂, -N₃, -NO₂, alcoxy C₁₋₆ substitué ou non substitué, ou alkyle C₁₋₆ substitué ou non substitué ;
R₄ est un atome d'halogène ou un groupe -CN, -OH, -NR₈R₉, -N₃, -NO₂, alcoxy C₁₋₆ substitué ou non substitué, alkyle C₁₋₆ substitué ou non substitué, un cycle hétérocyclique C₅₋₁₈ ou un cycle carbocyclique C₅₋₁₈ ; dans lequel chacun des systèmes cycliques est optionnellement substitué indépendamment avec un atome d'halogène ou un groupe -CN, -OH, -NO₂, carbonyle, =O, oxo, alkyle C₁₋₆ substitué ou non substitué, alcoxy C₁₋₆ substitué ou non substitué, -NR₈R₉ ou -CH₂NR₈R₉ ;
R₅ est -H, un atome d'halogène ou un groupe -CN, -OH, -NR₈R₉, -N₃, -NO₂, alkyle C₁₋₆, alcoxy C₁₋₆, aryle C₆₋₁₀, arylalkyle C₆₋₁₀, hétéroaryle C₆₋₁₀, un cycle hétérocyclique C₅₋₁₈ ou un cycle carbocyclique C₅₋₁₈ ; et dont chacun est optionnellement substitué indépendamment avec un atome d'halogène ou un groupe -CN, -OH, -N₃, -NO₂, -NH₂, carbonyle, =O, oxo, alkyle C₁₋₆ substitué ou non substitué, alcoxy C₁₋₆ substitué ou non substitué, (CH₂)ₖNR₈R₉, (CH₂)ₖNHC(O)OR₈ ou C(O)R₈ ; et k vaut 0, 1 ou 2 ;
chaque R₈ et R₉ est indépendamment -H, un atome d'halogène ou un groupe -CN, - OH, -N₃, -NO₂, alkyle C₁₋₆, alcoxy C₁₋₆, alcényle C₂₋₆, NH(alkyle C₁₋₆), N(alkyle C₁₋₆)₂, un cycle hétérocyclique C₅₋₁₀ ou un cycle carbocyclique C₅₋₁₀ ; et dont chacun peut être optionnellement substitué indépendamment avec un atome d'halogène ou un groupe -CN, -OH, -NH₂, -N₃, -NO₂, -alkylène C₁₋₆-NH₂, -alkylène C₁₋₆-NH-alkyle C₁₋₆, -alkylène C₁₋₆-N(alkyle C₁₋₆)₂, -NHBoc, -CH₂NHBoc, -NH-alkyle C₁₋₆, -N(alkyle C₁₋₆)₂, -NH-alcoxy C₁₋₆, -N(alcoxy C₁₋₆)₂, alkyle C₁₋₆ substitué ou non substitué, ou alcoxy C₁₋₆ substitué ou non substitué ; et chaque cycle hétérocyclique contient 1, 2, 3 ou 4 hétéroatomes sélectionnés parmi N, O ou S ;
dans lequel le groupe alkyle est constitué de radicaux hydrocarbonés monovalents saturés comportant des fractions linéaires, ramifiées ou cycliques, le groupe alcoxy est un groupe alkyle linéaire, à chaîne ramifiée ou cyclique, et l'hétérocyclique est un système cyclique non aromatique mono- ou polycyclique non substitué et substitué contenant un ou plusieurs hétéroatomes.

2. Composé selon la revendication 1, dans lequel
lorsque R₂ se combine avec R₃ pour former un groupe hétéroaryle à 5 à 10 chaînons, un cycle carbocyclique à 5 à 10 chaînons ou un cycle hétérocyclique à 5 à 10 chaînons, R₁ est -H, -F, -Cl, -Br, -I ou un groupe -CN, -OH, -NH₂, alcoxy C₁₋₃ substitué ou non substitué, ou alkyle C₁₋₃ substitué ou non substitué ;
lorsque R₁ se combine avec R₃ pour former un groupe hétéroaryle à 5 à 10 chaînons, un cycle carbocyclique à 5 à 10 chaînons ou un cycle hétérocyclique à 5 à 10 chaînons, R₂ est -H, -F, -Cl, -Br, -I ou un groupe -CN, -OH, -NH₂, alcoxy C₁₋₃ substitué ou non substitué, ou alkyle C₁₋₃ substitué ou non substitué ;
R₄ est -F, -Cl, -Br ou un groupe -CN, -OH, -NR₈R₉, alkyle C₁₋₆ substitué ou non substitué, alcoxy C₁₋₆ substitué ou non substitué, un cycle hétérocyclique C₅₋₁₈ ou un cycle carbocyclique C₅₋₁₀, dans lequel chacun des systèmes cycliques est optionnellement substitué avec -F, -Cl, -Br ou un groupe -CN -OH, -NO₂, carbonyle, =O, oxo, alkyle C₁₋₆ substitué ou non substitué, alcoxy C₁₋₆ substitué ou non substitué, -NH₂, -NH-alkyle C₁₋₆, -NH-alcoxy C₁₋₆, -alkylène C₁₋₆-NH₂ ou -alkylène C₁₋₆-NH-alkyle C₁₋₆ ;
R₅ est -H, -F, -Cl, -Br, -I ou un groupe -NR₈R₉, alkyle C₁₋₃, alcoxy C₁₋₃, aryle C₆₋₉, arylalkyle C₆₋₉, hétéroaryle C₆₋₉, un cycle hétérocyclique C₆₋₁₇ ou un cycle carbocyclique C₆₋₁₇, dans lequel chacun d'entre eux est optionnellement substitué indépendamment avec un atome d'halogène ou un groupe -CN, -OH, -N₃, -NO₂, -NH₂, carbonyle, =O, oxo, alkyle C₁₋₆ substitué ou non substitué, alcoxy C₁₋₆ substitué ou non substitué, (CH₂)ₖNR₈R₉, (CH₂)ₖNHC(O)OR₈ ou C(O)R₈ ; et k vaut 0, 1 ou 2 ; dans lequel le groupe hétéroaryle C₆₋₉ contient 1, 2, 3 ou 4 hétéroatomes sélectionnés parmi N, O ou S, et le groupe hétéroaryle C₆₋₉ est un groupe hétéroaryle à 6 chaînons, hétéroaryle à 7 chaînons, hétéroaryle à 8 chaînons, ou hétéroaryle à 9 chaînons ; le cycle hétérocyclique C₆₋₁₇ contient 1, 2, 3, 4, 5 ou 6 hétéroatomes sélectionnés parmi N, O ou S, et le cycle hétérocyclique C₆₋₁₇ est un cycle hétérocyclique à 6 chaînons, un cycle hétérocyclique à 7 chaînons, un cycle hétérocyclique à 8 chaînons, un cycle hétérocyclique à 9 chaînons, un cycle hétérocyclique à 10 chaînons, un cycle hétérocyclique à 11 chaînons, un cycle hétérocyclique à 12 chaînons, un cycle hétérocyclique à 13 chaînons, un cycle hétérocyclique à 14 chaînons, un cycle hétérocyclique à 15 chaînons, un cycle hétérocyclique à 16 chaînons ou un cycle hétérocyclique à 17 chaînons ;
chaque R₈ et R₉ est indépendamment -H, un atome d'halogène ou un groupe -CN, - OH, -NO₂, -alkylène C₁₋₆-NH₂, -alkylène C₁₋₆-NH-alkyle C₁₋₆, -alkylène C₁₋₆-N(alkyle C₁₋₆)₂, -CH₂NHBoc, -NH-alkyle C₁₋₆, -N(alkyle C₁₋₆)₂, -NH-alcoxy C₁₋₆, -N(alcoxy C₁₋₆)₂, alkyle C₁₋₆, alcoxy C₁₋₆, alcényle C₂₋₆, un cycle hétérocyclique C₅₋₁₀ ou un cycle carbocyclique C₅₋₁₀ ; chacun d'entre eux pouvant être optionnellement substitué avec un atome d'halogène ou un groupe -CN, -OH, -NH₂, -N₃, -NO₂, -alkylène C₁₋₆-NH₂, - alkylène C₁₋₆-NH-alkyle C₁₋₆, -alkylène C₁₋₆-N(alkyle C₁₋₆)₂, -NHBoc, -CH₂NHBoc, -NH-alkyle C₁₋₆, -N(alkyle C₁₋₆)₂, -NH-alcoxy C₁₋₆, -N(alcoxy C₁₋₆)₂, alkyle C₁₋₆ substitué ou non substitué, ou alcoxy C₁₋₆ substitué ou non substitué ; et chaque cycle hétérocyclique contient 1, 2, 3 ou 4 hétéroatomes sélectionnés parmi N, O ou S.

3. Composé selon les revendications 1 ou 2, dans lequel
lorsque R₂ se combine avec R₃ pour former un groupe hétéroaryle à 5 à 10 chaînons, un cycle carbocyclique à 5 à 10 chaînons ou un cycle hétérocyclique à 5 à 10 chaînons, R₁ est -H, -F, -Cl ou un groupe -NH₂, méthyle, éthyle, propyle, isopropyle, méthoxy, éthoxy, propoxy ou isopropoxy ; et chaque groupe méthyle, éthyle, propyle, isopropyle, méthoxy, éthoxy, propoxy ou isopropoxy est optionnellement substitué indépendamment avec un atome d'halogène ou un groupe OH ou NH₂ ;
lorsque R₁ se combine avec R₃ pour former un groupe hétéroaryle à 5 à 10 chaînons, un cycle carbocyclique à 5 à 10 chaînons ou un cycle hétérocyclique à 5 à 10 chaînons, R₂ est -H, -F, -Cl ou un groupe -NH₂, méthyle, éthyle, propyle, isopropyle, méthoxy, éthoxy, propoxy ou isopropoxy ; et chaque groupe méthyle, éthyle, propyle, isopropyle, méthoxy, éthoxy, propoxy ou isopropoxy est optionnellement substitué indépendamment avec un atome d'halogène ou un groupe OH ou NH₂ ;
R₄ est -F, -Cl, -CN ou un groupe -OH, -NH₂, un groupe alkyle C₁₋₃ substitué ou non substitué, un groupe alcoxy C₁₋₃ substitué ou non substitué, un cycle hétérocyclique à 5 chaînons contenant 1, 2 ou 3 hétéroatomes sélectionnés parmi N ou O, un cycle hétérocyclique à 6 chaînons contenant 1, 2 ou 3 hétéroatomes sélectionnés parmi N ou O, un cycle carbocyclique à 5 chaînons ou un cycle carbocyclique à 6 chaînons ; dans lequel chacun des systèmes cycliques est optionnellement substitué avec -F, - Cl, -Br ou un groupe -CN -OH, carbonyle, =O, oxo, alkyle C₁₋₃ substitué ou non substitué, alcoxy C₁₋₃ substitué ou non substitué, -NH₂, -NH-alkyle C₁₋₃, -NH-alcoxy C₁₋₃, -alkylène C₁₋₃-NH₂ ou -alkylène C₁₋₃-NH-alkyle C₁₋₃ ;
R₅ est -F, -Cl, -Br ou un groupe -NR₈R₉, méthyle, éthyle, propyle, isopropyle, méthoxy, éthoxy, propoxy, isopropoxy, aryle à 6 chaînons, aryle à 7 chaînons, aryle à 8 chaînons, aryle à 9 chaînons, arylalkyle à 6 chaînons, arylalkyle à 7 chaînons, arylalkyle à 8 chaînons, arylalkyle à 9 chaînons, hétéroaryle à 6 chaînons, hétéroaryle à 7 chaînons, hétéroaryle à 8 chaînons, hétéroaryle à 9 chaînons, un cycle hétérocyclique à 6 chaînons, un cycle hétérocyclique à 7 chaînons, un cycle hétérocyclique à 8 chaînons, un cycle hétérocyclique à 9 chaînons, un cycle hétérocyclique à 10 chaînons, un cycle hétérocyclique à 11 chaînons, un cycle hétérocyclique à 12 chaînons, un cycle hétérocyclique à 13 chaînons, un cycle hétérocyclique à 14 chaînons, un cycle hétérocyclique à 15 chaînons, un cycle hétérocyclique à 16 chaînons, un cycle carbocyclique à 6 chaînons, un cycle carbocyclique à 7 chaînons, un cycle carbocyclique à 8 chaînons, un cycle carbocyclique à 9 chaînons, un cycle carbocyclique à 10 chaînons, un cycle carbocyclique à 11 chaînons, un cycle carbocyclique à 12 chaînons, un cycle carbocyclique à 13 chaînons, un cycle carbocyclique à 14 chaînons, un cycle carbocyclique à 15 chaînons ou un cycle carbocyclique à 16 chaînons ; et chaque groupe hétéroaryle contient 1, 2 ou 3 hétéroatomes sélectionnés parmi N, O ou S, et chaque cycle hétérocyclique contient 1, 2, 3 ou 4 hétéroatomes sélectionnés parmi N, O ou S ; dans lequel chacun d'entre eux est optionnellement substitué indépendamment avec -F, -Cl, -Br, -I ou un groupe -CN, -OH, -NO₂, -NH₂, carbonyle, =O, oxo, alkyle C₁₋₃ substitué ou non substitué, alcoxy C₁₋₃ substitué ou non substitué, (CH₂)ₖNR₈R₉, (CH₂)ₖNHC(O)OR₈, ou C(O)R₈ substitué ou non substitué ;
et k vaut 0, 1 ou 2 ; et/ou
chaque R₈ et R₉ est indépendamment -H, -F, -Cl ou un groupe -CN, -OH, -NO₂, - alkylène C₁₋₃-NH₂, -alkylène C₁₋₃-NH-alkyle C₁₋₃, -alkylène C₁₋₃-N(alkyle C₁₋₃)₂, CH₂NHBoc, -NH-alkyle C₁₋₃, -N(alkyle C₁₋₃)₂, -NH-alcoxy C₁₋₃, -N(alcoxy C₁₋₃)₂, alkyle C₁₋₄, alcoxy C₁₋₃, alcényle C₂₋₃, un cycle hétérocyclique à 5 chaînons, un cycle hétérocyclique à 6 chaînons, un cycle hétérocyclique à 7 chaînons, un cycle hétérocyclique à 8 chaînons, un cycle hétérocyclique à 9 chaînons, un cycle hétérocyclique à 10 chaînons, un cycle carbocyclique à 5 chaînons, un cycle carbocyclique à 6 chaînons, un cycle carbocyclique à 7 chaînons, un cycle carbocyclique à 8 chaînons, un cycle carbocyclique à 9 chaînons, un cycle carbocyclique à 10 chaînons ; et dont chacun peut être optionnellement substitué indépendamment avec un atome d'halogène ou un groupe -CN, -OH, -NH₂, -N₃, -NO₂, -alkylène C₁₋₆-NH₂, -alkylène C₁₋₆-NH-alkyle C₁₋₆, -alkylène C₁₋₆-N(alkyle C₁₋₆)₂, -NHBoc, -CH₂NHBoc, -NH-alkyle C₁₋₆, -N(alkyle C₁₋₆)₂, -NH-alcoxy C₁₋₆, -N(alcoxy C₁₋₆)₂, alkyle C₁₋₆ substitué ou non substitué, ou alcoxy C₁₋₆ substitué ou non substitué ; et chaque cycle hétérocyclique contient 1, 2, 3 ou 4 hétéroatomes sélectionnés parmi N, O ou S.

4. Composé selon l'une quelconque des revendications 1 à 3, dans lequel R₁ se combine avec R₃, ou R₂ se combine avec R₃, pour former un groupe hétéroaryle à 5 chaînons, un groupe hétéroaryle à 6 chaînons, un groupe hétéroaryle à 7 chaînons, un groupe hétéroaryle à 8 chaînons, un cycle hétérocyclique à 5 chaînons, un cycle hétérocyclique à 6 chaînons, un cycle hétérocyclique à 7 chaînons ou un cycle hétérocyclique à 8 chaînons, dans lequel chacun des systèmes cycliques contient 1, 2, 3 ou 4 hétéroatomes sélectionnés parmi N, O ou S, et est optionnellement substitué avec -F, -Cl, -Br, -I ou un groupe -CN, -OH, -NH₂, carbonyle, =O, oxo, alkyle C₁₋₃ substitué ou non substitué, alcoxy C₁₋₃ substitué ou non substitué ou C(O)R₈.

5. Composé selon l'une quelconque des revendications 1 à 4, dans lequel R₁ se combine avec R₃ pour former un groupe hétéroaryle à 5 chaînons, un groupe hétéroaryle à 6 chaînons, un cycle hétérocyclique à 5 chaînons ou un cycle hétérocyclique à 6 chaînons, dans lequel chacun des systèmes cycliques contient 1 ou 2 hétéroatomes sélectionnés parmi N ou O, et est optionnellement substitué avec -F, -Cl, -Br ou un groupe -OH, -NH₂, carbonyle, =O, oxo, méthyle, éthyle, propyle, isopropyle, méthoxy, CHF₂, CH₂F, CF₃ ou C(O)R₈ ; et chaque groupe méthyle, éthyle, propyle, isopropyle, méthoxy, CHF₂, CH₂F ou C(O)R₈ est optionnellement substitué indépendamment avec -F, -Cl, -Br ou -I.

6. Composé selon l'une quelconque des revendications 1 à 5, dans lequel R₂ se combine avec R₃ pour former un groupe hétéroaryle à 5 chaînons, un groupe hétéroaryle à 6 chaînons, un groupe hétéroaryle à 7 chaînons, un groupe hétéroaryle à 8 chaînons, un cycle hétérocyclique à 5 chaînons, un cycle hétérocyclique à 6 chaînons, un cycle hétérocyclique à 7 chaînons ou un cycle hétérocyclique à 8 chaînons, dans lequel chacun des systèmes cycliques contient 1, 2, 3 ou 4 hétéroatomes sélectionnés parmi N, O ou S, et est optionnellement substitué avec - F, -Cl, -Br, -I ou un groupe -CN, -OH, -NH₂, carbonyle, =O, oxo, alkyle C₁₋₃ substitué ou non substitué, alcoxy C₁₋₃ substitué ou non substitué ou C(O)R₈ ; et le groupe alkyle C₁₋₃ est indépendamment un groupe méthyle, éthyle, propyle, isopropyle ou cyclopropyle ; le groupe alcoxy C₁₋₃ est indépendamment un groupe méthoxy, éthoxy, propoxy, isopropoxy ou cyclopropyloxy.

7. Composé selon l'une quelconque des revendications 1 à 6, dans lequel R₄ est -Cl ou un groupe -NH₂, méthyle ou pipéridinyle, dans lequel le groupe pipéridinyle est optionnellement substitué avec un groupe méthyle, -NH₂ ou -CH₂NH₂.

8. Composé selon l'une quelconque des revendications 1 à 7, dans lequel :
R₅ est 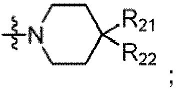
chaque R₂₁ et R₂₂ est indépendamment un atome d'halogène ou un groupe alkyle C₁₋₃, -NH₂, -alkylène C₁₋₃-NH₂, -alkylène C₁₋₂-NH-alkyle C1-3, -alkylène C₁₋₂-N(alkyle C₁₋₃)₂, -NHBoc ou -CH₂NHBoc,
ou R₂₁ et R₂₂ conjointement avec l'atome de carbone auquel ils sont tous deux fixés forment un groupe hétéroaryle à 5 à 10 chaînons, un cycle carbocyclique à 5 à 10 chaînons ou un cycle hétérocyclique à 5 à 10 chaînons, dans lequel chacun des systèmes cycliques est optionnellement substitué avec un atome d'halogène ou un groupe -CN, -OH, carbonyle, =O, oxo, -alkylène C₁₋₃-NH₂, -alkylène C₁₋₂-NH-alkyle C-₁₋₃, -alkylène C₁₋₂-N(alkyle C₁₋₃)₂, -NHBoc, -CH₂NHBoc, -NH₂, alcoxy C₁₋₃ ou alkyle C1-3.

9. Composé selon la revendication 8, dans lequel, R₂₁ et R₂₂ conjointement avec l'atome de carbone auquel ils sont tous deux fixés forment un groupe hétéroaryle à 5 chaînons, un groupe hétéroaryle à 6 chaînons, un groupe hétéroaryle à 7 chaînons, un groupe hétéroaryle à 8 chaînons, un groupe hétéroaryle à 9 chaînons, un groupe hétéroaryle à 10 chaînons, un cycle hétérocyclique à 5 chaînons, un cycle hétérocyclique à 6 chaînons, un cycle hétérocyclique à 7 chaînons, un cycle hétérocyclique à 8 chaînons, un cycle hétérocyclique à 9 chaînons ou un cycle hétérocyclique à 10 chaînons ; dans lequel chacun des systèmes cycliques contient 1, 2 ou 3 hétéroatomes sélectionnés parmi N, O ou S, et est optionnellement substitué indépendamment avec un atome d'halogène ou un groupe -CN, -OH, carbonyle, =O, oxo, -NH₂, alcoxy C₁₋₃ ou alkyle C₁₋₃ ; et le groupe alcoxy C₁₋₃ est indépendamment un groupe méthoxy, éthoxy, propoxy, isopropoxy ou cyclopropyloxy ; le groupe alkyle C₁₋₃ est indépendamment un groupe méthyle, éthyle, propyle, isopropyle ou cyclopropyle.

10. Composé selon l'une quelconque des revendications 1 à 9, dans lequel R₅ est un groupe -NH₂,

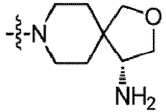

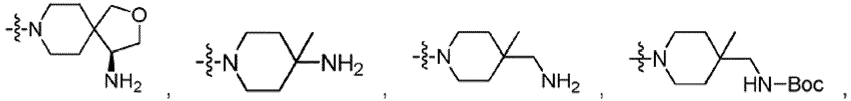

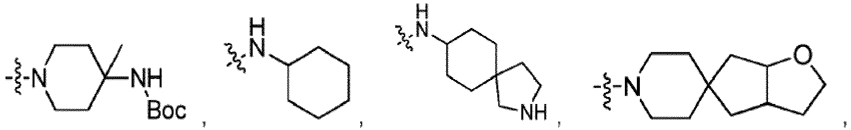

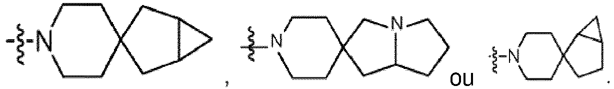

11. Composé selon l'une quelconque des revendications 1 à 10, dans lequel R₈ et R₉ sont indépendamment -H ou un groupe méthyle, tert-butyle, -CH=CH₂, N(CH₃)₂,

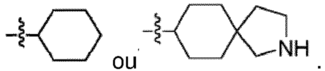

12. Composé selon l'une quelconque des revendications 1 à 11, dans lequel
X est S ;
Y₁ est N et Y₂ est un groupe CR₂ ;
Y₂ est N et Y₁ est un groupe CR₁ ; et/ou
R₄ est un groupe NH₂ ;

13. Composé selon l'une quelconque des revendications 1 à 12, le composé ayant la Formule II : 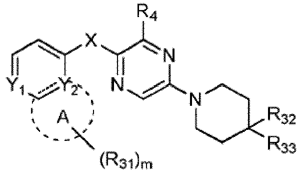 II
dans lequel
X est S ;
Y₁ est N ou un groupe CR₂₅ ;
Y₂ est C ;
R₂₅ est H, un atome d'halogène ou un groupe alkyle C1-3 ou alcoxy C1-3 ;
le cycle 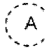 est un groupe hétéroaryle à 5 à 8 chaînons contenant 1, 2, 3 ou 4 hétéroatomes sélectionnés parmi N, O ou S, un cycle carbocyclique à 5 à 8 chaînons ou un cycle hétérocyclique à 5 à 8 chaînons contenant 1, 2, 3 ou 4 hétéroatomes sélectionnés parmi N, O ou S ;
R₃₁ est -H, un atome d'halogène ou un groupe -OH, -NH₂, -(C=O)alkyle C1-3, -CN,-NO₂, carbonyle, =O, oxo, carboxyle, -alkylène C₁₋₃-NH₂, -NH-alkyle C1-3, -N(alkyle C₁₋₃)₂, -NH-alcoxy C₁₋₃, -N(alcoxy C₁₋₃)₂, alkyle C1-3 substitué ou non substitué, ou alcoxy C₁₋₃ substitué ou non substitué ;
m vaut 0, 1, 2, 3, ou 4 ;
R₄ est -H, un atome d'halogène ou un groupe -NH₂, alcoxy C1-3 substitué ou non substitué, ou alkyle C₁₋₃ substitué ou non substitué ;
chaque R₃₂ et R₃₃ est indépendamment -H, un atome d'halogène ou un groupe -OH, -NH₂, -CN, -NO₂, -alkylène C₁₋₃-NH₂, -alkylène C₁₋₂-NH-alkyle C1-3, - alkylène C₁₋₂-N(alkyle C₁₋₃)₂, -NHBoc, -CH₂NHBoc, -NH-alkyle C₁₋₃, -N(alkyle C₁₋₃)₂, - NH-alcoxy C₁₋₃, -N(alcoxy C₁₋₃)₂, alkyle C1-3 substitué ou non substitué, ou alcoxy C₁₋₃ substitué ou non substitué ;
ou R₃₂ et R₃₃ conjointement avec l'atome de carbone auquel ils sont tous deux fixés forment un groupe hétéroaryle à 5 à 8 chaînons contenant 1, 2 ou 3 hétéroatomes sélectionnés parmi N, O ou S, ou un cycle hétérocyclique à 5 à 8 chaînons contenant 1, 2 ou 3 hétéroatomes sélectionnés parmi N, O ou S, dans lequel chacun des systèmes cycliques est optionnellement substitué avec un atome d'halogène ou un groupe -CN, -OH, -NH₂, carbonyle, =O, oxo, -CH₂NH₂, -alkylène C₁₋₃-NH₂, -alkylène C₁₋₂-NH-alkyle C1-3, -alkylène C₁₋₂-N(alkyle C₁₋₃)₂, -NHBoc, -CH₂NHBoc, -NH-alkyle C1-3, -N(alkyle C₁₋₃)₂, -NH-alcoxy C₁₋₃, -N(alcoxy C₁₋₃)₂, alkyle C1-3 substitué ou non substitué, ou alcoxy C₁₋₃ substitué ou non substitué ;

14. Composé selon la revendication 13, dans lequel
le cycle 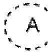 est un groupe hétéroaryle à 5 chaînons, un groupe hétéroaryle à 6 chaînons, un groupe hétéroaryle à 7 chaînons, un cycle hétérocyclique à 5 chaînons, un cycle hétérocyclique à 6 chaînons, un cycle hétérocyclique à 7 chaînons, un cycle carbocyclique à 5 chaînons, un cycle carbocyclique à 6 chaînons, un cycle carbocyclique à 7 chaînons ou un cycle carbocyclique à 8 chaînons ; et chacun des systèmes cycliques contient 1, 2 ou 3 hétéroatomes sélectionnés parmi N, O ou S ;
R₃₁ est -H, -F, -Cl, -Br, -I ou un groupe -OH, -NH₂, carbonyle, =O, oxo, -(CO)alkyle C₁₋₃, -alkylène C₁₋₃-NH₂, -NH-alkyle C1-3, -N(alkyle C₁₋₃)₂, alkyle C1-3 substitué ou non substitué, ou alcoxy C₁₋₃ substitué ou non substitué ;
R₃₂ et R₃₃ conjointement avec l'atome de carbone auquel ils sont tous deux fixés forment un cycle hétérocyclique à 5 chaînons, un cycle hétérocyclique à 6 chaînons ou un cycle hétérocyclique à 7 chaînons ; dans lequel chacun des systèmes cycliques contient 1 ou 2 hétéroatomes sélectionnés indépendamment parmi O ou N, et est optionnellement substitué avec un atome d'halogène ou un groupe -CN, -OH, -NH₂, carbonyle, =O, oxo, -NO₂, alkyle C1-3 ou alcoxy C1-3 ; et/ou
chaque R₃₂ et R₃₃ est indépendamment un groupe -CH₂NH₂, -CH₂NHBoc ou méthyle.

15. Composé selon la revendication 13 ou 14, dans lequel
le cycle 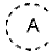 est un cycle hétérocyclique à 5 chaînons contenant 1, 2 ou 3 hétéroatomes sélectionnés parmi N ou O, un cycle hétérocyclique à 6 chaînons contenant 1 ou 2 hétéroatomes sélectionnés parmi N ou O ou un cycle carbocyclique à 5 chaînons ;
R₃₁ est -F ou un groupe -COCH₃, carbonyle, =O, oxo, -CH₃ ou -CF₃ ; et/ou
R₃₂ et R₃₃ conjointement avec l'atome de carbone auquel ils sont tous deux fixés forment un cycle hétérocyclique à 5 chaînons ; et le cycle hétérocyclique contient 1 hétéroatome sélectionné parmi O ou N, et est optionnellement substitué avec -F, -Cl ou un groupe -OH, -NH₂, carbonyle, =O, oxo, méthyle ou méthoxy.

16. Composé selon l'une quelconque des revendications 13 à 15, dans lequel
Y₁ est N ;
Y₂ est C ;
R₂₅ est -H ou -Cl ; et/ou
R₄ est un groupe NH₂ ;

17. Composé selon l'une quelconque des revendications 1 à 12, le composé ayant la Formule III : 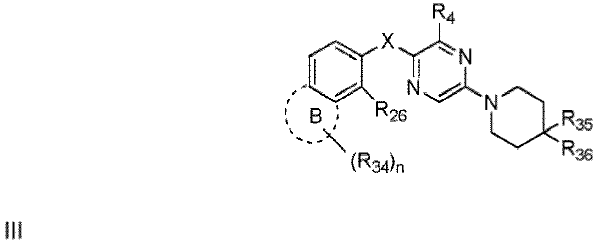 dans lequel
X est S ;
R₂₆ est -H, un atome d'halogène ou un groupe alkyle C1-3 substitué ou non substitué, ou alcoxy C1-3 substitué ou non substitué ;
le cycle 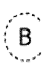 est un groupe hétéroaryle à 5 à 8 chaînons ou un cycle hétérocyclique à 5 à 8 chaînons ; et chacun des systèmes cycliques contient indépendamment 1, 2, 3 ou 4 hétéroatomes sélectionnés parmi N, O ou S ;
R₃₄ est -H, un atome d'halogène ou un groupe -OH, -NR₃₅R₃₆, -CN, -NO₂, carbonyle, =O, oxo, alkyle C₁₋₃ substitué ou non substitué, ou alcoxy C1-3 substitué ou non substitué ;
n vaut 0, 1, 2 ou 3 ;
R₄ est un atome d'halogène ou un groupe -NH₂, alcoxy C₁₋₆ substitué ou non substitué, ou alkyle C₁₋₆ substitué ou non substitué ;
chaque R₃₅ et R₃₆ est indépendamment -H, un atome d'halogène ou un groupe -OH, -NH₂, -CN, -NO₂, -CH₂NH₂, alkyle C1-3 substitué ou non substitué, ou alcoxy C₁₋₃ substitué ou non substitué ;
ou R₃₅ et R₃₆ conjointement avec l'atome de carbone auquel ils sont tous deux fixés forment un cycle hétéroaryle à 5 à 8 chaînons ou un cycle hétérocyclique à 5 à 8 chaînons, dans lequel chacun des systèmes cycliques contient indépendamment 1, 2 ou 3 hétéroatomes sélectionnés parmi N, O ou S, et est optionnellement substitué avec un atome d'halogène ou un groupe -CN, -OH, -NH₂, carbonyle, =O, oxo, - CH₂NH₂, -alkylène C₁₋₃-NH₂, -alkylène C₁₋₂-NH-alkyle C1-3, -alkylène C₁₋₂-N(alkyle C₁₋₃)₂, -NHBoc, -CH₂NHBoc, -NH-alkyle C1-3, -N(alkyle C₁₋₃)₂, -NH-alcoxy C₁₋₃, -N(alcoxy C₁₋₃)₂, alkyle C1-3 substitué ou non substitué, ou alcoxy C1-3 substitué ou non substitué.

18. Composé selon la revendication 17, dans lequel
le cycle 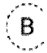 est un groupe hétéroaryle à 5 chaînons, un groupe hétéroaryle à 6 chaînons, un groupe hétéroaryle à 7 chaînons, un cycle hétérocyclique à 5 chaînons, un cycle hétérocyclique à 6 chaînons ou un cycle hétérocyclique à 7 chaînons ; et chacun des systèmes cycliques contient indépendamment 1, 2 ou 3 hétéroatomes sélectionnés parmi N, O ou S, et/ou
R₃₅ et R₃₆ conjointement avec l'atome de carbone auquel ils sont tous deux fixés forment un cycle hétérocyclique à 5 chaînons, un cycle hétérocyclique à 6 chaînons ou un cycle hétérocyclique à 7 chaînons ; dans lequel chacun des systèmes cycliques contient indépendamment 1 ou 2 hétéroatomes sélectionnés indépendamment parmi O ou N, et est optionnellement substitué avec un atome d'halogène ou un groupe - CN, -OH, -NH₂, carbonyle, =O, oxo, -NO₂, alkyle C1-3 ou alcoxy C₁₋₃.

19. Composé selon la revendication 17 ou 18, dans lequel
le cycle 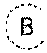 est un cycle hétérocyclique à 5 chaînons ou un cycle hétérocyclique à 6 chaînons ; et chacun des systèmes cycliques contient indépendamment 1 ou 2 hétéroatomes sélectionnés parmi N ou O ; et/ou
R₃₅ et R₃₆ conjointement avec l'atome de carbone auquel ils sont tous deux fixés forment un cycle hétérocyclique à 5 chaînons ; dans lequel le système cyclique contient 1 hétéroatome sélectionné indépendamment parmi O ou N, et est optionnellement substitué avec -F, -Cl, -OH ou un groupe -NH2, carbonyle, =O, oxo, méthyle ou méthoxy.

20. Composé selon l'une quelconque des revendications 17 à 19, dans lequel
R₂₆ est -H ou -Cl ;
R₄ est un groupe -NH₂ ; et/ou
R₃₄ est -F ou un groupe -COCH₃, =O, -CH₃ ou -CF₃.

21. Composé selon l'une quelconque des revendications 1 à 20, dans lequel chaque groupe alkyle C₁₋₆ substitué ou non substitué est indépendamment un groupe alkyle C₁₋₆, ou alkyle C₁₋₆ substitué avec un atome d'halogène ou un groupe -OH, -CN, NH₂, -NO₂, carbonyle, =O, oxo, -alkylène C₁₋₆-NH₂, -alkylène C₁₋₆-NH-alkyle C₁₋₆, ou - alkylène C₁₋₆-N(alkyle C₁₋₆)₂ ; chaque groupe alcoxy C₁₋₆ substitué ou non substitué est indépendamment un groupe alcoxy C₁₋₆, ou alcoxy C₁₋₆ substitué avec un atome d'halogène ou un groupe -OH, -CN, NH₂, -NO₂, carbonyle, =O, oxo, -alkylène C₁₋₆-NH₂, -alkylène C₁₋₆-NH-alkyle C1-3 ou -alkylène C₁₋₆-N(alkyle C₁₋₆)₂.

22. Composé selon l'une quelconque des revendications 1 à 21, dans lequel
chaque groupe alkyle C₁₋₆ est indépendamment un groupe méthyle, éthyle, propyle, isopropyle, cyclopropyle, n-butyle, isobutyle, tert-buyle, n-pentyle, néopentyle, isopentyle, cyclopentyle, n-hexyle ou cyclohexyle ;
chaque groupe alcoxy C₁₋₃ est indépendamment un groupe méthoxy, éthoxy, propoxy, isopropoxy ou cyclopropyloxy ;
chaque groupe alcényle C₂₋₃ est indépendamment un groupe -CH=CH₂, -CH₂-CH=CH₂ ou -CH=CH-CH₃ ;
chaque groupe alkynyle C₂₋₃ est indépendamment un groupe -C=CH, -CH₂-C≡CH ou -C≡C-CH₃ ; et/ou
chaque atome d'halogène est indépendamment -F, -Cl, -Br ou -I.

23. Composé selon l'une quelconque des revendications 1 à 22, dans lequel chaque groupe cyclique hétérocyclique et chaque groupe cyclique carbocyclique comprend un cycle simple, un cycle spiro, un cycle ponté, un cycle condensé et toutes les diverses combinaisons de cycle spiro, de cycle ponté et/ou de cycle condensé.

24. Composé selon la revendication 23, ledit cycle spiro comprenant 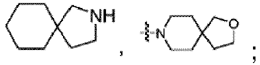 et lesdites diverses combinaisons de cycle spiro, de cycle ponté et/ou de cycle condensé comprennent

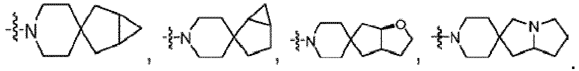

25. Composé selon la revendication 1, dans lequel le composé est :
la (S)-1-(4-((3-amino-5-(4-amino-2-oxa-8-azaspiro[4.5]décan-8-yl)pyrazin-2-yl)thio)-3,3-difluoroindolin-1-yl)éthanone.
